(19) Europäisches Patentamt
European Patent Office
Office européen des brevets

(11) EP 3 307 912 B1

(12) EUROPEAN PATENT SPECIFICATION

(45) Date of publication and mention of the grant of the patent:
**15.09.2021 Bulletin 2021/37**

(21) Application number: **16810619.3**

(22) Date of filing: **15.06.2016**

(51) Int Cl.:
***C12Q 1/6816*** *(2018.01)* ***C12Q 1/6827*** *(2018.01)*
***C12Q 1/6881*** *(2018.01)*

(86) International application number:
**PCT/AU2016/050499**

(87) International publication number:
**WO 2016/201507 (22.12.2016 Gazette 2016/51)**

(54) **METHOD OF MEASURING CHIMERISM**

VERFAHREN ZUR MESSUNG VON CHIMÄRISMUS

PROCÉDÉ DE MESURE DU CHIMÉRISME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.06.2015 AU 2015902274**

(43) Date of publication of application:
**18.04.2018 Bulletin 2018/16**

(73) Proprietor: **Murdoch Childrens Research Institute**
**Parkville, Victoria 3052 (AU)**

(72) Inventor: **SLATER, Howard**
**Parkville, Victoria 3052 (AU)**

(74) Representative: **Dehns**
**St. Bride's House**
**10 Salisbury Square**
**London EC4Y 8JD (GB)**

(56) References cited:
**WO-A1-2009/039589 WO-A1-2015/042649**
**WO-A2-2012/012703 WO-A2-2012/142334**

- **DAVID GEORGE ET AL: "Detection and quantification of chimerism by droplet digital PCR", CHIMERISM, vol. 4, no. 3, 1 July 2013 (2013-07-01), pages 102-108, XP055521058, ISSN: 1938-1956, DOI: 10.4161/chim.25400**
- **BENJAMIN J. HINDSON ET AL: "High-Throughput Droplet Digital PCR System for Absolute Quantitation of DNA Copy Number", ANALYTICAL CHEMISTRY, vol. 83, no. 22, 15 November 2011 (2011-11-15), pages 8604-8610, XP055047554, ISSN: 0003-2700, DOI: 10.1021/ac202028g**
- **LEONARDO B. PINHEIRO ET AL: "Evaluation of a Droplet Digital Polymerase Chain Reaction Format for DNA Copy Number Quantification", ANALYTICAL CHEMISTRY, vol. 84, no. 2, 17 January 2012 (2012-01-17), pages 1003-1011, XP055047877, ISSN: 0003-2700, DOI: 10.1021/ac202578x**
- **WEAVER S ET AL: "Taking qPCR to a higher level: Analysis of CNV reveals the power of high throughput qPCR to enhance quantitative resolution", METHODS, ACADEMIC PRESS, US, vol. 50, no. 4, 1 April 2010 (2010-04-01), pages 271-276, XP026966680, ISSN: 1046-2023 [retrieved on 2010-01-15]**
- **BRUNO, DL. ET AL.: 'Use of Copy Number Deletion Polymorphisms to Assess DNA Chimerism' CLINICAL CHEMISTRY vol. 60, no. 8, 2014, pages 1105 - 1114, XP009183734**
- **BADER, P. ET AL.: 'How and when should we monitor chimerism after allogeneic stem cell transplantation?' BONE MARROW TRANSPLANTATION. vol. 35, no. 2, 2005, pages 107 - 119, XP055002342**
- **WU, D. ET AL.: 'In situ genetic analysis of cellular chimerism' NATURE MEDICINE. vol. 15, no. 2, 2009, pages 215 - 219, XP055337581**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **CONRAD, D. ET AL.: 'Origins and functional impact of copy number variation in the human genome' NATURE vol. 464, no. 7289, 2010, pages 704 - 712, XP055146767**

Remarks:

The file contains technical information submitted after the application was filed and not included in this specification

## Description

## FIELD OF THE INVENTION

The present disclosure relates to methods of measuring chimerism in a biological sample using informative copy number variations (CNV).

## BACKGROUND OF THE INVENTION

Chimerism describes the co-existence of cells originating from more than one individual. One of the most frequently encountered examples of chimerism is biological samples from individuals who have received hematopoietic stem-cell transplantation (HSCT).

HSCT is a common treatment for patients suffering from certain malignant and non-malignant haematological disorders. An important component of the clinical management of HSCT is chimerism analysis for timely assessment of treatment success (e.g. engraftment) or failure (e.g. disease relapse, graft rejection and graft-versus-host disease).

One of the most common methods for monitoring chimerism following HSCT is by PCR amplification of short tandem repeat (STR) loci followed by capillary electrophoresis (Thiede et al. Bone Marrow Transplant, 23:1055-1060, 1999; Nuckols et al. Am J Clin Pathol, 113:135-140, 2000). However, multiple STR loci must be analysed in order to identify loci that differentiate the donor and recipient. This is especially true since the donor and recipients are often related and thus often share multiple genetic determinants.

Another established method of monitoring chimerism includes Fluorescence in situ Hybridization (FISH) of nuclei using X- and Y-specific chromosome probes for cytological distinction of donor and recipient cells. However, FISH has the restriction of requiring a gender mismatch between donor and recipient. FISH also has sensitivity of about >1%, which is inadequate for early relapse detection.

More recently described methods for monitoring chimerism include TaqMan real-time PCR of SNP or indel markers. The disadvantage of these real-time formats is that each marker has a relatively low discrimination power and therefore a relatively large numbers of loci need to be evaluated. Furthermore, informative polymorphisms are not available in all cases.

Accordingly, there remains an unmet need for accurate and efficient methods of measuring chimerism in biological samples.

## SUMMARY OF THE INVENTION

The present inventors have found that they are able to accurately measure chimerism in a biological sample by assessing informative copy number variation (CNV) polymorphisms in genetically distinct cell populations. These findings suggest that assessing CNV polymorphisms in genomic DNA may provide a useful *in-vitro* method of measuring chimerism. Accordingly, in one example, the present disclosure relates to a method of measuring chimerism in a biological sample obtained from a subject comprising two or more genetically distinct cell populations, the method comprising isolating the genomic DNA from the cells in the sample, determining the level of genomic DNA in the sample from a first genetically distinct cell population based on a copy number variation (CNV) polymorphism that is an informative marker of the first genetically distinct cell population, determining the total level of genomic DNA isolated from the genetically distinct cell populations in the biological sample, wherein the level of genomic DNA in the sample from said first genetically distinct cell population and the total level of genomic DNA isolated from the genetically distinct cell populations provides the measure of chimerism in the sample.

In another example, the method further comprises determining the level of genomic DNA in the sample from a second genetically distinct cell population based on a CNV polymorphism that is an informative marker of the second genetically distinct cell population, wherein the levels of genomic DNA from said first and second genetically distinct cell populations and the total level of genomic DNA isolated from the genetically distinct cell populations provide the measure of chimerism in the sample.

In an example, an informative marker of a genetically distinct cell population comprises:

- a CNV polymorphism that is homozygous present in isolated genomic DNA from the first cell population and homozygous not present in isolated genomic DNA from a second cell population; or,
- a CNV polymorphism that is homozygous present in the isolated genomic DNA from a second cell population and homozygous not present in the isolated genomic DNA from the first cell population.

The present inventors have also found that they are able to validate the accuracy of the methods of the present disclosure using an internal validation step. Accordingly, in an example, the methods of the present disclosure further comprise validating the level of isolated DNA from a genetically distinct cell population by determining the total isolated genomic DNA in the sample, wherein levels of isolated genomic DNA from the genetically distinct cell populations that are about equal to the level of total DNA indicates that the levels of isolated genomic DNA are validated. In an example, the measure of chimerism is expressed as a percentage of genetically distinct cells in the total cell population in the sample. In another example, the measure of chimerism is expressed as a ratio of genetically distinct cells in the sample (first cell population:second cell population).

In another example, the CNV polymorphism is a copy number deletion (CND) polymorphism. In an example, an informative marker of a genetically distinct cell population comprises:

- a CND polymorphism that is homozygous deleted in isolated genomic DNA from the first cell population and heterozygous or homozygous non-deleted in the isolated genomic DNA from a second cell population; and/or,
- a CND polymorphism that is homozygous deleted in isolated genomic DNA from a second cell population and heterozygous or homozygous non-deleted in the isolated genomic DNA from the first cell population.

In another example, an informative marker of a genetically distinct cell population comprises:

- at least two CND polymorphisms that are homozygous deleted in isolated genomic DNA from the first cell population and heterozygous or homozygous non-deleted in the isolated genomic DNA from a second cell population; and/or,
- at least two CND polymorphisms that are homozygous deleted in isolated genomic DNA from a second cell population and heterozygous or homozygous non-deleted in the isolated genomic DNA from the first cell population.

In another example, an informative marker of a genetically distinct cell population comprises:

- at least three CND polymorphisms that are homozygous deleted in isolated genomic DNA from the first cell population and heterozygous or homozygous non-deleted in the isolated genomic DNA from a second cell population; and/or,
- at least three CND polymorphisms that are homozygous deleted in isolated genomic DNA from a second cell population and heterozygous or homozygous non-deleted in the isolated genomic DNA from the first cell population.

In an example, an informative marker of a genetically distinct cell population comprises:

- at least 4, at least 5, at least 6, at least 7, at least 8, at least 9, at least 10 CND polymorphisms that are homozygous deleted in isolated genomic DNA from the first cell population and heterozygous or homozygous non-deleted in the isolated genomic DNA from a second cell population; and/or,
- at least 4, at least 5, at least 6, at least 7, at least 8, at least 9, at least 10 CND polymorphisms that are homozygous deleted in isolated genomic DNA from a second cell population and heterozygous or homozygous non-deleted in the isolated genomic DNA from the first cell population.

In another example, the informative markers of the genetically distinct cell populations comprise at least 5, or at least 6, or at least 7, or at least 10, or at least 20, or at least 30, or at least 38 CND's. In another example, determining the level of isolated genomic DNA from the genetically distinct cell populations comprises subjecting the DNA to an amplification reaction with primers which target the CNV polymorphisms that are informative markers of the genetically distinct cell populations. In an example, the primers target an internal region of the informative CNV(s). In another example, the CNV polymorphisms are amplified using the primers shown in Table 7.

In another example, determining the level of isolated genomic DNA from a genetically distinct cell population comprises assessing the DNA with a quantitative amplification-independent detection means which target the CNV polymorphisms that are informative markers of the genetically distinct cell populations.

In another example, the CNV polymorphisms are selected from the CND polymorphisms listed in Table 1. In another example, the CNV polymorphisms are selected from the polymorphisms CND_01 to CND_10 listed in Table 1.

In an example, the level of isolated genomic DNA from the genetically distinct cell populations is determined by assessing the CNV polymorphisms that are informative markers of the genetically distinct cell populations using NGS, NanoString technology, droplet digital PCR, quantitative RT-PCR. For example, the level of isolated genomic DNA from the genetically distinct cell populations can be determined using droplet digital PCR.

In an example, the biological sample has been processed to substantially remove circulating cell free DNA from the sample. In another example, the biological sample is a blood sample. In another example, the biological sample is peripheral blood mononuclear cells. In another example, the biological sample is a purified immune cell population or purified mixture of immune cells. In another example, the immune cells are white blood cells. In another example, the biological sample is a purified population of T-cells, B-cells, granulocytes or a mixture thereof. In another example, the biological sample obtained from the subject is purified to provide at least two cell populations and chimerism is measured in each cell population. In an example, the two cell populations comprise B-cells and granulocytes . In another example, the biological sample obtained from the subject is purified to provide at least three cell populations and chimerism is measured in each cell population. In an example, the three cell populations comprise B-cells, granulocytes and T-cells. In another example, the biological sample is cord blood.

In an example, the genetically distinct cell populations comprise self cells (first cell population) and non-self cells (second cell population), for example foetal cells and maternal cells. In another example, the subject is a haematopoietic stem cell transplant (HSCT) recipient. In another example, in the methods of the present disclosure, the biological sample is obtained from a HSCT recipient and comprises a self cell population and a non-self cell population, and the level of non-self cells determines the level of engraftment in a HSCT recipient. In this example, a level of non-self cells greater than about 90% indicates engraftment of the transplant.

In another example, in the methods of the present disclosure, the biological sample is obtained from a HSCT recipient and comprises a self cell population and a non-self cell population and the level of self cells monitors the reestablishment of a haematopoietic stem cell transplant (HSCT) recipient's own bone marrow post HSCT transplant. In this example, preferably a level of self cells greater than about 10% indicates reestablishment of the HSCT recipient's bone marrow.

In an example, the methods of the present disclosure are performed daily, weekly, monthly, bi-monthly or every three months. In these examples, the methods of the present disclosure can be used to monitor levels of chimerism over time. In an example, an increase in the level of self cells and/or a decrease in the level of non-self cells indicates reestablishment of the HSCT recipient's bone marrow. In an example, monitoring chimerism over time may assist a clinician in administering or modifying a patients treatment or treatment regimen. In an example, an increase in the level of self cells and/or a decrease in the level of non-self cells indicates that immunosuppressive therapy should be administered to the HSCT recipient or the HSCT recipient's immunosuppressive therapy should be modified. In another example, the methods of the present disclosure encompass monitoring graft versus host disease (GVHD). In another example, in the methods of the present disclosure, the biological sample is obtained from a HSCT recipient and comprises a self cell population and a non-self cell population, and an increase in the level of self cells or a decrease in the level of non-self cells monitors minimal residual disease in a patient in need thereof.

In an example, the HSCT recipient received a HSCT for the treatment of a haematological disorder. In an example, the haematological disorder is a haematological malignancy. In an example, the haematological malignancy is leukaemia. In an example, a level of self cells greater than about 1% indicates relapse of the HSCT recipient's haematological disorder. In an example, an increase in the level of self cells and/or a decrease in the level of non-self cells indicates relapse of the HSCT recipient's haematological disorder. In an example, an increase in the level of self cells and/or a decrease in the level of non-self cells indicates that therapy for the haematological disorder should be administered to the HSCT recipient or the HSCT recipients therapy should be modified.

In another example, the genetically distinct cell populations comprise foetal cells (first cell population) and maternal cells (second cell population). In an example, the biological sample is cord blood. In another example, the methods of the present disclosure encompass identifying maternal contamination of a cord blood sample. In an example, a ratio of chimerism (foetal cells:maternal cells) greater than about 1:100, about 1:50, about 1:20, about 1:10, about 3:20 indicates the cord blood sample is not suitable for use in a haematopoietic stem cell transplant.

The present disclosure also provides a kit comprising the primers selected from the primers listed in Table 7 for use in the methods of the present disclosure.

In another example, the present disclosure relates to a method of measuring HSCT engraftment in a biological sample obtained from a HSCT transplant recipient, the method comprising: isolating the genomic DNA from the cells in the sample, determining the level of genomic DNA in the sample from the self and non-self cells based on copy number variation (CNV) polymorphisms that are informative markers of the self and non-self cells, determining the total level of genomic DNA isolated from the self and non-self cells in the sample, wherein the informative markers comprise:

- a CND polymorphism that is homozygous deleted in isolated genomic DNA from self cells and heterozygous or homozygous non-deleted in isolated genomic DNA from non-self cells; and
- a CND polymorphism that is homozygous deleted in the isolated genomic DNA from non-self cells and heterozygous or homozygous non-deleted in the isolated genomic DNA from self cells;

wherein the level of genomic DNA in the sample from the self and non-self cells and the total level of genomic DNA isolated from the self and non-self cells provides the measure of chimerism in the sample, and a level of genomic DNA in the sample from the non-self cells greater than about 90% indicates engraftment of the HSCT recipient's bone marrow.

In another example, the present disclosure relates to a method of measuring minimal residual disease in a biological sample obtained from a HSCT recipient, the method comprising: isolating the genomic DNA from the cells in the sample, determining the level of genomic DNA in the sample from self and non-self cells based on copy number variation (CNV) polymorphisms that are informative markers of the self and non-self cells, determining the total level of genomic DNA isolated from the self and non-self cells in the sample,
wherein the informative markers comprise:

- a CND polymorphism that is homozygous deleted in isolated genomic DNA from self cells and heterozygous or homozygous non-deleted in isolated genomic DNA from non-self cells; and
- a CND polymorphism that is homozygous deleted in the isolated genomic DNA from non-self cells and heterozygous or homozygous non-deleted in the isolated genomic DNA from self cells;

wherein the level of genomic DNA in the sample from the self and non-self cells and the total level of genomic DNA isolated from the self and non-self cells provides the measure of chimerism in the sample, and a level of self cells greater than about 1% indicates reestablishment of the HSCT recipient's bone marrow.

In another example, the present disclosure relates to a method of measuring maternal contamination in a cord blood sample comprising: isolating the genomic DNA from the cells in the cord blood sample, determining the level of genomic DNA in the sample from foetal and maternal cells based on copy number variation (CNV) polymorphisms that are informative markers of the foetal and maternal cells, determining the total level of genomic DNA isolated from the foetal and maternal cells in the sample,
wherein the informative markers comprise:

- a CND polymorphism that is homozygous deleted in isolated genomic DNA from foetal cells and heterozygous or homozygous non-deleted in isolated genomic DNA from maternal cells; and
- a CND polymorphism that is homozygous deleted in the isolated genomic DNA from maternal cells and heterozygous or homozygous non-deleted in the isolated genomic DNA from foetal cells;

wherein the level of genomic DNA in the cord blood sample from the foetal and maternal cells and the total level of genomic DNA isolated from the foetal and maternal cells provides the measure of chimerism in the cord blood sample, and a ratio of foetal cells:maternal cells greater than about 1:100, about 1:50, about 1:20, about 1:10, about 3:20 indicates the cord blood sample is not suitable for use in a haematopoietic stem cell transplant.

In particular, the present inventors have found that they are able to accurately measure chimerism in a blood sample containing self and non-self cells by isolating genomic DNA from the cells in the blood sample and then measuring the level of self DNA based on a CND polymorphism that is heterozygous or homozygous non-deleted in the self DNA and homozygous deleted in the non-self DNA and measuring the level of non-self DNA based on a CND that is heterozygous or homozygous non-deleted in the non-self DNA and homozygous deleted in the self DNA. Measuring the level of self DNA based on a CND polymorphism that is heterozygous or homozygous non-deleted in the non-self DNA and homozygous deleted in the self DNA and measuring the level of non-self DNA based on a CND that is heterozygous in the self DNA and homozygous deleted in the non-self DNA is advantageous as DNA levels can be assessed without encountering background influence from the self or non-self DNA.

Any example herein shall be taken to apply mutatis mutandis to any other example unless specifically stated otherwise.

The present invention is not to be limited in scope by the specific examples described herein, which are intended for the purpose of exemplification only.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or group of compositions of matter.

The invention is hereinafter described by way of the following non-limiting Examples and with reference to the accompanying drawings.

## BRIEF DESCRIPTION OF THE ACCOMPANYING DRAWINGS

**Figure 1:** Genotyping results obtained for 21 control samples.

**Figure 2:** Distributions of 'homozygous deleted' CND frequencies observed in three independent population cohorts.

**Figure 3:** Measurement linearity across the range and limit of detection using ddPCR of three representative target markers (CNV01B, CNV02B and CNV09B); each was spiked into DNA which was homozygous deleted for these markers to give the chimeric fractions indicated and measured in triplicate.

**Figure 4:** Intra- and inter-assay variation of 3 CNV markers measured in triplicate across a spiking series made by diluting DNA from an individual known to be heterozygous deleted for the respective marker into DNA from an individual known to be homozygous deleted.

**Figure 5:** Inter-assay variation (standard deviation, CV) of 8 informative donor CNV markers and 5 informative recipient CNV markers at each time point during longitudinal sampling from a clinical case in which chimerism analysis was used to guide decision making regarding immunosuppression management in graft-versus-host disease.

**Figure 6:** ddPCR absolute quantitative raw data.

**Figure 7:** Chimerism results of sample 1.

**Figure 8:** Chimerism results of sample 2.

**Figure 9:** Chimerism results of sample 3.

**Figure 10:** Chimerism results of sample 4. Contamination of pure donor DNA into pure recipient DNA samples for CND15 and CND3B detected as trace levels by the highly sensitive ddPCR/CND assays.

**Figure 11:** Correlation of CNV chimerism with previous STR analysis.

**Figure 12:** Comparison of chimerism measurements using the CNV method - (left panel) with the X/Y FISH method using a spiked series of male and female cells - (right panel) with the SNP/rtPCR method using DNA from 15 longitudinally collected blood samples from 4 transplant recipients.

**Figure 13:** Histograms of method informativity in 32 transplant recipients from 35 donors. In all individuals genotyped, at least one informative marker was present (left panel). Individuals were typically genotyped using panels of approximately 15 or 30 CNV markers, although in 5/67 (7%) cases 37-38 CNV markers were assayed to maximise the number of informative markers (middle panel). In the majority of individuals, 10-30% of tested markers were informative (right panel).

**Figure 14:** CND engraftment (A) and minimal residual disease (B) analysis in post-transplant samples.

**Figure 15:** CND analysis of post-transplant samples

**Figure 16:** CND analysis of cord blood sample

## DETAILED DESCRIPTION OF THE INVENTION

### General techniques and selected definitions

Unless specifically defined otherwise, all technical and scientific terms used herein shall be taken to have the same meaning as commonly understood by one of ordinary skill in the art (e.g., in molecular genetics, expression analysis, biochemistry, diagnostics).

As will be understood by those of skill in the art, various molecular techniques and DNA modification and detection methods utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al., Molecular Cloning: A Laboratory Manual, Cold Spring Harbour Laboratory Press (1989), T.A. Brown (editor), Essential Molecular Biology: A Practical Approach, Volumes 1 and 2, IRL Press (1991), D.M. Glover and B.D. Hames (editors), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbour Laboratory, (1988), and J.E. Coligan et al. (editors) Current Protocols in Immunology, John Wiley & Sons (including all updates until present).

Those skilled in the art will appreciate that the disclosure described herein is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features. For example, one of skill in the art would be aware of "linkage disequilibrium" which relates to the non-random association of alleles at two or more loci that descend from single, ancestral chromosomes. As outlined below the present disclosure describes a series of copy number variations (CNV), in particular, copy number deletions (CND) that can be used to determine the level of recipient or donor DNA in a biological sample. The CNVs of the present disclosure encompass related CNVs in linkage disequilibrium. Moreover, it is envisaged that determining the level of self or non-self DNA based on the CNVs of the present disclosure includes determining the level of self or non-self DNA by assessing other CNVs in linkage disequilibrium with the particular disclosed CNVs.

The term "and/or", e.g., "X and/or Y" shall be understood to mean either "X and Y" or "X or Y" and shall be taken to provide explicit support for both meanings or for either meaning.

As used herein, the term "about", unless stated to the contrary, refers to +/- 10%, more preferably +/- 5%, of the designated value.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

As used herein, the "patient" can be any organism from which a sample can be obtained comprising self and non-self cells. In an example, the patient is a mammal. The mammal may be a companion animal such as a dog or cat, or a livestock animal such as a horse or cow. In one example, the patient is a human. For example, the patient can be an adult. In another example, the patient can be a child. In another example, the patient can be an adolescent. Terms such as "patient", "subject" or "individual" are terms that can, in context, be used interchangeably in the present disclosure.

As used herein terms such as "self", "self nucleic acid" and "self DNA" are used to refer to nucleic acids and DNA from the subject from whom a biological sample has been obtained (e.g. HSCT recipient). Conversely, terms such as "non-self", "non-self nucleic acid" and "non-self DNA" are used to define nucleic acids and DNA that are foreign to the subject from whom the sample has been obtained (e.g. HSCT donor).

Throughout this specification, unless specifically stated otherwise or the context requires otherwise, the term DNA is used to refer to intracellular genomic DNA. For example, "self DNA" and "non-self DNA" refer to isolated genomic DNA from self and non-self cells in a biological sample.

In a HSCT transplant recipient blood sample, self DNA is from the transplant recipient and non-self DNA is from the transplant donor. It will be appreciated by those of skill in the art that transplant donor "non-self DNA" may comprise DNA from multiple individuals. For example, a transplant donor may have themselves previously received a transplant, in which their sample may also comprise non-self DNA. In this instance, a sample may comprise self DNA and multiple types of non-self DNA. It is envisaged that the methods of the present disclosure allow for the assessment of "self DNA" and multiple types of "non-self DNA" in a sample. In a cord blood sample, the self DNA is the foetal DNA whereas the non-self DNA is maternal DNA.

The methods of the present disclosure can be performed as an *in-vitro* assay. As one of skill in the art would appreciate, an assay is an investigative (analytic) procedure or method for qualitatively assessing or quantitatively measuring the presence or amount or the functional activity of a target.

In an example, a method or assay according to the present disclosure may be incorporated into a treatment regimen. For example, a method of treating a condition in a subject in need thereof may comprise performing an assay that embodies the methods of the present disclosure. In an example, a clinician or similar may wish to perform or request performance of an assay according to the present disclosure before administering or modifying treatment to a patient. For example, a clinician may perform or request performance of an assay according to the present disclosure on a HSCT recipient before electing to administer or modify therapy such as immunotherapy.

## Copy number variation (CNV) polymorphisms

When performing the methods of the present disclosure the level of isolated genomic DNA from a genetically distinct cell population is determined based on a copy number variation (CNV) polymorphism that is an informative marker of the cell population. A "CNV" polymorphism includes a copy number deletion (CND) an insertion or duplication. The term "informative marker" is used in the context of the present disclosure to refer to a marker that genetically distinguishes one cell population from another cell population in a biological sample. An informative marker allows for the level of isolated genomic DNA from the genetically distinct cell population to be determined (e.g. by using quantitative methods such as droplet digital PCR).

In an example, an informative marker can be a CNV polymorphism that is present in isolated genomic DNA from a first cell population that is not present in isolated genomic DNA from a second genetically distinct cell population in a biological sample. In an example, an informative marker can be a CNV polymorphism that is present in isolated genomic DNA from a self cell population that is not present in isolated genomic DNA from a non-self cell population in a biological sample. In another example, an informative marker can be at least two CNV polymorphisms, one that is present in isolated genomic DNA from a first cell population that is not present in the isolated genomic DNA from a second genetically distinct cell population in a biological sample and one that is not present in isolated genomic DNA from the first cell population that is present in the isolated genomic DNA from the second cell population.

CNV polymorphisms present in isolated genomic DNA may be referred to as "homozygous present", "homozygous not present" or "heterozygous". The term "homozygous present" is used in the context of the present disclosure when 2 copies of a CNV polymorphism are present (e.g., a diploid individual has a copy of the same CNV at a locus for each of two homologous chromosomes). The term "homozygous not present" is used in the context of the present disclosure when 2 copies of a CNV polymorphism are not present (e.g., a diploid individual has 0 copies of a CNV at a locus for each of two homologous chromosomes). The term "heterozygous" is used in the context of the present disclosure when 1-copy of a CNV polymorphism is present (e.g., a diploid individual with 1-copy each of two different alleles). Thus, the term "heterozygous" encompasses "heterozygous present" where 1-copy of a CNV polymorphism is present and "heterozygous not present" where 1-copy of a CNV polymorphism is not present.

In the context of a copy number deletion, the term "homozygous present" is used to refer to a genotype where 2 copies of the deletion are present (i.e. "homozygous deleted"; "2-copy deleted") while "homozygous not present" is used to refer to a genotype where 2 copies of the deletion are not present (i.e. "homozygous non-deleted"; "0-copy deleted"). The term "heterozygous" is used to refer to a genotype where 1-copy of the deletion is present (i.e. "heterozygous deleted"; "1-copy deleted").

In the context of a copy number duplication, "homozygous present" is used to refer to a genotype where 2 copies of the duplication are present (i.e. "homozygous duplicated"; 2-copy duplicated) while "homozygous not present" is used to refer to a genotype where 2 copies of the duplication are not present (i.e. "homozygous non-duplicated"; 0-copy duplicated). The term "heterozygous" is used to refer to a genotype where 1-copy of the duplication is present (i.e. "heterozygous duplicated"; 1-copy duplicated).

In an example, an informative CNV is homozygous present in a genetically distinct cell population. In another example, an informative CNV is heterozygous in a genetically distinct cell population. In another example, an informative CNV is homozygous not present in a genetically distinct cell population.

In an example, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 heterozygous CNV polymorphisms. In another example, informative markers of genetically distinct cell populations comprise is at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous present CNV polymorphisms. In other examples, informative markers of a genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous not present CNV polymorphisms.

In another example, an informative CNV polymorphism is heterozygous in a first population and homozygous present or homozygous not present in a genetically distinct cell population. In another example, an informative CNV polymorphism is homozygous present in a first population and homozygous not present or heterozygous in a genetically distinct cell population. In another example, an informative CNV polymorphism is homozygous not present in a first population and heterozygous, homozygous present in a genetically distinct cell population. In another example, an informative CNV polymorphism is heterozygous in a self population and homozygous deleted, homozygous present or homozygous not present in a non-self population. In another example, an informative CNV polymorphism is homozygous present in a self population and heterozygous or homozygous not present in a non-self population. In another example, an informative CNV polymorphism is homozygous present in a self population and heterozygous or homozygous not present in a non-self population.

In another example, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous present CNV polymorphisms that are homozygous not present in a genetically distinct cell population in the biological sample. In another example, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous present CNV polymorphisms that are heterozygous in a genetically distinct cell population in the biological sample. In another example, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 heterozygous CNV polymorphisms that are homozygous not present in a genetically distinct cell population in the biological sample.

In another example, at least one CNV is assessed in a biological sample to identify an informative marker. In other examples, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 CNV polymorphisms are assessed in a biological sample to identify an informative marker. For example, at least 4 CNV polymorphisms are assessed in a biological sample to identify an informative marker. For example, at least 6 CNV polymorphisms are assessed in a biological sample to identify an informative marker.

In an example, the CNV is a copy number deletion (CND) polymorphism. In an example, an informative CND is heterozygous deleted in a genetically distinct cell population. In another example, an informative CND is homozygous deleted in a genetically distinct cell population. In another example, an informative CND is homozygous non-deleted in a genetically distinct cell population.

For example, an informative marker can be a CND polymorphism that is homozygous deleted in isolated genomic DNA from a first cell population that is heterozygous or homozygous non-deleted in isolated genomic DNA from a second genetically distinct cell population in a biological sample. In an example, an informative marker is a CND polymorphism that is homozygous deleted in isolated genomic DNA from a self cell population that is heterozygous or homozygous non-deleted in isolated genomic DNA from a non-self cell population in a biological sample. In another example, an informative marker can be at least two CND polymorphisms, one that is homozygous deleted or heterozygous deleted in isolated genomic DNA from a first cell population that is homozygous non-deleted in isolated genomic DNA from a second genetically distinct cell population in a biological sample and one that is homozygous non-deleted in isolated genomic DNA from the first cell population that is homozygous deleted or heterozygous deleted in the isolated genomic DNA from the second cell population.

In other examples, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 heterozygous deleted CND polymorphisms. In another example, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous deleted CND polymorphisms.

In another example, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 heterozygous deleted CND polymorphisms that are homozygous deleted in a genetically distinct cell population in the biological sample. In another example, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 heterozygous deleted CND polymorphisms that are homozygous non-deleted in a genetically distinct cell population in the biological sample. In another example, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous deleted CND polymorphisms that are homozygous non-deleted in a genetically distinct cell population in the biological sample.

For example, informative CND polymorphisms can be heterozygous deleted in a first population and homozygous deleted or homozygous non-deleted in a genetically distinct cell population. In another example, informative CND polymorphisms can be homozygous deleted in a first population and heterozygous or homozygous non-deleted in a genetically distinct cell population. In another example, informative CND polymorphisms can be homozygous non-deleted in a first population and heterozygous or homozygous deleted in a genetically distinct cell population. In another example, informative CND polymorphisms can be heterozygous deleted in a self population and homozygous deleted or homozygous non-deleted in a non-self population. In another example, informative CND polymorphisms can be homozygous deleted in a self population and heterozygous deleted or homozygous non-deleted in a non-self population. In another example, informative CND polymorphisms can be homozygous non-deleted in a self population and homozygous deleted or heterozygous deleted in a non-self population.

In another example, an informative CND is heterozygous deleted in isolated genomic DNA from a first population of cells and homozygous deleted or homozygous non-deleted in isolated genomic DNA from a genetically distinct population of cells. In another example, an informative CND is homozygous deleted in isolated genomic DNA from a first population of cells and heterozygous or homozygous non-deleted in isolated genomic DNA from a genetically distinct population of cells. In another example, an informative CND is homozygous non-deleted in isolated genomic DNA from a first population of cells and heterozygous or homozygous deleted in isolated genomic DNA from a genetically distinct population of cells. For example, an informative CND is heterozygous deleted in isolated genomic DNA from self cells and homozygous deleted or homozygous non-deleted in isolated genomic DNA from non-self cells. For example, an informative CND is homozygous deleted in isolated genomic DNA from self cells and heterozygous or homozygous non-deleted in isolated genomic DNA from non-self cells. For example, an informative CND is homozygous non-deleted in isolated genomic DNA from self cells and heterozygous or homozygous deleted in isolated genomic DNA from non-self cells.

For example, an informative marker of a genetically distinct cell population can be between about 5 to 40, about 7 to 20, about 6 to 30, about 8 to 10 CND polymorphisms. In these examples the CNDs can be selected from Table 1. For example, an informative marker of a genetically distinct cell population can comprise 10 CND polymorphisms from Table 1. For example, informative markers can be selected from the group consisting of CND 01 - CND 10 from Table 1. Other informative markers comprising combinations of CNDs from Table 1 are discussed below.

In other examples, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38 heterozygous CND polymorphisms from Table 1. In other examples, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38 homozygous deleted CND polymorphisms from Table 1.

In other examples, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38 heterozygous deleted CND polymorphisms from Table 1 that are homozygous deleted or homozygous non-deleted in a genetically distinct cell population. In other examples, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38 homozygous deleted CND polymorphisms from Table 1 that are heterozygous deleted or homozygous non-deleted in a genetically distinct cell population. In other examples, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38 homozygous non-deleted CND polymorphisms from Table 1 that are heterozygous deleted or homozygous deleted in a genetically distinct cell population. In an example, the CND is not located at a HLA locus.

In an example, at least one CND is assessed in a biological sample to identify an informative marker. In other examples, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 CND polymorphisms are assessed in a biological sample to identify an informative marker. For example, at least 4 CND polymorphisms are assessed in a biological sample to identify an informative marker. For example, at least 6 CND polymorphisms are assessed in a biological sample to identify an informative marker.

Table 1:

| Na me | CNP_id | chr | start_hg18 | end_hg18 | Size (bp) | **homozygo us deleted frequency** (0-copy PCR product) | **heterozygo us deleted frequency** (1-copy PCR product) | **homozygo us non-deleted frequency** (2-copy PCR product) |
|---|---|---|---|---|---|---|---|---|
| CND 12 | CNVR3451.1 | Chr 7 | 73,467,042 | 73,469,172 | 2,130 | 0.55 | 0.33 | 0.12 |
| CND 13 | CNVR4596.1 | Chr 10 | 4,698,559 | 4,700,493 | 1,934 | 0.61 | 0.31 | 0.08 |
| CND 14 | CNVR801_full | Chr 2 | 54,419,132 | 54,420,976 | 1,844 | 0.59 | 0.37 | 0.04 |
| CND 15 | CNVR5853_full | Chr 13 | 38,831,627 | 38,833,387 | 1,760 | 0.55 | 0.33 | 0.12 |
| CND 16 | CNVR842.1 | Chr 2 | 76,627,234 | 76,628,943 | 1,709 | 0.6 | 0.33 | 0.08 |
| CND 17 | CNVR431.1 | Chr 1 | 185,731,458 | 185,733,106 | 1,648 | 0.62 | 0.33 | 0.04 |
| CND 18 | CNVR4203.1 | Chr 9 | 17,900,038 | 17,901,633 | 1,595 | 0.61 | 0.36 | 0.03 |
| CND 20 | CNVR952.1 | Chr 2 | 121,513,996 | 121,515,257 | 1,261 | 0.53 | 0.36 | 0.1 |
| CND 21 | CNVR1138.2 | Chr 2 | 219,761,436 | 219,762,572 | 1,136 | 0.39 | 0.42 | 0.18 |
| CND 22 | CNVR5923.1 | Chr 13 | 71,743,783 | 71,744,892 | 1,109 | 0.41 | 0.55 | 0.04 |
| CND 23 | CNVR8147.1 | Chr 22 | 33,975,445 | 33,976,472 | 1,027 | 0.45 | 0.43 | 0.12 |
| CND 24 | CNVR3004.1 | Chr 6 | 95,250,114 | 95,251,113 | 999 | 0.4 | 0.47 | 0.13 |
| CND 25 | 1343* | Chr 8 | 112,363,455 | 112,364,436 | 981 | 0.57 | 0.38 | 0.05 |
| CND 26 | CNVR381.1 | Chr 1 | 157,915,386 | 157,916,253 | 867 | 0.6 | 0.35 | 0.05 |

(continued)

| Name | CNP_id | chr | start_hg18 | end_hg18 | Size (bp) | **homozygo us deleted frequency** (0-copy PCR product) | **heterozygo us deleted frequency** (1-copy PCR product) | **homozygo us non-deleted frequency** (2-copy PCR product) |
|---|---|---|---|---|---|---|---|---|
| CND 27 | CNVR4374.1 | Chr 9 | 88,344,772 | 88,345,596 | 824 | 0.56 | 0.36 | 0.07 |
| CND 28 | CNVR2799.1 | Chr 6 | 18,510,099 | 18,510,860 | 761 | 0.41 | 0.44 | 0.15 |
| CND 29 | CNVR6540.1 | Chr 15 | 97392250 | 97392985 | 735 | 0.51 | 0.43 | 0.06 |
| CND 30 | CNVR6357.1 | Chr 15 | 37531690 | 37532136 | 446 | 0.52 | 0.42 | 0.07 |
| CND 31 | 158 | Chr 1 | 208148236 | 208150607 | 2,371 | 0.383 | 0.450 | 0.167 |
| CND 32 | CNVR358.1 | Chr 1 | 150822234 | 150856715 | 34,481 | 0.393 | 0.459 | 0.148 |
| CND 33 | CNVR217.1 | Chr 1 | 72538870 | 72584557 | 45,687 | 0.393 | 0.459 | 0.148 |
| CND 34 | CNVR7808.1 | Chr 20 | 21234267 | 21236529 | 2,262 | 0.516 | 0.418 | 0.066 |
| CND 36 | 554 | Chr 4 | 9823254 | 9844366 | 21,112 | 0.550 | 0.333 | 0.117 |
| CND 37 | CNVR2469.1 | Chr 5 | 57359283 | 57369499 | 10,216 | 0.615 | 0.361 | 0.025 |
| CND 38 | CNVR3009.1 | Chr 6 | 100141275 | 100141994 | 719 | 0.623 | 0.377 | 0.000 |
| CND 39 | CNVR3609.1 | Chr 7 | 147704059 | 147707263 | 3,204 | 0.656 | 0.311 | 0.033 |
| CND 41 | CNVR2304.1 | Chr 5 | 1977604 | 1978111 | 507 | 0.672 | 0.328 | 0.000 |
| CND 01 | CNVR376.1 | Chr 1 | 157,134,152 | 157,136,674 | 2,522 | 0.51 | 0.42 | 0.07 |

(continued)

| Name | CNP_id | chr | start_hg18 | end_hg18 | Size (bp) | **homozygo us deleted frequency** (0-copy PCR product) | **heterozygo us deleted frequency** (1-copy PCR product) | **homozygo us non-deleted frequency** (2-copy PCR product) |
|---|---|---|---|---|---|---|---|---|
| CND 02 | CNVR7344.1 | Chr 18 | 53,097,735 | 53,099,702 | 1,967 | 0.52 | 0.4 | 0.08 |
| CND 03 | CNVR4014.1 | Chr 8 | 112,363,260 | 112,365,469 | 2,209 | 0.5 | 0.41 | 0.08 |
| CND 04 | CNVR6084.1 | Chr 14 | 21,951,540 | 21,952,070 | 530 | 0.46 | 0.43 | 0.11 |
| CND 05 | CNVR6676.1 | Chr 16 | 25,247,611 | 25,250,595 | 2,984 | 0.45 | 0.47 | 0.08 |
| CND 06 | CNVR3319.1 | Chr 7 | 24,004,755 | 24,006,584 | 1,829 | 0.47 | 0.38 | 0.15 |
| CND 07 | CNVR5850.1 | Chr 13 | 37,955,318 | 37,958,191 | 2,873 | 0.43 | 0.43 | 0.14 |
| CND 08 | CNVR3753_full | Chr 8 | 4,110,308 | 4,112,335 | 2,027 | 0.43 | 0.43 | 0.13 |
| CND 09 | CNVR7301.1 | Chr 18 | 33,560,073 | 33,560,645 | 572 | 0.42 | 0.45 | 0.12 |
| CND 10 | CNVR4886.1 | Chr 10 | 108,020,303 | 108,022,518 | 2,215 | 0.41 | 0.42 | 0.17 |
| CND 19 | CNVR1037.1 | Chr 2 | 159,668,040 | 159,669,209 | 1,169 | 0.5 | 0.43 | 0.07 |

In another example, the CNV is a copy number duplication polymorphism. In an example, an informative copy number duplication is homozygous duplicated in a genetically distinct cell population. In another example, an informative copy number duplication is heterozygous duplicated in a genetically distinct cell population. In another example, an informative copy number duplication is homozygous non-duplicated in a genetically distinct cell population. In other examples, informative markers of genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 heterozygous duplicated copy number duplication polymorphisms. In other examples, informative markers of a genetically distinct cell populations comprise at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous duplicated copy number duplication polymorphisms. In another example, an informative marker of a genetically distinct cell population is at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous duplicated copy number duplication polymorphisms that are heterozygous or homozygous non-duplicated in a genetically distinct cell population in the biological sample. In another example, an informative marker of a genetically distinct cell population is at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 homozygous non-duplicated copy number duplication polymorphisms that are heterozygous or homozygous duplicated in a genetically distinct cell population in the biological sample. In another example, an informative marker of a genetically distinct cell population is at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 heterozygous duplicated copy number duplication polymorphisms that are homozygous non-duplicated or homozygous duplicated in a genetically distinct cell population in the biological sample.

For example, informative copy number duplication polymorphisms can be heterozygous duplicated in a first population and homozygous duplicated or homozygous non-duplicated in a genetically distinct cell population. In another example, informative copy number duplication polymorphisms can be homozygous duplicated in a first population and heterozygous or homozygous non-duplicated in a genetically distinct cell population. In another example, informative copy number duplication polymorphisms can be homozygous non-duplicated in a first population and heterozygous or homozygous duplicated in a genetically distinct cell population. In another example, informative copy number duplication polymorphisms can be heterozygous in a self population and homozygous duplicated or homozygous non-duplicated in a non-self population. In another example, informative copy number duplication polymorphisms can be homozygous duplicated in a self population and heterozygous duplicated or homozygous non-duplicated in a non-self population. In another example, informative copy number duplication polymorphisms can be homozygous non-duplicated in a self population and heterozygous duplicated or homozygous duplicated in a non-self population.

In an example, at least one copy number duplication is assessed in a biological sample to identify an informative marker. In other examples, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45 copy number duplication polymorphisms are assessed in a biological sample to identify an informative marker. For example, at least 4 copy number duplication polymorphisms are assessed in a biological sample to identify an informative marker. For example, at least 6 copy number duplication polymorphisms are assessed in a biological sample to identify an informative marker.

In an example, an informative CNV polymorphism is at least about 0.5kb - 100 mega bases (Mb) in size. In another example, an informative CNV polymorphism is at least about 1kb - 50Mb in size. In another example, an informative CNV polymorphism is at least about 1.5kb - 25Mb in size. In another example, an informative CNV polymorphism is at least about 2kb - 10Mb in size. In another example, an informative CNV polymorphism is at least about 2.5kb - 1Mb in size. In another example, an informative CNV polymorphism is less than about 5kb, about 4.5kb, about 4kb, about 3.5kb, about 3kb, about 2.5kb, about 2kb, about 1.5kb, about 1kb, about 0.5kb in size. In an example, an informative CNV polymorphism is less than 3kb in size. In another example, all informative CNV's are less than 3kb in size.

In an example, an informative CNV is polymorphic. In another example, an informative CNV has no known intrinsic clinical significance.

In an example, an informative CNV has a zero copy allele frequency of greater than about 0.1, about 0.2, about 0.3, about 0.4, about 0.5, about 0.6, about 0.7, about 0.8, about 0.9.

In other examples, an informative CND polymorphism can include deletions of from 50bp to 100 Mb. Accordingly, in an example, a CND can be used in the context of the present disclosure to describe losses or gains of genetic segments more than 50 base pairs long. In other examples, a CND can include deletions of at least about 50, about 60, about 70, about 80, about 90 about 100bp, about 10, about 20, about 30, about 40, about 50, about 60, about 70, about 80, about 90, about 100, about 200, about 300, about 400, about 500, about 600, about 700, about 800, about 900 or about 1,000kb. In another example, a CND polymorphism can include deletions of at least about 1, about 2, about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 51, about 52, about 53, about 54, about 55, about 56, about 57, about 58, about 59, about 60, about 61, about 62, about 63, about 64, about 65, about 66, about 67, about 68, about 69, about 70, about 71, about 72, about 73, about 74, about 75, about 76, about 77, about 78, about 79, about 80, about 81, about 82, about 83, about 84, about 85, about 86, about 87, about 88, about 89, about 90, about 91, about 92, about 93, about 94, about 95, about 96, about 97, about 98, about 99, or about 100Mb.

One of skill in the art could easily identify CNV polymorphisms that are informative markers of genetically distinct cell populations. For example, one of skill in the art could use any one or all of the primer sets listed in Table 7 to identify informative markers in genomic DNA isolated from a biological sample. Informative CNV polymorphisms can be identified in various biological samples. For example, informative CNV polymorphisms may be identified in DNA obtained from a fluid sample. For example, an informative CNV may be identified by assessing DNA isolated from a blood sample.

In an example, informative CNV polymorphisms in self and/or non-self DNA can be identified by comparison with DNA obtained from a sample free of chimerism. In an example, informative CNV polymorphisms in self DNA are identified by comparison with DNA obtained from a known self-cell population. In another example, informative CNV polymorphisms in non-self DNA are identified by comparison with DNA obtained from a known non-self cell population. Exemplary samples from which known self or non-self cells can be obtained include biopsy and resection material, hemolysate, lymph, synovial fluid, spinal fluid, urine, semen, stool, sputum, mucus, amniotic fluid, lacrimal fluid, cyst fluid, sweat gland secretion, bile, milk, tears or saliva. In another example, known self or non-self cells can be obtained can be buccal cells obtained via cheek swab.

## Genomic chimerism

The present disclosure relates to a method of measuring "chimerism" in a biological sample obtained from a subject. The term "chimerism" is used in the context of the present disclosure to describe the coexistence of genetically distinct cell populations in a biological sample obtained from a subject. For example, the term "chimerism" encompasses the coexistence of self and non-self cell populations in a biological sample obtained from a subject. Various examples of chimerism are known in the art. For example, chimerism can exist in a subject that has received a HSCT. In this example, genetically distinct cell populations in a biological sample from a subject that has received a HSCT include a cell population from the HSCT donor and a cell population from the HSCT recipient. Foetal-maternal chimerism is another example. In the context of foetal-maternal chimerism, genetically distinct cell populations can co-exist in a biological sample obtained from the umbilical cord of a foetus. For example, genetically distinct cell populations in a biological sample obtained from the umbilical cord can comprise a cell population from the mother and a cell population from the foetus.

The term chimerism is not intended to be limited to the coexistence of two genetically distinct populations of cells. For example, chimerism can exist in a subject that has received a HSCT from a donor who themselves has received a previous HSCT. In this example, the genetically distinct cell populations in a biological sample obtained from this subject can comprise a cell population from the HSCT donors donor, a cell population form the HSCT donor, and a cell population from the HSCT recipient. Thus, in an example, a biological sample can comprise at least 3 genetically distinct cell populations. In other examples, the biological sample can comprise at least 4, at least 5, at least 6, at least 7, at least 8, at least 9, at least 10 genetically distinct cell populations. In an example, genetically distinct cell populations can be characterised as a first population and a second population. In another example, genetically distinct cell populations can be characterised as a first population, a second population and any one or more of a third, fourth, fifth, sixth, seventh, eighth, ninth or tenth population.

The term, "genetically distinct" is used in the context of the present disclosure to distinguish two cell populations on the basis of their genomic make up. Thus, genetically distinct cell populations have different genotypes. For example, non-self cells are genetically distinct from self cells. For example, cells from a bone marrow transplant donor (non-self cells) are genetically distinct from the bone marrow transplant recipients cells (self cells). In another example, foetal cells are genetically distinct from maternal cells.

In an example, cell populations are characterised as being genetically distinct based on the presence or absence of CNV polymorphisms. For example, two genetically distinct cell populations are distinguished from each other on the basis that one cell population comprises a CNV polymorphism in its isolated genomic DNA that is not present in the isolated genomic DNA from the other cell population. In another example, two genetically distinct cell populations are distinguished from each other on the basis that one cell population comprises at least two, at least three, at least four, at least five, at least six, at least seven, at least eight, at least nine, at least ten, at least 15, at least 20, at least 25, at least 30, at least 35, at least 38 CNV polymorphisms in its isolated genomic DNA that are not present in the isolated genomic DNA from the other cell population. In another example, two genetically distinct cell populations are distinguished from each other on the basis of at least two CNV polymorphisms, one CNV being present in isolated genomic DNA from the first cell population and not present in the isolated genomic DNA from the second cell population and one CNV being not present in isolated genomic DNA from the first cell population and present in the isolated genomic DNA from the second cell population.

In another example, two genetically distinct cell populations can be distinguished from each other on the basis of at least three CNV polymorphisms, two CNVs being present in isolated genomic DNA from the first cell population and not present in the isolated genomic DNA from the second cell population and one CNV not present in isolated genomic DNA from the first cell population that is present in the isolated genomic DNA from the second cell population. In another example, two genetically distinct cell populations can be distinguished from each other on the basis of at least four CNV polymorphisms, two CNVs being present in isolated genomic DNA from the first cell population and not present in the isolated genomic DNA from the second cell population and two CNVs not present in isolated genomic DNA from the first cell population and present in the isolated genomic DNA from the second cell population. It follows that genetically distinct cell populations can be distinguished based on various other combinations of CNVs.

Measure of chimerism

In an example, the present disclosure relates to a method of measuring chimerism in a biological sample obtained from a subject. In a method of measuring chimerism in a sample from a subject comprising two of more genetically distinct cell populations according to the invention, the level of genomic DNA in the sample from said first genetically distinct cell population and the total level of genomic DNA isolated from the genetically distinct populations provides the measure of chimerism. In an example, the level of isolated genomic DNA from a genetically distinct cell population provides the measure of chimerism in a biological sample. In the context of the present disclosure, the isolated genomic DNA will be predominately from "intracellular genomic DNA" isolated from intact cells within the sample. The term "intracellular genomic DNA" is used in contrast to the term circulating cell-free DNA (ccfDNA), which may be present in an individual's blood plasma. ccfDNA is released into an individual's blood from apoptotic and necrotic cells as they breakdown. The term "intracellular genomic DNA chimerism" is used in the context of the present disclosure to refer to a situation in which a mixture of genetically distinct (e.g. "self' and "non-self") DNA is present in DNA isolated from cells in an biological sample obtained from a subject. When measuring intracellular DNA chimerism, isolated DNA from genetically distinct cell populations such as self and non-self cells is assessed. The term "plasma DNA chimerism" refers to the situation in which mixtures of genetically distinct (e.g. "self' and "non-self') circulating cell-free DNA (ccfDNA) are present in an individual's blood plasma. For the avoidance of doubt the term "intracellular DNA chimerism" does not encompass "plasma DNA chimerism" and *vice versa.*

In another example, the number of cells in a genetically distinct cell population in a biological sample provides the measure of chimerism in the sample. Diploid cells generally contain a known quantity of DNA. Thus, in this example, the level of isolated DNA from the genetically distinct cell population can be used to calculate the number of genetically distinct cells in the population. For example, if the level of genomic DNA from the HSCT donor (i.e. a first genetically distinct cell population) is 80% of the total DNA in the biological sample and the level of genomic DNA from the HSCT recipient (i.e. a second genetically distinct cell population) is 20% of the total DNA in the sample, then the measure of chimerism in the sample can be expressed as comprising 80% donor cells (non-self) and 20% recipient cells (self). In an example, the measure of chimerism in the sample in turn provides a level of chimerism in the subject, which allows an assessment of HSCT transplant success (e.g engraftment) or failure (e.g. transplant rejection).

In another example, referring to measuring chimerism in a cord blood sample, if the level of genomic DNA from the foetus (i.e. a first genetically distinct cell population) is 98% of the total DNA in the biological sample and the level of genomic DNA from the mother (i.e. a second genetically distinct cell population) is 2% of the total DNA in the sample, then the measure of chimerism in the sample can be expressed as comprising 98% foetal cells and 2% maternal cells. In an example, the measure of chimerism in the sample in turn provides a level of chimerism in the cord blood sample, which indicates the level of maternal contamination in the cord blood.

In another example, the levels of isolated DNA from two or more genetically distinct cell populations are compared to provide a measure of chimerism in the sample. In these examples, the total level of genomic DNA isolated from the genetically distinct cells can be determined. For example, the total level of DNA can be determined based on a non-polymorphic target sequence. A "non-polymorphic target sequence" is used in the context of the present disclosure to refer to a homozygous two-copy control. Put another way, two copies of the non-polymorphic target sequence are present in the genetically distinct cell populations. For example, the total level of DNA can be determined based on a target sequence within the gene encoding Angiotensin I converting enzyme (e.g. #NM_000789).

In another example, the total level of DNA is determined based on a homozygous polymorphism that is present in the non-self DNA and the self DNA. For example, the total level of DNA can be determined based on a homozygous CNV polymorphism that is present in the non-self DNA and the self DNA.

In another example, the level of self DNA is compared with the total level of DNA to provide a ratio of self DNA/total DNA. Thus, in an example:

Level of self DNA / Total DNA =
Level of self DNA : Total DNA

In another example, the level of non-self DNA is compared with the total level of DNA to provide a ratio of non-self DNA/total DNA. Thus, in an example:

Level of non-self DNA / Total DNA =
Level of non-self DNA : Total DNA

In another example, the level of non-self DNA is compared with the level of self DNA to provide a ratio of non-self DNA/self DNA. Thus, in an example:

Level of non-self DNA / self DNA =
Level of non-self DNA : self DNA

In another example, the level of self DNA and non-self DNA are compared with the total level of DNA to provide a ratio of self DNA/total DNA and a ratio of non-self DNA/total DNA. In another example, the level of self DNA can be expressed as a percentage of total DNA. In another example, the level of non-self DNA can be expressed as a percentage of total DNA.

In an example, the methods of the present disclosure can include an internal validation step. For example, the level of isolated genomic DNA from a first cell population and the level of isolated genomic DNA from a second genetically distinct cell population can be validated via comparison with the level of total DNA in the sample. In an example, the level of isolated genomic DNA from a first cell population and the level of isolated genomic DNA from a second genetically distinct cell population are validated when:

$$\text{Level of isolated DNA from the first cell population} + \text{Level of isolated DNA from the second cell population are about} = \text{Total DNA}$$

In this example, when the level of DNA from the first population + the level of DNA from the second cell population does not equal the level of total DNA, the result can indicate that an additional genetically distinct cell population is present in the sample.

In an example,

- the level of isolated DNA from a first cell population is determined based on an informative CNV polymorphism;
- the level of isolated DNA from a second cell population is determined based on an informative CNV polymorphism; and
- the level of total DNA is measured based on a non-polymorphic target sequence such as Angiotensin I converting enzyme,

wherein the levels of DNA from the first and second cell populations are validated when the sum of the levels DNA are about equal to the level of total DNA.

In another example,

- the level of isolated DNA from a first cell population is determined based on a CND polymorphism that is heterozygous or homozygous non-deleted in isolated DNA from a first cell population and homozygous deleted in isolated DNA from a second genetically distinct cell population;
- the level of isolated DNA from the second cell population is determined based on a CND polymorphism that is homozygous deleted in isolated DNA from a first cell population and heterozygous or homozygous non-deleted in isolated DNA from a second genetically distinct cell population; and
- the level of total DNA is measured based on a non-polymorphic target sequence such as Angiotensin I converting enzyme,

wherein the levels of DNA from the first and second cell populations are validated when the sum of the levels of DNA are about equal to the level of total DNA.

In another example, when the total level of DNA is calculated based on a homozygous CNV that is present in the isolated genomic DNA from the first and second cell populations, a level of isolated DNA from the first cell population + level of isolated DNA from the second cell population that does not equal about the level of total DNA can also indicate that the levels of DNA are inaccurate.

In another example,

- the level of isolated DNA from a first cell population is determined based on an informative CNV polymorphism;
- the level of isolated DNA from a second cell population is determined based on an informative CNV polymorphism; and
- the level of total DNA is measured based on a CNV polymorphism that is homozygous present in isolated genomic DNA from both the first and second cell populations,

wherein the levels of DNA are validated when the sum of the levels is about equal to the level of total DNA.

In another example,

- the level of isolated DNA from a first cell population is determined based on a CND polymorphism that is heterozygous or homozygous non-deleted in isolated DNA from a first cell population and homozygous deleted in isolated DNA from a second genetically distinct cell population;
- the level of isolated DNA from the second cell population is determined based on a CND polymorphism that is homozygous deleted in isolated DNA from a first cell population and heterozygous or homozygous non-deleted in isolated DNA from a second genetically distinct cell population; and
- the level of total DNA is measured based on a CNV polymorphism that is homozygous present in isolated genomic DNA from both the first and second cell populations,

wherein the levels of DNA are validated when the sum of the levels is about equal to the level of total DNA.

In these examples, the levels of isolated DNA from the first and second cell populations may be inaccurate when the combined levels of DNA do not equal about the total level of DNA. For example, other genetically distinct cell populations may be present in the sample.

In another example, the level of isolated DNA from two or more genetically distinct cell populations is used to calculate the number of cells in those populations. In an example, the number of genetically distinct cells in each population are compared to provide a comparative measure of chimerism in the sample.

In an example, the cell population numbers in the biological sample are compared to provide a percentage of chimerism. For example, the number of non-self and self cells can be compared to provide a percentage of chimerism. In an example, the cell populations in the biological sample are compared to provide a ratio. For example, the number of self and non-self cells in the biological sample can be compared to represent the measure of chimerism as a ratio of self cells:non-self cells. Thus, in an example:

$$\text{Number of self cells / Number of non-self cells} = \text{Number of self cells: Number of non-self cells}$$

In another example, the level of total DNA can be used to calculate the total number of cells in the sample.

Determining the level of genomic DNA

In an example, the level of isolated genomic DNA from a genetically distinct cell population is determined based on the informative CNV polymorphisms discussed above. Levels of DNA can be determined based on these CNVs using various methods known in the art. For example, levels of DNA can be determined via amplification reaction using primers which target a desired CNV or via amplification independent detection means. In an example, determining the level of DNA comprises subjecting isolated DNA to an amplification reaction with primers which target an informative CND polymorphism. Once an informative CNV is identified, one of skill in the art can readily design primers that target the region. For example, a primer design tool such as PrimerQuest (Integrated DNA Technologies) could be used to generate suitable primers based on the nucleic acid sequence of an informative CNV. In an example, levels of DNA can be determined via amplification reaction using primers which target an internal region of an informative CNV. In another example, levels of DNA can be determined via amplification reaction using primers which target the boundary of an informative CNV. In another example, levels of DNA can be determined via amplification reaction using primers which target a region external to an informative CNV and primers which target an internal region of an informative CNV. One of skill in the art will appreciate that in this example, a heterozygous deleted CND will provide a 1-copy PCR product, a homozygous deleted CND will provide a 0-copy PCR product and a homozygous non-deleted CND will provide a 2-copy PCR product. In another example, levels of DNA can be determined via amplification reaction using primer sets which target opposing sides of a CNV boundary (ie. a region immediately external to an informative CNV "external CNV boundary" and a region immediately internal to an informative CNV "internal CNV boundary". In another example, levels of DNA can be determined via amplification reaction using primers which target an internal region of an informative CND. In another example, levels of DNA can be determined via amplification reaction using primers which target the boundary of an informative CND. In another example, levels of DNA can be determined via amplification reaction using primers which target a region external to an informative CNV and primers which target an internal region of an informative CND. In another example, levels of DNA can be determined via amplification reaction using primer sets which target opposing sides of a CND boundary (ie. a region immediately external to an informative CND "external CND boundary" and a region immediately internal to an informative CND "internal CND boundary". In another example, the DNA levels can be determined based on amplification of informative CND polymorphisms using the primers shown in Table 7. Exemplary amplification based detection methods include droplet digital PCR and quantitative RT-PCR.

One of skill in the art will appreciate that identifying a CND polymorphism that is heterozygous or homozygous non-deleted in a first cell population and homozygous deleted in a genetically distinct cell population is advantageous as the level of isolated genomic DNA from the first cell population can be determined without background amplification of the isolated DNA from the genetically distinct cell population ("zero background"). It is also advantageous to identify a CND polymorphism that is heterozygous or homozygous non-deleted in the second cell population and homozygous deleted in the first population as the level of isolated genomic DNA from the second cell population can also be determined with zero background. Accordingly, in an example, the level of DNA in a first cell population is determined based on at least one, at least 2, at least 3, at least 4, at least 5, at least 6, at least 7, at least 8, at least 9, at least 10 CND polymorphisms that are homozygous deleted in isolated genomic DNA from the genetically distinct cell population and heterozygous or homozygous non-deleted in isolated genomic DNA from the first population. In another example, the level of DNA in a second cell population is determined based on at least one, at least 2, at least 3, at least 4, at least 5, at least 6, at least 7, at least 8, at least 9, at least 10 CND polymorphisms that are homozygous deleted in isolated genomic DNA from the first population and heterozygous or homozygous non-deleted in isolated genomic DNA from the genetically distinct cell population. In these examples, the level of genomic DNA can be determined using primers which target an internal region of informative CNDs.

In another example, determining the level of DNA comprises assessing the DNA with a quantitative amplification-independent detection means which target informative CNV polymorphisms. For example, the level of DNA can be determined by assessing an informative CNV polymorphism using methods such as next generation sequencing (NGS), massive parallel sequencing and NanoString technology. One of skill in the art can determine the quantity of isolated genomic DNA from genetically distinct cell populations based on the signal strength of the CNV (e.g. as measured by quantitative amplification or via amplification-independent NGS or NanoString technology). For example DNA concentration can be calculated using the formula described in Lo et al. Am J HumGenet. 62:768 1998.

Sample preparation and analysis

Chimerism is assessed using the methods of the present disclosure on a biological sample isolated from a subject. It is considered that terms such as "biological sample" and "specimen" are terms that can, in context, be used interchangeably in the present disclosure. In an example, the sample is isolated from a human. For example, the sample can be isolated from a child, adolescent or adult subject. In an example, the subject is a HSCT recipient. In an example, the subject received a bone marrow transplant. In another example, the subject received a cord blood transplant.

In the present disclosure, any cellular material can be used as the above-mentioned biological sample so long as it can be collected from a subject and DNA can be isolated from cells and analysed according to the methods of the present disclosure. For example, the sample may be a fluid sample. For example, the sample can be a blood sample (e.g. isolated venous blood). In an example, the sample is a blood sample containing self and non-self cells.

The term "blood sample" is used in the context of the present disclosure to refer to a sample of whole blood or a sub-population of cells in whole blood. Sub-populations of cells in whole blood include platelets, red blood cells (erythrocytes), platelets and white blood cells (i.e., peripheral blood leukocytes, which are made up of neutrophils, lymphocites, eosinophils, basophils and monocytes). These five types of white blood cells can be further divided into two groups, granulocytes (which are also known as polymorphonuclear leukeocytes and include neutrophils, eosinophils and basophils) and mononuclear leukocytes (which include monocytes and lymphocytes). Lymphocytes can be further divided into T-cells, B-cells and NK cells. The blood sample may be treated to remove whole cells such as by centrifugation, affinity chromatography (e.g. immunoabsorbent means) and filtration.

In an example, the sample can comprise a specific cell type or mixture of cell types isolated directly from the subject or purified from a sample obtained from the subject. For example, the sample may be peripheral blood mononuclear cells (pBMC). In another example, any of the above referenced cell types can be purified from a blood sample and assessed using the methods of the present disclosure. For example, genomic DNA levels can be assessed in T-cells. In another example, genomic DNA levels can be assessed in B-cells. In another example, genomic DNA levels can be assessed in granulocytes. In another example, genomic DNA levels can be assessed in T-cells, B-cells and NK cells obtained from a subject's blood sample. In another example, genomic DNA levels can be assessed in T-cells, B-cells and granulocytes obtained from a subject's blood sample. Various methods of purifying sub-populations of cells are known in the art. For example, pBMC can be purified from whole blood using various known Ficoll based centrifugation methods (e.g. Ficoll-Hypaque density gradient centrifugation). Methods of purifying pBMC from whole blood are also exemplified below. Other cell populations can be immunoselected based on their expression of various cell surface markers using techniques such as fluorescence-activated cell sorting (FACS) or magnetic bead based separation techniques. For example, T-cells can be selected based on their expression of CD3 and CD4 and/or CD8, B-cells can be selected based on their expression of CD19 and NK cells can be selected based on their expression of CD16 and/or CD56. In other examples, monocytes can be purified based on their expression of CD14. In another example, T-cells and granulocytes are purified based on expression of CD3 and CD19, T-cells being CD3+CD19+ and granulocytes being CD3-CD19-.

In another example, the biological sample is purified or processed to remove circulating cell free nucleic acids before isolating genomic DNA from cells. For example, the plasma can be purified from the biological sample using centrifugation. In another example, a whole blood sample can be obtained from a subject and the serum can be removed via centrifugation after clotting.

DNA is extracted from the cells in the biological sample for analysis. In an example, the DNA extracted from the cells is genomic DNA. Various methods of isolating DNA, in particular genomic DNA from cells are known to those of skill in the art. In general, known methods involve disruption and lysis of the starting material followed by the removal of proteins and other contaminants and finally recovery of the DNA. For example, techniques involving alcohol precipitation; organic phenol/chloroform extraction and salting out have been used for many years to extract and isolate DNA. One example of DNA isolation is exemplified below (e.g. Qiagen All-prep kit). However, there are various other commercially available kits for genomic DNA extraction (e.g. Life technologies; Qiagen). Purity and concentration of DNA can be assessed by various methods, for example, spectrophotometry.

<u>Detecting genetically distinct cell populations in a biological samples</u>

HSCT provides a recipient with at least two genetically distinct cell populations (e.g. self and non-self cells). Prior to HSCT a recipients bone marrow (self-cell population) is ablated. For example subjects generally receive intensive chemotherapy and/or radiation prior to administration of HSCT. The ablated bone marrow is replaced by donor bone marrow (non-self cells). The donor bone marrow may then begin producing non-self cells such a blood cells. This process is known as engraftment. In an example, the methods of the present disclosure are directed towards determining the level of engraftment in a HSCT recipient. For example, the methods of the present disclosure can be used to determine the level of engraftment in a HSCT recipient by determining the level of non-self cells in a biological sample obtained from a HSCT recipient. Thus, in an example, the methods of the present disclosure are directed towards a method of determining the level of engraftment in a HSCT recipient, wherein a biological sample is obtained from the HSCT recipient and the level of isolated genomic DNA from a genetically distinct cell population based on a copy number variation (CNV) polymorphism that is an informative marker of the genetically distinct cell population is determined, wherein the level of isolated genomic DNA from the genetically distinct cell population provides the measure of engraftment in the HSCT recipient. In this example, the genetically distinct cell population can be genetically distinct from the subject. For example, the genetically distinct cell population can consist of non-self cells. In another example, the genetically distinct cell population can be self-cells.

In an example, the levels of isolated genomic DNA from self and non-self cells are compared to provide the measure of engraftment in the HSCT recipient. In another example, the level of self DNA and/or the level non-self DNA are compared with the total level of DNA to provide a percentage of engraftment.
For example, the percentage of non-self cells to total cells provides the percentage of engraftment. In various examples, a percentage of non-self cells greater than about 60%, about 65%, about 70%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% indicates engraftment of the HSCT. In another example, 100% non-self cells indicates complete engraftment. In another example, a percentage of self cells less than about 40%, about 35%, about 30%, about 25%, about 24%, about 23%, about 22%, about 21%, about 20%, about 19%, about 18%, about 17%, about 16%, about 15%, about 14%, about 13%, about 12%, about 11%, about 10%, about 9%, about 8%, about 7%, about 6%, about 5%, about 4%, about 3%, about 2%, about 1% indicates engraftment of the HSCT. In another example, 0% self cells indicates complete engraftment.

In another example, the level of self DNA and/or the level non-self DNA are compared with the total level of DNA to provide a ratio of engraftment. In an example, a ratio of self cells:non-self cells greater than about 3:20; about 1:10; about 1:20, about 1:50; about 1:100 indicates engraftment in a HSCT subject.

In another example, the methods of the present disclosure can be used to measure reestablishment of a HSCT recipients own bone marrow (i.e. reestablishment of self cells). For example, the methods of the present disclosure are directed towards a method of determining the level of reestablishment of the HSCT recipients bone marrow, wherein a biological sample is obtained from the HSCT recipient and the level of isolated genomic DNA from a genetically distinct cell population based on a copy number variation (CNV) polymorphism that is an informative marker of the genetically distinct cell population is determined, wherein the level of isolated genomic DNA from the genetically distinct cell population indicates whether the HSCT recipients bone marrow is restabilised. In this example, the genetically distinct cell population can be genetically distinct from the subject. For example, the genetically distinct cell population can consist of non-self cells. In another example, the genetically distinct cell population can be self-cells.

In an example, the levels of isolated genomic DNA from self and non-self cells are compared to provide the measure of engraftment in the HSCT recipient.

In another example, the level of self DNA and/or the level non-self DNA are compared with the total level of DNA to provide a percentage of bone marrow reestablishment. For example, the percentage of self cells to total cells provides the percentage of bone marrow reestablishment. In another example, the percentage of non-self cells provides the percentage of bone marrow reestablishment. In various examples, a percentage of self cells greater than about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 30%, about 35%, about 40% indicates reestablishment of the HSCT recipients bone marrow. In another example, a percentage of non-self cells less than about 60%, about 65%, about 70%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99% indicates reestablishment of the HSCT recipients bone marrow. In another example, the level of self DNA and/or the level non-self DNA are compared with the total level of DNA to provide a ratio of reestablishment of the HSCT recipients bone marrow. In an example, a ratio of self cells:non-self cells greater than about 3:20; about 1:10; about 1:20, about 1:50; about 1:100 indicates reestablishment of the HSCT recipients bone marrow.

The methods of the present disclosure can be used to measure chimerism in subjects that have received HSCT for various reasons. In an example, the subject received the HSCT for treatment of a haematological disorder. In an example, the subject received the HSCT for treatment of a haematological malignancy. Exemplary haematological malignancies include mantle cell lymphoma (MCL), multiple myeloma (MM), chronic lymphocytic leukaemia (CLL), non-Hodgkin's lymphoma (NHL), acute lymphocytic leukaemia (ALL), chronic or acute myeloid leukaemia (AML), small lymphocytic lymphoma (SLL) or acute lymphatic leukemia, follicular lymphoma, Waldenstrom's macroglobulinemia (WM), B-cell lymphoma, diffuse large B-cell lymphoma (DLBCL), myelodysplastic neoplasia or myeloproliferative neoplasia. In an example, the subject received the HSCT for treatment of a B-cell disorder. In another example, the subject received the HSCT for treatment of a B-cell malignancy. In another example, the subject received the HSCT for treatment of a plasma cell disorder. In another example, the subject received the HSCT for treatment of a lymphoproliferative disorder. For example, the subject can have received the HSCT for treatment of a multiple myeloma, B-cell lymphoma, Hodgkin's and non-Hodgkin lymphoma and macroglobulinemia. In another example, the subject can have received the HSCT for treatment of an immune deficiency. For example, the subject can have received the HSCT for treatment of Wiskott-Aldrich syndrome, chronic granulomatous disease and thalassemia. In other examples, the subject can have received the HSCT for treatment of a metabolic disorder, hemoglobinopathy, an autoimmune disease or bone marrow failure.

In another example, the methods of the present disclosure can be used to measure reestablishment of a HSCT recipients haematological disorder. For example, the methods of the present disclosure are directed towards a method of determining reestablishment of a HSCT recipients B-cell disorder, wherein a biological sample is obtained from the HSCT recipient and the level of isolated genomic DNA from a genetically distinct cell population based on a copy number variation (CNV) polymorphism that is an informative marker of the genetically distinct cell population is determined, wherein the level of isolated genomic DNA from the genetically distinct cell population indicates whether the HSCT recipients haematological disorder is restabilised. In this example, the genetically distinct cell population can be genetically distinct from the subject. For example, the genetically distinct cell population can consist of non-self cells. In another example, the genetically distinct cell population can be self-cells.

In another example, the level of self DNA and/or the level non-self DNA are compared with the total level of DNA to provide a percentage of haematological disorder reestablishment. In an example, the percentage of self cells to total cells provides the percentage of haematological disorder reestablishment. In an example, a percentage of self cells greater than about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 21%, about 22%, about 23%, about 24%, about 25%, about 30%, about 35%, about 40% indicates reestablishment of the HSCT recipients haematological disorder.
In another example, the percentage of non-self cells provides the percentage of haematological disorder reestablishment. In an example, a percentage of non-self cells less than about 99%, about 98%, about 97%, about 96%, about 95%, about 94%, about 93%, about 92%, about 91%, about 90%, about 89%, about 88%, about 87%, about 86%, about 85%, about 84%, about 83%, about 82%, about 81%, about 80%, about 79%, about 78%, about 77%, about 76%, about 75%, about 70%, about 65%, about 60% indicates reestablishment of the HSCT recipients haematological disorder.

In another example, the level of self DNA and/or the level non-self DNA are compared with the total level of DNA to provide a ratio of haematological disorder reestablishment. In an example, a ratio of self cells:non-self cells greater than about 3:20; about 1:10; about 1:20, about 1:50; about 1:100 indicates reestablishment of the HSCT recipients haematological disorder.

In another example, the methods of the present disclosure can be used to measure disease relapse in a patient. In another example, the methods of the present disclosure can be used to measure minimal residual disease in a patient in need thereof. For example, the methods of the present disclosure are directed towards a method of measuring minimal residual disease, wherein a biological sample is obtained from a patient and the level of isolated genomic DNA from a genetically distinct cell population based on a copy number variation (CNV) polymorphism that is an informative marker of the genetically distinct cell population is determined, wherein the level of isolated genomic DNA from the genetically distinct cell population indicates whether the level of minimal residual disease. In another example, the genetically distinct cell population can be self-cells. In another example, the level of isolated genomic DNA from self and non-self cells is determined according to the methods of the present disclosure. In an example, the levels of isolated genomic DNA from self and non-self cells are compared to determine the level of minimal residual disease. In another example, the level of total DNA in the sample is determined according to the methods of the present disclosure. In another example, the level of self DNA and/or the level non-self DNA are compared with the total level of DNA to provide a percentage of minimal residual disease. In another example, the level of self DNA and/or the level non-self DNA are compared with the total level of DNA to provide a ratio of minimal residual disease.

In the above examples, chimerism can be measured across cell types. Exemplary cell types are outlined above. For example, chimerism can be assessed in T-cells, B-cells and granulocytes. In this example, three measures of chimerism are obtained. These measures of chimerism can inform treatment status. In an example, reduced levels of recipient T-cells can indicate that immunosuppression is too high. In this instance, the treating physician can reduce immunosuppression in the recipient. In contrast, elevated levels of recipient T-cells provides scope to increase immunosuppression if required. These examples can be particular advantageous in T-cell mediated disease such as GVHD In these pathologies, treatment aims to maintain an appropriate balance between T-cell suppression and transplant survival. The methods of the present disclosure may be used in methods of treating GVHD. In such methods, chimerism may be measured in T-cells, B-cells and granulocytes and treatment administered based on the measured levels of chimerism. Such treatment methods do not form aspects of the invention.

In another example, the methods of the present disclosure can be used to identify contamination of a biological sample. In this example, the term "contamination" is used to define the presence of two or more genetically distinct cell populations in a biological sample. In an example, the methods of the present disclosure are directed towards determining the level of contamination in a blood sample. For example, it is envisaged that the methods of the present disclosure encompass identifying maternal contamination of a cord blood sample (e.g. the presence of maternal "non-self' cells in a foetal "self' cord blood sample). For example, the methods of the present disclosure are directed towards a method of determining the level of maternal contamination in a cord blood sample, the method comprising determining in a cord blood sample the level of isolated genomic DNA from a genetically distinct cell population based on a copy number variation (CNV) polymorphism that is an informative marker of the genetically distinct cell population, wherein the level of isolated genomic DNA from the genetically distinct cell population provides the measure of contamination in the cord blood sample. In this example, the genetically distinct cell population can be genetically distinct from the foetus. For example, the genetically distinct cell population can be maternal cells. In another example, the genetically distinct cell population can be foetal cells.

In an example, the levels of isolated genomic DNA from self and non-self cells are compared to provide the measure of contamination in the cord blood sample. In another example, the level of total DNA in the sample is determined according to the methods of the present disclosure. In another example, the level of self DNA and/or the level non-self DNA are compared with the total level of DNA to provide a percentage of contamination. For example, the percentage of self cells to total cells provides the percentage of contamination. For example, the percentage of self cells to total cells provides the percentage of contamination. In another example, the level of self DNA and/or the level non-self DNA are compared with the total level of DNA to provide a ratio of contamination. In an example, a ratio of self cells:non-self cells greater than about 3:20; about 1:10; about 1:20, about 1:50; about 1:100 indicates contamination of the cord blood sample.

Monitoring

By performing the methods of the present disclosure over at least two time points, the methods of the present disclosure can be used to monitor chimerism in a subject over time. For example, the methods of the present disclosure can be used to monitor a subject after HSCT. In another example, the methods of the present disclosure can be used to monitor engraftment. In another example, the methods of the present disclosure can be used to monitor reestablishment of a subject's bone marrow. In another example, the methods of the present disclosure can be used to monitor reestablishment of a subject's haematological disorder. In another example, the methods of the present disclosure can be used to monitor minimal residual disease. For example, the methods of the present disclosure can be used to monitor graft versus host disease (GVHD). In another example, the methods of the present disclosure can be used to monitor disease relapse. In another example, the methods of the present disclosure can be used to monitor graft HSCT rejection. In another example, the methods of the present disclosure can be used to predict treatment failure. In an example, the methods of the present disclosure can be performed daily. In an example, the methods of the present disclosure can be performed weekly. In another example, the methods of the present disclosure can be performed monthly. In another example, the methods of the present disclosure can be performed bi-monthly. In another example, the methods of the present disclosure can be performed every three months, every four months, every six months. In another example, the methods of the present disclosure can be performed yearly.

In an example, an increase in the level of self cells and/or a decrease in the level of non-self cells indicates that reestablishment of a HSCT recipients bone marrow. Reestablishment of a subject's bone marrow can indicate HSCT rejection. Accordingly, an increase in the level of self cells and/or a decrease in the level of non-self cells can indicate that immunosuppressive therapy should be administered to the HSCT recipient or the HSCT recipient's immunosuppressive therapy should be modified. In another example, an increase in the level of self cells and/or a decrease in the level of non-self cells can indicate that therapy for the haematological disorder should be administered to the HSCT recipient or the HSCT recipients therapy should be modified. Exemplary modifications to immunosuppressive therapy include increasing immunosuppressive therapy when an increase in the level of self cells and/or a decrease in the level of non-self cells is observed.

In an example, the methods of the present disclosure may be incorporated into an assay for use in determining a suitable treatment regimen. For example, a method of treating HSCT rejection in a subject may be performed, the method comprising monitoring the level of isolated genomic DNA from self and non-self cells in samples obtained from a HSCT recipient, wherein immunosuppressive therapy is administered to the HSCT recipient or the HSCT recipient's immunosuppressive therapy is modified if an increase in the level of self cells and/or a decrease in the level of non-self cells is detected. In the alternative a method of treating a haematological disorder in a subject may be performed, the method comprising monitoring the level of isolated genomic DNA from self and non-self cells in samples obtained from a HSCT recipient, wherein therapy is administered to the HSCT recipient or the HSCT recipient's therapy is modified if an increase in the level of self cells and/or a decrease in the level of non-self cells is detected. The therapy may comprise chemotherapy, radiotherapy, immunotherapy, antibody therapy, HSCT or other pharmacological agent. Therapeutic modifications include increasing therapy when an increase in the level of self cells and/or a decrease in the level of non-self cells is observed.

Computer implemented method

It is envisaged that the methods of the present disclosure may be implemented by a system such as a computer implemented method. For example, the system may be a computer system comprising one or a plurality of processors which may operate together (referred to for convenience as "processor") connected to a memory. The memory may be a non-transitory computer readable medium, such as a hard drive, a solid state disk or CD-ROM. Software, that is executable instructions or program code, such as program code grouped into code modules, may be stored on the memory, and may, when executed by the processor, cause the computer system to perform functions such as determining that a task is to be performed to assist a user to determine informative CNV polymorphisms in DNA obtained from a biological sample from a subject; receiving data indicating the level of DNA (e.g. self and/or non-self DNA) in the sample;

processing the data to detect the level of chimerism in the sample based on the levels of DNA; outputting the level of chimerism in the sample.

For example, the memory may comprise program code which when executed by the processor causes the system to determine the measure of chimerism in a subject, or receive data indicating the measure of chimerism in the subject; process the data to measure chimerism based on the level of isolated genomic DNA from genetically distinct cell populations; report the measure of chimerism in a subject.

In another example, the system may be coupled to a user interface to enable the system to receive information from a user and/or to output or display information. For example, the user interface may comprise a graphical user interface, a voice user interface or a touchscreen.

In an example, the system may be configured to communicate with at least one remote device or server across a communications network such as a wireless communications network. For example, the system may be configured to receive information from the device or server across the communications network and to transmit information to the same or a different device or server across the communications network. In other examples, the system may be isolated from direct user interaction.

In another example, performing the methods of the present disclosure to measure chimerism in a subject, by determining the level of obtained DNA from genetically distinct cell populations in the subject, the level of DNA being determined based on informative CNV polymorphisms, enables establishment of a diagnostic or prognostic rule based on the DNA level. For example, the diagnostic or prognostic rule can be based on the measure of chimerism relative to a control.

In another example, the diagnostic or prognostic rule is based on the application of a statistical and machine learning algorithm. Such an algorithm uses relationships between measures of chimerism and disease status observed in training data (with known disease status) to infer relationships which are then used to predict the status of patients with unknown status. An algorithm is employed which provides an index of probability that, for example:

- engraftment has occurred;
- a patient's bone marrow has re-established;
- a patient's haematological disorder has relapsed.

In an example, the algorithm performs a multivariate or univariate analysis function.

The present disclosure also provides to a method of allowing a user to determine the status, prognosis and/or treatment response of a HSCT recipient, the method including (a) receiving data indicating the measure of chimerism in the subject; b) processing the data to determine the measure of chimerism in the subject; and c) outputting the status, prognosis and/or treatment response of a subject.

The present disclosure also provides a method of allowing a user to determine the status, prognosis and/or treatment response of a subject with an disorder, the method including (a) receiving data indicating the measure of chimerism in the subject; b) processing the data to determine the measure of chimerism in the subject; and c) outputting the status, prognosis and/or treatment response of a subject.

In an example, the measure of chimerism provides a correlation to the presence, state, classification, remission or progression of disease.

Compositions/kits

The present disclosure also provides a kit comprising PCR primer pairs specifically configured to amplify the CNV polymorphisms outlined in the present disclosure (see for example Table 7) for use in the methods of the present disclosure. For example, the kit can comprise:

a) probes and/or primers specific for CNV polymorphisms shown in Table 1; and/or
b) probes and/or primers shown in Table 7.

In an example, the kit components may be packaged in or with a suitable solvent or in lyophilised form. The kit components may optionally be packaged in a suitable container with written instructions for performing the methods of the present disclosure. The present disclosure also provides the use of the primers disclosed herein in the manufacture of a non-invasive in-vitro diagnostic assay for performing a method of the present disclosure.

It will be appreciated by persons skilled in the art that numerous variations and/or modifications may be made to the above-described embodiments, but still fall within the appended claims. The present embodiments are, therefore, to be considered in all respects as illustrative and not restrictive. Any discussion of documents, acts, materials, devices, articles or the like which has been included in the present specification is solely for the purpose of providing a context for the present disclosure. It is not to be taken as an admission that any or all of these matters form part of the prior art base or were common general knowledge in the field relevant to the present disclosure as it existed before the priority date of each claim of this application. The present application claims priority from AU 2015902274 filed 15 June 2015.

## EXAMPLES

### Example 1 - Selection of CNV-deletion loci

Every individual genome has a different copy number variant (CNV) profile consisting of many thousands of losses (i.e. deletions) and gains (i.e. delectations, amplifications, etc.) spread out throughout the genome. Some of these occur at copy number variable regions which have been identified in large-scale CNV genotyping studies but many are also unique. Of known CNV regions, these can be stratified in terms of allelic distribution of copy number states (i.e. 0, 1, 2, 3, etc), genomic size, and population frequency. For the purposes of detecting chimerism in DNA samples, CNV regions are selected having an allelic distribution of 0, 1, 2 (but not duplications) and having a homozygous deleted (0-copy PCR product) allele frequency of greater than 0.4. The latter is not to be taken as a restriction but rather reflected an approach for development of a panel of assays capable of detecting chimerism in the vast majority of samples without the need for screening the paternal genome. Such CNVs are common in the population and an initial panel of 10 (CNDs_01 - CND_10; Table 1) was selected by in silico analysis of public HapMap data (Conrad et al. Nature, 4(54):704-712, 2010); McCarrol et al. Nature Genet 40(10):1166-1174, 2008). The Hap Map data include copy number information for each CNV identified by a high resolution microarray analysis. For frequency calculations, only data from unrelated individuals were included (n=122). CNVs with homozygous deleted (0-copy PCR product), heterozygous deleted (1-copy PCR product) and homozygous non-deleted (2-copy PCR product) genotypes were selected for analysis. CNVs with allelic distributions extending to more than 2 copies and chromosomes X and Y CNVs were excluded. For each selected CNV, genotype frequencies were calculated. CNVs overlapping with segmental duplications were excluded. All ten CNVs were less than 3kb in size; however the method applies to CNVs of all size ranges. In terms of clinical application it is important to note that all ten CNV regions are polymorphic and none of them is of any intrinsic clinical significance.

Table 1:

| Name | CNP_id | chr | start_hg18 | end_hg18 | Size (bp) | **homozygo us deleted frequency** (0-copy PCR product) | **heterozygo us deleted frequency** (1-copy PCR product) | **homozygo us non-deleted frequency** (2-copy PCR product) |
|---|---|---|---|---|---|---|---|---|
| CND 12 | CNVR3451.1 | Chr 7 | 73,467,042 | 73,469,172 | 2,130 | 0.55 | 0.33 | 0.12 |
| CND 13 | CNVR4596.1 | Chr 10 | 4,698,559 | 4,700,493 | 1,934 | 0.61 | 0.31 | 0.08 |
| CND 14 | CNVR801_full | Chr 2 | 54,419,132 | 54,420,976 | 1,844 | 0.59 | 0.37 | 0.04 |
| CND 15 | CNVR5853_full | Chr 13 | 38,831,627 | 38,833,387 | 1,760 | 0.55 | 0.33 | 0.12 |
| CND 16 | CNVR842.1 | Chr 2 | 76,627,234 | 76,628,943 | 1,709 | 0.6 | 0.33 | 0.08 |
| CND 17 | CNVR431.1 | Chr 1 | 185,731,458 | 185,733,106 | 1,648 | 0.62 | 0.33 | 0.04 |
| CND 18 | CNVR4203.1 | Chr 9 | 17,900,038 | 17,901,633 | 1,595 | 0.61 | 0.36 | 0.03 |
| CND 20 | CNVR952.1 | Chr 2 | 121,513,996 | 121,515,257 | 1,261 | 0.53 | 0.36 | 0.1 |
| CND 21 | CNVR1138.2 | Chr 2 | 219,761,436 | 219,762,572 | 1,136 | 0.39 | 0.42 | 0.18 |
| CND 22 | CNVR5923.1 | Chr 13 | 71,743,783 | 71,744,892 | 1,109 | 0.41 | 0.55 | 0.04 |
| CND 23 | CNVR8147.1 | Chr 22 | 33,975,445 | 33,976,472 | 1,027 | 0.45 | 0.43 | 0.12 |
| CND 24 | CNVR3004.1 | Chr 6 | 95,250,114 | 95,251,113 | 999 | 0.4 | 0.47 | 0.13 |
| CND 25 | 1343* | Chr 8 | 112,363,455 | 112,364,436 | 981 | 0.57 | 0.38 | 0.05 |
| CND 26 | CNVR381.1 | Chr 1 | 157,915,386 | 157,916,253 | 867 | 0.6 | 0.35 | 0.05 |

(continued)

| Name | CNP_id | chr | start_hg18 | end_hg18 | Size (bp) | **homozygo us deleted frequency** (0-copy PCR product) | **heterozygo us deleted frequency** (1-copy PCR product) | **homozygo us non-deleted frequency** (2-copy PCR product) |
|---|---|---|---|---|---|---|---|---|
| CND 27 | CNVR4374.1 | Chr 9 | 88,344,772 | 88,345,596 | 824 | 0.56 | 0.36 | 0.07 |
| CND 28 | CNVR2799.1 | Chr 6 | 18,510,099 | 18,510,860 | 761 | 0.41 | 0.44 | 0.15 |
| CND 29 | CNVR6540.1 | Chr 15 | 97392250 | 97392985 | 735 | 0.51 | 0.43 | 0.06 |
| CND 30 | CNVR6357.1 | Chr 15 | 37531690 | 37532136 | 446 | 0.52 | 0.42 | 0.07 |
| CND 31 | 158 | Chr 1 | 208148236 | 208150607 | 2,371 | 0.383 | 0.450 | 0.167 |
| CND 32 | CNVR358.1 | Chr 1 | 150822234 | 150856715 | 34,481 | 0.393 | 0.459 | 0.148 |
| CND 33 | CNVR217.1 | Chr 1 | 72538870 | 72584557 | 45,687 | 0.393 | 0.459 | 0.148 |
| CND 34 | CNVR7808.1 | Chr 20 | 21234267 | 21236529 | 2,262 | 0.516 | 0.418 | 0.066 |
| CND 36 | 554 | Chr 4 | 9823254 | 9844366 | 21,112 | 0.550 | 0.333 | 0.117 |
| CND 37 | CNVR2469.1 | Chr 5 | 57359283 | 57369499 | 10,216 | 0.615 | 0.361 | 0.025 |
| CND 38 | CNVR3009.1 | Chr 6 | 100141275 | 100141994 | 719 | 0.623 | 0.377 | 0.000 |
| CND 39 | CNVR3609.1 | Chr 7 | 147704059 | 147707263 | 3,204 | 0.656 | 0.311 | 0.033 |
| CND 41 | CNVR2304.1 | Chr 5 | 1977604 | 1978111 | 507 | 0.672 | 0.328 | 0.000 |
| CND 01 | CNVR376.1 | Chr 1 | 157,134,152 | 157,136,674 | 2,522 | 0.51 | 0.42 | 0.07 |

(continued)

| Name | CNP_id | chr | start_hg18 | end_hg18 | Size (bp) | **homozygo us deleted frequency** (0-copy PCR product) | **heterozygo us deleted frequency** (1-copy PCR product) | **homozygo us non-deleted frequency** (2-copy PCR product) |
|---|---|---|---|---|---|---|---|---|
| CND 02 | CNVR7344.1 | Chr 18 | 53,097,735 | 53,099,702 | 1,967 | 0.52 | 0.4 | 0.08 |
| CND 03 | CNVR4014.1 | Chr 8 | 112,363,260 | 112,365,469 | 2,209 | 0.5 | 0.41 | 0.08 |
| CND 04 | CNVR6084.1 | Chr 14 | 21,951,540 | 21,952,070 | 530 | 0.46 | 0.43 | 0.11 |
| CND 05 | CNVR6676.1 | Chr 16 | 25,247,611 | 25,250,595 | 2,984 | 0.45 | 0.47 | 0.08 |
| CND 06 | CNVR3319.1 | Chr 7 | 24,004,755 | 24,006,584 | 1,829 | 0.47 | 0.38 | 0.15 |
| CND 07 | CNVR5850.1 | Chr 13 | 37,955,318 | 37,958,191 | 2,873 | 0.43 | 0.43 | 0.14 |
| CND 08 | CNVR3753_full | Chr 8 | 4,110,308 | 4,112,335 | 2,027 | 0.43 | 0.43 | 0.13 |
| CND 09 | CNVR7301.1 | Chr 18 | 33,560,073 | 33,560,645 | 572 | 0.42 | 0.45 | 0.12 |
| CND 10 | CNVR4886.1 | Chr 10 | 108,020,303 | 108,022,518 | 2,215 | 0.41 | 0.42 | 0.17 |
| CND 19 | CNVR1037.1 | Chr 2 | 159,668,040 | 159,669,209 | 1,169 | 0.5 | 0.43 | 0.07 |

**Example 2 - Genotyping**

PCRs have been designed with primers located within (i.e. internal PCR) and flanking (i.e. external PCR) the 10 'CNV-deletion' loci (CND_01 - CND_10; Table 1). The internal PCR gives a specific product for heterozygous deleted (1-copy PCR product), or homozygous non-deleted (2-copy PCR product) genotypes and an absence of a specific product indicates a homozygous deleted genotype (0-copy PCR product). The external PCR gives a precise product shorter than the "wild-type"(Note: the larger, "wild-type" product is not present as the undeleted allele is too large to be amplified) confirming all the deletions detected by the internal PCRs. The combination of internal and external PCR was used to determine the genotype status for each CND (i.e. homozygous non-deleted (2-copy PCR product), heterozygous deleted (1-copy PCR product) or homozygous deleted (2-copy PCR product)) in the maternal cellular DNA. Genotyping results obtained for 21 control samples are shown in Figure 1. Absence of a band indicates an individual with a "homozygous deleted" (0-copy PCR product) genotype (Figure 1a) and it is observed in 12 individuals. This is consistent with the predicted 50% frequency for CND_01. The 9 individuals showing a PCR product band were either "heterozygous deleted (1-copy PCR product) or homozygous non-deleted (2-copy PCR product), as inferred from the external PCR results (Figure 1b). The results from the external PCR (Figure 1b) also confirm that the homozygous deletions (0-copy PCR product) were real rather than PCR failures. To prove unequivocally that the genotyping assay was accurate, the "internal PCR" and the "external PCR" products shown were sequenced and mapped back to a genome location using BLAT. In all cases this was to the correct, unique genome locus. The expected frequencies of all CNV-deletions were confirmed by genotyping DNA samples of 100 individuals sourced from a local general population (Table 1). The expected and observed frequencies were similar for most CNDs, except for CND_10, which had a relatively low homozygous deleted (0-copy PCR product) frequency in our control population.

**Example 3 - Spiking experiment**

For each CND in the panel, the lower sensitivity limit and specificity in measurement of chimerism were modelled empirically by using titrations of "chimeric" DNA obtained by mixing control DNA from individuals differing in their genotype status. Briefly, a DNA sample from an individual heterozygously deleted (1-copy PCR product) for a selected CNV-deletion locus was spiked into a DNA sample from an individual "homozygous deleted" (0-copy PCR product) for that selected CNV-deletion locus. CNV-deletion qPCR assay and SRY based qPCR assays were performed on spiked samples.

**Example 4 - In silico selection of CND markers**

CNDs with homozygous deleted (0-copy PCR product) deletion frequencies between 0.3 and 0.7 were identified by in silico analysis of two high-resolution data sets. Data-set 1: genotype data for 5238 CNV loci generated by testing 450 HapMap samples at 500bp resolution (Conrad et al. (2010) supra). Data-set 2: genotype data for 1319 CNV loci generated by testing 270 HapMap samples at 2Kb resolution (McCarrol et al. (2008) supra). Analyses used the CEPH data only. As the CEPH HapMap data include trio data on proband and parents, calculation of homozygous deleted (0-copy PCR product), heterozygous deleted (1-copy PCR product) and homozygous non-deleted (2-copy PCR product) genotype frequencies was performed using only parental data to minimise overestimation. No further correction, such as for autozygosity (Stevens et al. (2012) EJHG 20:657-667; Stevens et al. (2012) PLOS One 7:49575), was done. *In silico* selection used the following criteria: each CNV constitutes a single locus in the human genome that spanned less than 3 kilobases (accounting for gender bias). This resulted in 38 candidates; all these CNV regions are polymorphic and none have any known intrinsic clinical significance.

**Example 5 - The CND panel**

Of the 38 CNDs identified above, the 10 predicted to have the most informative "homozygous deleted" (0-copy PCR product) genotype frequency ranking were selected as an initial panel for in vitro assessments (CND_01 - CND_10; Table 1). The homozygous deleted genotype (0-copy PCR product) frequencies range between 0.410 and 0.508, the mean value being 0.452 with a standard deviation of 0.038. Based on these homozygous deleted (0-copy PCR product) genotype frequencies, it was calculated and later confirmed by observation that 99% of individuals are likely to be homozygous deleted (0-copy PCR product) for at least one CND in the panel. *In-vitro* estimation of CND frequencies

The estimated population frequencies for the 10 CNDs obtained from the in silico selection were compared with those in an unselected sample of 93 healthy individuals. The local population from which this sample was taken is ethnically very diverse and it is assumed that our random sampling reflects this. Despite a paucity of information for these CNDs in different ethnic populations, the potential of these CND markers for chimerism testing in diverse populations was highlighted by the in silico and in vitro frequencies being were very similar.

**Example 6** - **Sample genotyping**

PCR primers were designed to locate within (i.e. internal PCR) and flanking the deleted region (i.e. external PCR) of each CND marker in the panel. Short amplicons, ranging in size from 58-74 bp (average 65 bp) were chosen for the internal PCRs. Samples with a homozygous deleted (0-copy PCR product) genotype give no 'internal' PCR product. Verification of the homozygous deleted (0-copy PCR product) genotypes was made using the external PCR assays, which give unique PCR products. The identities of the internal and external PCR amplicons were confirmed using Sanger sequencing. All internal PCR amplicon sequences of non-deleted alleles mapped to the expected regions within the CND loci and external PCR amplicons of deleted alleles mapped to the flanking regions of the CND loci. The combination of internal/external PCR results distinguishes the homozygous deleted (0-copy PCR product), heterozygous deleted (1-copy PCR product) and homozygous non-deleted (2-copy PCR product) genotypes (see Figure 1 for examples).

The distributions of 'homozygous deleted' (0-copy PCR product) CND frequencies observed in three independent population cohorts are almost superimposable and indicate that, on average, the 10 panel genotype for any individual contains 4-5 homozygous deleted (0-copy PCR product) CNDs (Figure 2).

**Example 7** - **Validation study**

In allogeneic bone marrow transplantation cases, post-transplantation monitoring is used to predict treatment failure, such as disease relapse, graft rejection and graft-versus host diseases (GVHD). In this context, chimerism analysis is critical in predicting patient outcome and helping clinicians to set up the appropriate patient management strategy.

A simple, efficient chimerism analysis has been established for monitoring engraftment in bone marrow transplantation cases by using copy number deletion variation markers and droplet digital PCR (ddPCR). The method has been successful in monitoring engraftment in an extremely complex bone marrow transplantation case, involving 4 individuals (three were closely related) (see Example 9).

Sensitivity assessment

Three randomly selected CNV markers from the panel underwent sensitivity analysis. Genomic DNA from an individual shown to be heterozygous deleted for the respective CNV marker was spiked into DNA from an individual known to be homozygous deleted for that marker to create a spiking series across the fractional range of 100% to 0.049%. Due to the small quantities of DNA below the chimeric fraction 0.049%, serial dilutions were used to generate chimeric fractions from 0.049% down to 0.0061%.

Spiked samples in triplicate underwent ddPCR as described using a standardised input of 100ng of genomic DNA per well across the entire fractional range. A linear model for each CNV assay was determined. Intra-assay variation was measured by calculating the coefficient of variation (CV) for the triplicate measurement of each CNV marker at each dilution. Inter-assay variation was measured by calculating CV for all three CNV markers at each dilution step. Limit of detection was defined as the lowest chimeric fraction at which all replicates measured the informative CNV marker.

Inter-assay variation was also assessed in practice using longitudinal chimerism analysis data from a highly informative unrelated BMT donor-recipient pair. The standard deviation and CV were calculated from all informative markers for the donor and recipient at each time point.

All three CNV markers detected chimerism down to 0.006%. The lower limit of detection was determined at 0.012%. Performance for all three CNV markers across the dynamic range (from 0% to 100%) was linear ($r>0.99$, $p<0.001$) (Figure 3). CV within the replicates for each marker (intra-assay variation) ranged 4.6%-14.6% for chimeric fractions from 100% down to 0.1% (Figure 4). Between 0.1% and 0.01% chimerism, the CV ranged from 35-41%. CV between means of the marker replicates (inter-assay variation) ranged 3.3%-15% from 100% chimerism down to 0.09% chimerism. Below this, inter-assay CV was 5%-34%.

The inter-assay CV results obtained from the spiking series matched those obtained from a longitudinally sampled clinical case in which the donor had 5 informative CNV markers and the recipient had 8 informative CNV markers (Figure 5). Inter-assay chimeric fraction standard deviation ranged from 0.003-0.03 and CV ranged from 2-11% across the chimeric fractional range 3%-96%.

Reproducibility assessment

A post-BMT chimeric genomic DNA sample extracted as described was quantified using Qubit fluorometric quantitation (Thermo Fisher Scientific, Waltham, MA, USA) and diluted to approximately 7.5ng/uL. Sixteen CNV markers were run against this sample using ddPCR as described. The experiment was repeated on the same sample on days 1, 2, 5, 6, 7 and 8. On day 8, the experiment was repeated in the morning (day 8a) and in the afternoon (day 8b). The concentration of each CNV marker in the reaction was used to calculate the reproducibility CV for each marker over time. Additionally, CNV chimerism analysis was performed at each time point and the resulting chimeric fractions were compared across time points.

Repeated analysis of the same chimeric sample using sixteen CNV markers on seven occasions yielded consistent results with all but one marker demonstrating CVs 3.3-5%. In one marker (CNV09B), the coefficient of variation was increased to 9.9% by an outlier on one run. When these markers were used to determine the chimeric fraction, chimerism results from the same sample were similarly consistent across time points ranging from 0.024%-0.050% (standard deviation 0.013%).

ddPCR was performed using BioRad Qx200 Droplet Digital system (Bio-red, Pleasanton, CA), as described below. The ddPCR absolute quantitative raw data from the individual runs is shown in Figure 6. The absolute measurements for individual CND assays substantially overlap between day to day runs with most of the CV range between 3.3-5.0% (exception - CND09; relatively high CV of 9.9% due to a single outlier measurement in the Friday assay). To compare the chimerism results from intra- and inter- days' runs, donor and recipient genotypes were compared to identify unique polymorphisms (informative markers). Informative markers were analysed separately to assess the total DNA copies of recipient and donor DNA (Table 2). Each individual informative CND polymorphism provides an independent measurement for calculating the chimerism results. Accordingly, the mean of all heterozygous deleted (1-copy PCR product) markers was used to represent the amount of recipient and donor DNA in the sample. The total DNA amount was then calculated by adding up the recipient and donor DNA as described below.

Table 2:

| | | **Mean Copy numbers of 1-copy markers** | | |
|---|---|---|---|---|
| **Day** | **Date** | **Recipient** | **Donor** | **Total** |
| Thursday | 14052015 | 1 | 4214 | 4215 |
| Friday | 15052015 | 2 | 4302 | 4304 |
| Monday | 18052015 | 1 | 4138 | 4139 |
| Tuesday | 19052015 | 1 | 4132 | 4133 |
| Wednesday | 20052015 | 2 | 4188 | 4190 |
| Thursday.1 | 21052015 | 2 | 4124 | 4126 |
| Thursday.2 | 21052015 | 2 | 4028 | 4030 |

Chimerism results were presented as a percentage of donor and recipient profile (Table 4). Standard deviation was presented as a percentage of inter- and intra- day run results (0.013%). Raw data from intra and inter day is shown in Table 5. Day to day variation results of informative markers are shown in Table 6. The low standard deviation demonstrates that the chimerism assay based on informative CND polymorphism markers is consistent and has a very high reproducibility.

Table 3:

| | | **Chimerism results** | |
|---|---|---|---|
| **Day** | **Date** | **Recipient%** | **Donor %** |
| Thursday | 14052015 | 0.024% | 99.976% |
| Friday | 15052015 | 0.046% | 99.954% |
| Monday | 18052015 | 0.024% | 99.976% |
| Tuesday | 19052015 | 0.024% | 99.976% |
| Wednesday | 20052015 | 0.048% | 99.952% |
| Thursday.1 | 21052015 | 0.048% | 99.952% |
| Thursday.2 | 21052015 | 0.050% | 99.950% |
| | | **SD=0.013%** | **SD=0.013%** |

Accuracy assessment

15 genomic DNA samples from 4 groups of patients were used as the comparator samples for assessing the assays. Each set of samples include donor, recipient and time-course mixed chimerism samples (see Table 4). Graft status had previously been determined in all samples by assessing short tandem repeats (STR). However, researchers were blinded from graft status prior to assessing chimerism using informative CNV polymorphisms. Graft status as assessed via STR and CNV was compared.

Table 4:

| Patient | Sample | ng/ul in Qubit |
|---|---|---|
| Patient1 | Recipient | 7.62 |
| Patient1 | Donor | 6.54 |
| Patient1 | TestA | 6.69 |
| Patient1 | TestB | 7.04 |
| Patient1 | TestC | 6.49 |
| Patient2 | Recipient | 6.9 |
| Patient2 | Donor | 7.18 |
| Patient2 | TestA | 6.73 |
| Patient2 | TestB | 6.52 |
| Patient2 | TestC | 6.26 |
| Patient2 | TestD | 5.91 |
| Patient3 | Recipient | 9.96 |
| Patient3 | Donor | 6.69 |
| Patient3 | TestA | 7.24 |
| Patient3 | TestB | 6.89 |
| Patient3 | TestC | 6.65 |
| Patient3 | TestD | 7.17 |
| Patient3 | TestE | 7.43 |
| Patient4 | Recipient | 40.5 |
| Patient4 | Donor | 39.6 |
| Patient4 | TestA | 50.5 |
| Patient4 | TestB | 40.9 |
| Patient4 | TestC | 46.5 |

All samples including donor, recipient and chimerism samples were screened using 15 CND polymorphism markers with a homozygous control (2-copy PCR product) (Figures 7 - 10). The chimerism results of each sample were analysed as described above. CNV chimerism analysis correlated well with previous STR analysis (0.997) (Figure 11). The strong correlation indicates that the CNV analysis is at least as accurate as STR analysis. Furthermore, CNV chimerism analysis has the following advantages over STR analysis:
i) the assay can be used in the gender transplants with no restriction to sex-mismatched HSCT;
ii) each informative CND marker provides an independent chimerism measurement;
iii) the assay is very robust with respect to technical replicates as shown by solid assay performance and absolute DNA copy number quantification;
iv) the assay has very low limits of detection due to the zero background and very high sensitivity;
v) A large panel of 38 CND markers is available which can be used in any non-identical donor-recipient bone marrow transplantation pair.

Table 5:

| | | Copies/ul of Markers | | | | | | | | | | | | | | |
|---|---|---|---|---|---|---|---|---|---|---|---|---|---|---|---|---|
| Day | Date | ACE | CND12 | CND13 | CND14 | CND15 | CN D16 | CN D18 | CND1B | CND20 | CND2B | CND3B | CN D4B | CND5B | CND6B | CND7B | CND9B |
| Thursday | 14052015 | 6700 | 0 | 0 | 4200 | 2 | 4670 | 4300 | 8460 | 1 | 0 | 1 | 1 | 4150 | 0 | 1 | 3750 |
| Friday | 15052015 | 6880 | 3 | 0 | 4500 | 1 | 5130 | 4530 | 9400 | 1 | 0 | 3 | 1 | 4480 | 0 | 2 | 2878 |
| Monday | 18052015 | 6830 | 0 | 0 | 4190 | 0 | 4500 | 4210 | 8860 | 2 | 0 | 3 | 0 | 4080 | 0 | 0 | 3710 |
| Tuesday | 19052015 | 6460 | 0 | 0 | 4190 | 0 | 4560 | 4160 | 8410 | 1 | 0 | 2 | 1 | 3890 | 0 | 0 | 3860 |
| Wednesday | 20052015 | 6380 | 1 | 0 | 3980 | 2 | 4770 | 4090 | 8480 | 3 | 0 | 3 | 1 | 4120 | 0 | 1 | 3980 |
| Thursday.1 | 21052015 | 6630 | 2 | 0 | 4110 | 0 | 4540 | 4250 | 9140 | 4 | 0 | 4 | 0 | 3950 | 0 | 2 | 3770 |
| Thursday.2 | 21052015 | 6420 | 1 | 0 | 4010 | 1 | 4480 | 3990 | 8600 | 4 | 0 | 3 | 0 | 3950 | 0 | 0 | 3710 |
| | mean | 6614 | 1 | 0 | 4169 | 1 | 4664 | 4219 | 8764 | 2 | 0 | 3 | 1 | 4112 | 0 | 1 | 3664 |
| | Standard DV | 200 | 1 | 0 | 171 | 1 | 229 | 172 | 383 | 2 | 0 | 1 | 1 | 207 | 0 | 1 | 363 |
| | CV | 3.0% | | | 4.1% | | 4.9% | 4.1% | 4.4% | | | | | 5.0% | | | 9.9% |

Table 6:

| Day To Day Variation Results of Informative Markers | | | | | | | | | | | | | | |
|---|---|---|---|---|---|---|---|---|---|---|---|---|---|---|
| | | | Recipient Markers | | | | | | Donor Markers | | | | | |
| Day | Date | ACE | CND12 | CND15 | CND20 | CND3B | CND4B | CND7B | CND1B | CND14 | CND16 | CND9B | CND5B | CND18 |
| Thursday | 14052015 | 6700 | 0 | 2 | 1 | 1 | 1 | 1 | 8460 | 4200 | 4670 | 3750 | 4150 | 4300 |
| Friday | 15052015 | 6880 | 3 | 1 | 1 | 3 | 1 | 2 | 9400 | 4500 | 5130 | 2870 | 4480 | 4530 |
| Monday | 18052015 | 6830 | 0 | 0 | 2 | 3 | 0 | 0 | 8860 | 4190 | 4500 | 3710 | 4080 | 4210 |
| Tuesday | 19052015 | 6460 | 0 | 0 | 1 | 2 | 1 | 0 | 8410 | 4190 | 4560 | 3860 | 3890 | 4160 |
| Wednesday | 20052015 | 6380 | 1 | 2 | 3 | 3 | 1 | 1 | 8480 | 3980 | 4770 | 3980 | 4120 | 4090 |
| Thursday.1 | 21052015 | 6630 | 2 | 0 | 4 | 4 | 0 | 2 | 9140 | 4110 | 4540 | 3770 | 3950 | 4250 |
| Thursday.2 | 21052015 | 6420 | 1 | 1 | 4 | 3 | 0 | 0 | 8600 | 4010 | 4480 | 3710 | 3950 | 3990 |

Comparison with FISH-based method

Whole blood samples were obtained from one female and one male. White blood cell counts were obtained for each sample. The samples were centrifuged and buffy coat isolated. The cells were washed twice in phosphate buffered saline before being diluted in phosphate buffered saline to the original sample volume. The female and male samples were then mixed to create a spiked series consisting of approximately 100%, 80%, 60%, 40%, 20% and 0% female cells.

Slides were prepared for FISH according to routine procedures and analysis performed using CytoCell Aneu-Vysion X centromeric (DXZ1) and Y centromeric (DYZ3) probes simultaneously following standard methods. A total of 300 interphase cells were analysed by two independent analysts (200 by the analyst and 100 by the checker) at each chimeric fraction.

The spiked series underwent CNV chimerism analysis as described. The concentration of each CNV marker in the reaction was used to calculate the CNV marker CV at each dilution step. Chimeric fractions were then calculated and compared with those measured by the FISH method.

Four unique informative markers for the male and female were used to determine chimerism fraction via the CNV method. The inter-assay CV among the markers at each dilution step was less than 8%. There was a strong linear correlation between chimerism fraction measured using the CNV method and chimerism fraction as determined by gender mismatch FISH analysis ($r=0.9957$, 95% CI 0.9591-0.9995, $p<0.001$) (Figure 12, left panel).

Comparison with SNP-Based Method

Fifteen longitudinally collected peripheral blood samples from four recipients of single-donor BMT underwent clinically-indicated institutional SNP-based rtPCR chimerism analysis, based on a method modified from Harries et al.(3) Donor and pre-transplant recipient blood samples were genotyped. White cells were separated and counted using a Coulter Ac-T diff Analyzer (Beckman Coulter, Lane Cove, New South Wales, Australia) then diluted with PBS to a concentration of approximately 5x10^6 white blood cells in 200uL. DNA extraction was performed using the QIAamp DNA Blood Mini-Kit (QIAGEN, Hilden, Germany) according to manufacturer instructions and concentration measured using the NanoDrop 2000 (Thermo Scientific, Wilmington, USA) to give a working solution of lOng/uL.

SNP-based allelic discrimination was performed using three primer-probe sets targeting TSC0955234, rs3918344 and rs338773(3). Genotyping from pre-transplant recipient and donor samples was used to confirm informative combinations. Each sample and positive control (50:50 mix of donor and recipient cells) was run in triplicate with a negative control. Each reaction consisted of 5uL working solution DNA, lOuL TaqMan Master Mix (Thermo Fisher Scientific, Waltham, MA, USA), 1uL primer/probe mix and 4uL distilled water. rtPCR was performed using the Corbett Rotorgene 3000 (QIAGEN, Hilden, Germany) running at 95° for 10 minutes followed by 40 cycles of 92° for 15 seconds and 60° for 60 seconds. Threshold definition and analysis was performed using the stock Rotor-Gene 6000 Series Software. Chimerism quantitation was performed using the delta delta cycle threshold method.
Blinded chimerism analysis was also performed on the same samples using the CNV method and chimeric fractions compared.

The chimerism fraction measured by SNP assay ranged from 10-95% (the limits of sensitivity for this assay). The number of unique donor and recipient informative CNVs were as follows: transplant pair 1 (recipient 3, donor 2), pair 2 (recipient 3, donor 4), pair 3 (recipient 1, donor 2) and pair 4 (recipient 5, donor 2). Chimerism fraction measured using the CNV methodology correlated linearly with SNP assay results ($r=0.997$, 95% CI 0.992-0.999, $p<0.001$) (Figure 12, right panel).

Informativity Analysis

Genotyping results from a series of 32 recipients (and 35 donor genomes) who had undergone CNV chimerism analysis for clinical indications at our institution were reviewed. The number of markers tested and the number of markers that were informative for each individual in the transplant arrangement was determined. The number of individuals meeting the target of three informative markers was calculated. The contribution of multi-donor transplant arrangements and relatedness between transplant pairs to reduced marker informativity was tested.

At least one informative marker was identified in all of the 67 donor and recipient genotyping data reviewed (Figure 13). 52/67 (78%) had three or more informative markers. 63/67 (94%) had two or more informative markers. In 4/15 (26%) of cases with less than three informative markers, incomplete genotyping using only a 15-16 marker panel had been performed due to need for expedited results. The likelihood of obtaining less than three informative markers was no different between donors and recipients (OR 1.3, 95% CI 0.36-5, $p=0.84$). 11/67 (16%) were part of multiple-donor transplant arrangements (i.e. the chimerism study had to distinguish the recipient and more than one possible donor genomes, either because of concurrent or prior transplantation). In 51 individuals where relatedness data was available, 18/51 (35%) were related. The proportion of tested CNV targets that were informative was lower in multiple-donor transplant arrangements (0.09 (0.05-0.13) vs 0.18 (0.13-0.23), p=0.003) and related pairs (0.10 (0.06-0.16) vs 0.2 (0.13-0.28), p<0.001). Despite this, there was no significant increase in the number of individuals with less than three informative markers if part of a multiple-donor transplant arrangement (OR 3.7, 95% CI 0.75-18.3, p=0.10) or related (OR 3.5, 95% CI 0.76-17.2, p=0.08).

**Example 8 - Use of polymorphic "homozygous deleted" copy number variants for monitoring engraftment and minimal residual disease in a complex bone marrow transplant exemplifies advantages over current methods for routine monitoring.**

Polymorphic "homozygous deleted" (0-copy PCR product) copy number variants were used to distinguish four different genomes in a patient following HSCT. The patient was an 18 year old male with an X-linked, immune deficiency disorder (Wiskott-Aldrich syndrome). The patient recently received a second bone marrow transplant from an unrelated male donor having 15 years earlier received a transplant (failing) from his similarly affected brother, who had himself received a transplant from an aunt (i.e. a piggy-back transplant).

Monitoring engraftment was investigated in this case by using a research chimerism assay based on ubiquitous, highly heterozygous copy number deletions. Droplet digital PCR (ddPCR) was used to assess a panel of common large copy number deletions (Table 1) in DNA samples.

Blood samples were available from the brother, aunt and 18 year old male recipient (obtained pre transplant). Blood samples were also collected from the unrelated male donor and the 18 year old male recipient pre-transplant #2 which potentially consisted of DNA from the aunt, brother and himself.

Genomic DNA was isolated from cells from all blood samples. All samples were genotyped to identify "homozygous deleted" (0-copy PCR product), "heterozygous deleted" (1-copy PCR product) and "homozygous non-deleted" (2-copy PCR product) CNDs using a panel of 38 CNDs as shown in Table 1 using the Bio-Rad QX200 Droplet Digital system (Bio-red, Pleasanton, CA). CND primer sequences are shown in Table 7. A non-polymorphic target sequence within the Angiotensin I converting enzyme (ACE; #NM_000789) was used as a homozygous (2-copy PCR product) control.

PrimerQuest (Integrated DNA Technologies), an online primer design tool was used to generate PCR Primer and Zen double-quenched probes (DQP-ZEN and Iowa Black FQ quenchers for all ddPCR assays. The specificity of each primer pair was tested by either ddPCR or High Resolution Melt Curve analysis on the ViiA 7 Real time PCR system (Applied Biosystems) with gel electrophoresis. To be able to perform duplex assays, the CND and ACE probes were generated with either fluorescent reporting dye FAM or HEX or VIC. Unique CNDs were identified for the aunt (three heterozygous deleted (1-copy PCR product) CNDs), the brother (two heterozygous deleted (1-copy PCR product) CNDs) and the recipient (one heterozygous deleted (1-copy PCR product) CND). These were used to determine the proportional contribution of each in the pre-transplant #2 sample. Almost all DNA was shown to originate from the recipient (98.5%); the remaining 1.5% originated from the aunt and none was contributed by the brother. To measure engraftment in post-transplant samples, two heterozygous deleted (1-copy PCR product) CNDs (Figure 14B) were identified in donor DNA which were homozygous deleted (0-copy PCR product) in the pre-transplant #2 DNA sample. Quantitation of these markers on five occasions over a period of 180 days post-transplant showed 100% engraftment (Figure 15). Figure 2 highlights the ability of this approach to monitor reestablishment of a patient's own bone marrow over time following HSCT. To measure minimal residual disease, one homozygous deleted (0-copy PCR product) (CND1) and five heterozygous deleted (1-copy PCR product) CNDs (CNDs 3,5,6,9 and 10; Figure 14A) were identified in the transplant #2 DNA sample which were homozygous deleted (0-copy PCR product) in the donor. None of these markers were detectable in the post-transplant samples (Figure 14).

This case exemplifies the potential for using polymorphic CNDs for monitoring engraftment and residual disease in any allogeneic bone marrow transplant. Despite the close genetic relationships of the original 'piggy-back' donors and recipient, unique CND markers were identified for all four individuals involved in the second transplant's work-up and monitoring. This required screening an extended panel of 38 CNDs to identify unique markers for each of the four individuals involved in the latest and previous the transplants. In a typical single donor situation at least five homozygous deleted (0-copy PCR product) CNDs from a panel of only ten common, highly heterozygous CNDs would likely be identified. Half of these CNDs would be expected to be informative, ie heterozygous deleted (1-copy PCR product) or homozygous deleted (0-copy PCR product) in the donor (Table 1).

Combining the CND approach with droplet digital PCR provides highly sensitive detection of low levels of chimerism. One unique advantage of using polymorphic CNDs over sequence polymorphisms such as SNPs, indels or microsatellites is the use of homozygous deleted alleles (0-copy PCR product) which both in theory and practice provides a zero recipient background against which donor DNA can be quantified robustly and with high sensitivity. Moreover, the use of multiple CNDs gives independent measurements of donor, recipient and total DNA levels. Accordingly, the above method allows for internal validation of donor and recipient DNA levels by cross checking against total DNA levels in sample.

**Example 9** - **Cord blood contamination**

Polymorphic "homozygous deleted" (0-copy PCR product) copy number variants were used to assess whether genetically distinct cell populations (maternal and fetal) were present in a cord blood sample. Unique CND polymorphisms were identified for the mother (two heterozygous deleted (0-copy PCR product) CND polymorphisms; CND1B, CND3B; marked * Figure 16) by assessing genomic DNA isolated from a blood sample obtained from the mother. These unique CND polymorphisms were used to assess the proportional contribution of the mother (if any) to the cord blood sample. A non-polymorphic target sequence within the Angiotensin I converting enzyme was used as a homozygous control (2-copy PCR product). Unique maternal CND polymorphisms CND1B and CND3B were not detected in the genomic DNA isolated from the cord blood sample. This result indicates that all isolated DNA from the cord blood sample originated from the foetus. A unique CND polymorphism was identified for the foetus (heterozygous deleted; CND12). CND7B was homozygous deleted (2-copy PCR product) and CND9B was heterozygous-deleted (1-copy PCR product) in both mother and foetus. CND15 was homozygous deleted (0-copy PCR product) in the mother and heterozygous deleted (1-copy PCR product) in the foetus (Figure 16).

This case exemplifies the potential for using polymorphic CNDs for identifying contamination of a cord blood sample. Despite the close genetic relationships of the mother and foetus, unique CND markers were identified for both individuals.

**Example 10** - **Materials and methods**

Sample processing and DNA extraction

Blood samples are generally collected via venepuncture in the presence of EDTA and are processed within 4-6 hours after blood collection. Blood samples are centrifuged at 1600g for 10 minutes in 15mL falcon tubes at 4°C to separate blood cells from plasma. DNA is extracted using the Chemagic DNA Blood Kit (CMG-1074; Perkin Elmer, Waltham, MA) according to the manufacturer’s instructions.

Genotyping samples

Samples can be genotyped to identify homozygous deleted (0-copy PCR product), heterozygous deleted (1-copy PCR product) or homozygous non-deleted (2-copy PCR product) CNDs by assessing a panel of 38 shown in Table 1 using primers shown in Table 7. Genotyping assays can be performed using a digital PCR (ddPCR) system (e.g. Bio-Rad QX200 Droplet Digital system (Bio-red, Pleasanton, CA)) or Real-Time PCR. A non-polymorphic target sequence within the Angiotensin I converting enzyme (ACE) can be used as a homozygous control (2-copy PCR product). ACE primer sequences are also shown in Table 7.

Alternatively, PrimerQuest (Integrated DNA Technologies), an online primer design tool can be used to generate PCR Primer and Zen double-quenched probes (DQP-ZEN and Iowa Black FQ quenchers) for all ddPCR assays. The specificity of each primer pair shown in Table 7 for the associated copy number variations shown in Table 1 has been tested by both High Resolution Melt Curve analysis on the ViiA 7 Real time PCR system (Applied Biosystems) and gel electrophoresis. To be able to perform duplex assays, CND and ACE probes can be generated with either fluorescent reporting dye FAM or HEX.

ddPCR

ddPCR assays can be performed according to the manufacturer’s instructions. In brief, 25ul of ddPCR reaction mixture is assembled including 2x Droplet PCR Supermix (Cat#1863024), primers(900nM), probes(250nM) and 2.5ul of DNA template. The 20ul ddPCR mixture and 70ul of droplet generator oil (cat#1863004) are then loaded into the Bio-Rad DG8 Cartridge (cat#1864008) and the cartridge placed is placed into the Bio-Rad QX200 Droplet Generator. The droplet preparations are transferred to an Eppendrof Twin Tec PCR Semi-skirted 96 well plate (cat#Epp0030128.605), which is heat sealed using the Bio-Rad PX1 PCR Plate Sealer and then placed into a Bio-Rad C1000TM Thermal Cycler for PCR. ’No-template controls’ were included in each ddPCR run. After PCR, plates are read by a Bio-Rad QX200 Droplet Reader.

ddPCR data is analyzed using the Bio-Rad QuantaSoft software. An amplitude threshold can be applied using the ’no-template control’ droplet amplitude level to distinguish positive from negative droplets. The absolute concentration data (copy/$\mu$l) can be converted to GE/mL using the formula described by Lo et al. Am J HumGenet. 62:768 1998.

CND ddPCR assay development

ddPCR assays for HSCT transplant recipient samples are developed with Zenprobes (IDT) based on the panel of 38 copy number deletion markers shown in Table 1. Primers sequences are shown in Table 7. Each ddPCR assay uses the copy number deletion -specific internal PCR primers and a target specific Zen probe (Table 7). The CND assays are labelled with FAM or HEX fluorescence reporter. Quantitative PCRs are performed in duplicate in a reaction volume of 10μL and the reactions are performed on the ViiA 7 Real Time PCR system (Applied Biosystems, Melbourne, Australia). ViiA 7 software version 1.2 (Applied Biosystems) is used for the data analysis. The quantification cycle (Cq) was based on the intersection between the amplification curve and an empirically adjusted threshold. PCR efficiencies have been standardized across all 38 CND qPCR assays; all assays had a slope between -3.3 and -3.6 (average of -3.4).

As an example, for the 1000GE standard curve point, the DNA input volume was 2.5μl so the concentration of input DNA was 400ϋE/μl. The final concentration of DNA in the reaction volume was 100GE/μl (1000GE total). The Cq was based on the intersection between the amplification curve and an empirically adjusted threshold.

Design of primers and probes

A panel of 38 CNV-deletion (CND) markers and Angiotensin I converting enzyme (ACE) control was developed for DDPCR assays. Angiotensin I converting enzyme (ACE) is present in 2-copies per diploid human genomic DNA and thus, can be used as a control to measure the total isolated genomic DNA level. PrimerQuest an online Primer designing tool from Integrated DNA Technologies was used to generate PCR Primer and Zen double-quenched probes (DQP-ZEN and Iowa Black FQ quenchers) for all ddPCR assays(IDT Zen probes have showed to be able to increase the accuracy and reliability of 5' nuclease qPCR experiments). The specificity of each primer pair was tested by both High Resolution Melt Curve analysis on the ViiA 7 Real time PCR system (Applied Biosystems) and gel electrophoresis. To be able to duplex the ddPCR assays in one ddPCR reaction, the CND markers and ACE were randomly generated with either fluorescent reporting dyes FAM or HEX. Sequence details of the 38 CND panel and the ACE control are shown below in Table 7.

Table 7:

| CND ID | Chromosome # | Internal PCR | | Amp Size (bp) | Probe |
|---|---|---|---|---|---|
| | | Forward Primer | Reverse Primer | | Probe |
| | | Sequence | Sequence (AS) | | Sequence(S) |
| 12 | 7 | AGCCTCAATTCAGGACCTA | CTCCTTGGAGAGCTTGTTATC | 72 | AATCTTAGGCACCCATCCACTGCC |
| 13 | 10 | TGTCATGTGCAATAGAGACTGTT | TTCTGGCCAGGGCAATTC | 72 | TCAAGGAAAGTATATGGACCACATCCAGAC |
| 14 | 2 | ACTGGCTTCCAAATGTGACTAA | TAACCTGCAGCTCTGGGA | 69 | ACAGTGCAACAGCCCTTGGC |
| 15 | 13 | ACTGACACACTCAAGCTATGG | AGCATAAATCTCTGCTCCCTTT | 73 | TTAAGCGCTCTCCATAACCACGCA |
| 16 | 2 | AGCAAATGATGATGTTGTATGAGC | TAAATGCCAGCACCCTGCTA | 68 | CACAGGCAGCTATTTCCATGCGTG |
| 17 | 1 | GCCATGCCTCCCACATT | TGGCCCTAACAGTTTGGATATG | 76 | TCAACATGAGATTTGAGCAGGGACA |
| 18 | 9 | TCACAGGTCCTACCAGCTTTA | GACGAAAGATGTCTTAATTTGGTTCAG | 70 | ACAACACCAGGCAGGTTGACAT |
| 20 | 2 | CCAGCGTCTTCTCTTATGTCAG | ACAAGCCAACAACGTACAGG | 63 | CCAGCCTGTGCCGATGTTCT |
| 21 | 2 | TTTGGTGACTGATACATGGCT | CCTAGCTACTATTCCCTCTAGAACA | 80 | TTGATGCATGCCTTTGCTTGTCCA |
| 22 | 13 | ATATCCTCTGGATTTGACAGTG | ATGTTGAGAGCATTGGACTT | 66 | TCCTTGTCTTCCTGGCATGACAGG |
| 23 | 22 | TTCCAAAGACACAAGGCAGTA | GGACATTCCTGATGGGAGAAA | 70 | ACCCTCCTTACCTCCTTTCACCCA |
| 24 | 6 | CCAAGTATGATGTTATTTGTGGGA | CACAAGTAGATTCAGATTAGAAGGG | 83 | TCATAGATGCTCTTTATCCAGTGCAGT |
| 25 | 8 | CATATTGAGATCCAGAAACAACCTT | AGAAATAAACTGAGGCCTGGATA | 80 | TCTAGGTAGGATCGTTGTACTCTAACA |
| 26 | 1 | CCTTACTGACACTCTCTGTTTCTT | GAGCACCCAGTAGGAATAGAATTAG | 83 | TCTGTTGCTTCCTGCAACTTGAGC |
| 27 | 9 | CAGGGAGAGAGTGTAGAAAGAATAG | GCC7GATCTCTAAACACTGGAG | 83 | AAGAACCAGGGTCTGGGAGTGAG |
| 28 | 6 | ACACTCCAATTCCAACTCTACC | GAGAAATCAGAGGCCCAAAGA | 78 | ACTCAGCTGTGTAATTGGGCACATGA |
| 29 | 15 | GCCTGTGCCTACCTGTTTAATA | AAATCCACAGCCAGTACCTTC | 81 | TCTGAGTCTGGGTTCAGTAGCCCA |
| 30 | 15 | GAGAAATCCATCTTCAGGTCAG | AAAGACCCTTCACACTTTGTT | 72 | TGTCCATCATCCAATGTTGATTGAGC |
| 31 | 1 | TACACCAGTGGAAGGACTAT | CCCTACTAATGACATCCAGAATAA | 69 | TGTAGGCCATTATCCCTGTTGGCC |
| 32 | 1 | GAGGAATGAGAGACAACACTTC | CCTTCTGTAGCCTCTTCCTA | 74 | TCATAGGGAGACCATGAGTTGCTGC |
| 33 | 1 | GGCAGCAATAATCACCTCTGA | CAATCTGATAAGCTTTCTAGGGTAGT | 76 | CCCAAAGGACCCATTTGGCTGAAT |
| 34 | 20 | TTCTTCCCTTTCCAGACTCA | GAAAGCCAGAGAATGAGAGAG | 84 | AAATACATGAGTCTAAGAGGACCAGTATCC |
| 36 | 4 | CTAGAACCTTCCATCCTTAATGT | GCTCCTACATGGCTACAAAG | 83 | TGATGACGCTGTTGGTGAAGGAGA |

(continued)

| | | Internal PCR | | | |
|---|---|---|---|---|---|
| CND ID | Chromosome # | Forward Primer Sequence | Reverse Primer Sequence (AS) | Amp Size (bp) | Probe Sequence(S) |
| 37 | 5 | GCTTTGTTGGGAGAGTTAGA | CAATTCATTGAGAAGTGCCAA | 71 | TAGCAGCCCATGTCTCCAAACCAA |
| 38 | 6 | AAAGCAGCTCAACCTCTTAAT | TGAAAGATTCCTGAGTTATTAGCC | 85 | AGACAGTAAACCAAATTCCACATCCCAGT |
| 39 | 7 | CCGACTTTCTACTTATTCCACA | GCTGTATAAACCCTCCACAG | 76 | CGAACCACTGTTCTACGTGTGAGGC |
| 41 | 5 | CCCAAACTCCTCTCATCTTTC | AAGGCTGATTAGCCGTAATG | 70 | AAACCATGCCACATCCTGACGTTT |
| 01B | 1 | AAAGTCAACTTCTCTGTGCGA | TTATTCTTCCACCATTATCCCTCAT | 67 | AATACACCAGCACCAGGGCT |
| 02B | 18 | TGTCGTTTCTAAGGATGGGAAAG | GTTTCATGAAGCTGCCTCTCT | 74 | TGAAGTGCTTCTCCTCCCTGATCCA |
| 03B | 8 | ATGCCTGGCTTCATGTTACT | AATTACTGACCTAGACCTTTCAAGA | 75 | TCTCCCTCTGCCCATAATATAGAGCA |
| 04B | 14 | GTGTGGACATTGGTTCCTTT | TTAGGGCAAACAAGATGAGAAA | 78 | TCTGTGTATACACAAGAGGGAGCTATGC |
| 05B | 16 | TTTCTTCCAGCAGAGCTCAC | GCCAGCCCTGGATGAAT | 70 | TTGCCTCCCACACATCTTCACTAGC |
| 06B | 7 | TCACTCTTCCCACCCTCTTAT | CATCTCAAGAAAGAGGGCTCAA | 72 | TGTACAATGACTCAGTGGCTCATCAAC |
| 07B | 13 | CCCAACTCAATTTCAAGCTG | CCAAAGATCCAATTAGCACATC | 73 | CCAATGTGACATCACTGAATTCAGAGGT |
| 08B | 8 | ACCTATCAACTTGGTTACCCTAAA | TTTGTGTGGGAATCTTTATCTGTG | 80 | ACACCATGGACCTATAGAAATAGCTGGC |
| 09B | 18 | TGTCCCAATGCACAGACTT | AAACAGGGTGGGCCTAAA | 67 | TGCATTCACCGTAAGGGAACAGAGC |
| 10C | 10 | CTTTCGGTTCTCTCCAACCA | AACAACTTCCCTGAGACGTG | 74 | CCCATCCTCTCTTAGCACAACCATGA |
| 19 | 2 | CACACCAAATTGTTAAGAGTTACC | GTCCTGGTAACAAATCCAGTTAATA | 80 | AGACACAGGATTAGAGAAGATGCCT |
| ACE | ACE | GGTCTTTAGTGGGCGTACC | TCCTCTCACCGAAGATACCA | | |

Statistical Analysis

All statistical analyses are performed using the R statistical programming language (http://www.r--project.org/).

## Claims

1. A method of measuring chimerism in a biological sample obtained from a subject comprising two or more genetically distinct cell populations, the method comprising:

   isolating the genomic DNA from the cells in the sample,
   determining the level of genomic DNA in the sample from a first genetically distinct cell population based on a copy number variation (CNV) polymorphism that is an informative marker of the first genetically distinct cell population,
   determining the total level of genomic DNA isolated from the genetically distinct cell populations in the biological sample,
   wherein the level of genomic DNA in the sample from said first genetically distinct cell population and the total level of genomic DNA isolated from the genetically distinct cell populations provides the measure of chimerism in the sample.

2. The method of claim 1, further comprising determining the level of genomic DNA in the sample from a second genetically distinct cell population based on a CNV polymorphism that is an informative marker of the second genetically distinct cell population, wherein the levels of genomic DNA from said first and second genetically distinct cell populations and the total level of genomic DNA isolated from the genetically distinct cell populations provide the measure of chimerism in the sample.

3. The method of claims 1 or 2, wherein an informative marker of a genetically distinct cell population comprises:

   - a CNV polymorphism that is homozygous deleted in isolated genomic DNA from the first cell population and homozygous non-deleted in isolated genomic DNA from a second cell population; or,
   - a CNV polymorphism that is homozygous deleted in the isolated genomic DNA from a second cell population and homozygous non-deleted in the isolated genomic DNA from the first cell population.

4. The method of any one of claims 1 to 3, further comprising validating the level of isolated DNA from a genetically distinct cell population by determining the total isolated genomic DNA in the sample,
   wherein levels of isolated genomic DNA from the genetically distinct cell populations equal to the level of total DNA indicates that the levels of isolated genomic DNA are validated.

5. The method of claim 4, wherein the measure of chimerism is expressed as:

   - a percentage of genetically distinct cells in the total cell population in the sample; or
   - a ratio of genetically distinct cells in the sample (first cell population:second cell population).

6. The method of any one of claims 1 to 5, wherein the CNV polymorphism is a copy number deletion (CND) polymorphism.

7. The method of claim 6, wherein an informative marker of a genetically distinct cell population comprises:

   (i)

   - a CND polymorphism that is homozygous deleted in isolated genomic DNA from the first cell population and heterozygous or homozygous non-deleted in the isolated genomic DNA from a second cell population; and/or,
   - a CND polymorphism that is homozygous deleted in isolated genomic DNA from a second cell population and heterozygous or homozygous non-deleted in the isolated genomic DNA from the first cell population;

   (ii)

   - at least two CND polymorphisms that are homozygous deleted in isolated genomic DNA from the first cell population and heterozygous or homozygous non-deleted in isolated genomic DNA from a second cell population; and/or,
- at least two CND polymorphisms that are homozygous deleted in isolated genomic DNA from the second cell population and heterozygous or homozygous non-deleted in the isolated genomic DNA from the first cell population;

(iii)

- at least three CND polymorphisms that are homozygous deleted in isolated genomic DNA from the first cell population and heterozygous or homozygous non-deleted in isolated genomic DNA from a second cell population; and/or,
- at least three CND polymorphisms that are homozygous deleted in isolated genomic DNA from the second cell population and heterozygous or homozygous non-deleted in the isolated genomic DNA from the first cell population;

(iv)

- at least 4, at least 5, or at least 6, or at least 7, or at least 10, or at least 20, or at least 30, or at least 38 CND polymorphisms that are homozygous deleted in isolated genomic DNA from the first cell population and heterozygous or homozygous non-deleted in isolated genomic DNA from a second cell population; or,
- at least 4, at least 5, or at least 6, or at least 7, or at least 10, or at least 20, or at least 30, or at least 38 CND polymorphisms that are homozygous deleted in isolated genomic DNA from the second cell population and heterozygous or homozygous non-deleted in the isolated genomic DNA from the first cell population.

**8.** The method of any one of claims 1 to 7, wherein determining the level of isolated genomic DNA from the genetically distinct cell populations comprises subjecting the DNA to an amplification reaction with primers which target the CNV polymorphisms that are informative markers of the genetically distinct cell populations, wherein the primers preferably target an internal region of the informative CNV(s) and the CNV polymorphisms are optionally amplified using primers selected from Table 7.

**9.** The method of any one of claims 1 to 8, wherein the CNV polymorphisms are selected from the CND polymorphisms listed in Table 1.

**10.** The method of any one of claims 1 to 9, wherein the biological sample:

- has been processed to substantially remove circulating cell free DNA from the sample;
- is a blood sample;
- is peripheral blood mononuclear cells;
- is a purified immune cell population or purified mixture of immune cells;
- is a purified population of white blood cells;
- is a purified population of T-cells, B-cells, granulocytes or a mixture thereof;
- is purified to provide at least two cell populations and chimerism is measured in each cell population;
- is purified to provide a population of B-cells and a population of granulocytes and chimerism is measured in each cell population;
- is purified to provide at least three cell populations and chimerism is measured in each cell population;
- is purified to provide a population of B-cells, a population of granulocytes and a population of T-cells and chimerism is measured in each cell population; or,
- is cord blood.

**11.** The method of any one of claims 1 to 10, wherein the genetically distinct cell populations comprise self cells (first cell population) and non-self cells (second cell population), wherein optionally, the self cells (first cell population) are foetal cells and the non-self cells (second cell population) are maternal cells.

**12.** The method of any one of claims 1 to 11, wherein the subject is a haematopoietic stem cell transplant (HSCT) recipient.

**13.** The method of any one of claims 1 to 12, wherein

- the biological sample has been obtained from a HSCT recipient and comprises a self cell population and a non-self cell population, and the level of non-self cells determines the level of engraftment in a HSCT recipient, preferably, wherein a level of non-self cells greater than about 90% indicates engraftment of the transplant;
- the biological sample has been obtained from a HSCT recipient and comprises a self cell population and a non-self cell population, and the level of self cells monitors the reestablishment of a haematopoietic stem cell transplant (HSCT) recipient's own bone marrow post HSCT transplant, preferably, wherein:

(a) a level of self cells greater than about 10% indicates reestablishment of the HSCT recipient's bone marrow;
(b) an increase in the level of self cells and/or a decrease in the level of non-self cells indicates reestablishment of the HSCT recipient's bone marrow; or,
(c) an increase in the level of self cells and/or a decrease in the level of non-self cells indicates that immunosuppressive therapy should be administered to the HSCT recipient or the HSCT recipient's immunosuppressive therapy should be modified;

- the biological sample has been obtained from a HSCT recipient and comprises a self cell population and a non-self cell population, and an increase in the level of self cells or a decrease in the level of non-self cells monitors minimal residual disease in a patient in need thereof, preferably, wherein a level of self cells greater than about 1% indicates relapse of the HSCT recipient's haematological disorder; or,
- the biological sample is a cord blood sample and comprises a foetal cell population and a maternal cell population, and the level of chimerism in the cord blood sample determines the level of maternal contamination of a cord blood sample, preferably, wherein a ratio of chimerism (foetal cells:maternal cells) greater than about 1:100, about 1:50, about 1:20, about 1:10, about 3:20 indicates the cord blood sample is not suitable for use in a haematopoietic stem cell transplant.

**14.** The method of claims 13, wherein the HSCT recipient received a HSCT for the treatment of a haematological disorder, preferably a haematological malignancy, more preferably, leukaemia and optionally, wherein:

- a level of self cells greater than about 1% indicates relapse of the HSCT recipient's haematological disorder;
- an increase in the level of self cells and/or a decrease in the level of non-self cells indicates relapse of the HSCT recipient's haematological disorder; or,
- an increase in the level of self cells and/or a decrease in the level of non-self cells indicates that therapy for the haematological disorder should be administered to the HSCT recipient or the HSCT recipients therapy should be modified.

**15.** A method of measuring HSCT engraftment in a biological sample obtained from a HSCT transplant recipient, the method comprising:

isolating the genomic DNA from the cells in the sample,
determining the level of genomic DNA in the sample from the self and non-self cells based on copy number variation (CNV) polymorphisms that are informative markers of the self and non-self cells,
determining the total level of genomic DNA isolated from the self and non-self cells in the sample,
wherein the informative markers comprise:

- a CND polymorphism that is homozygous deleted in isolated genomic DNA from self cells and heterozygous or homozygous non-deleted in isolated genomic DNA from non-self cells; and
- a CND polymorphism that is homozygous deleted in the isolated genomic DNA from non-self cells and heterozygous or homozygous non-deleted in the isolated genomic DNA from self cells;

wherein the level of genomic DNA in the sample from the self and non-self cells and
the total level of genomic DNA isolated from the self and non-self cells provides the measure of chimerism in the sample, and a level of genomic DNA in the sample from the non-self cells greater than about 90% indicates engraftment of the HSCT recipient's bone marrow.

**16.** A method of measuring minimal residual disease in a biological sample obtained from a HSCT recipient, the method comprising:

isolating the genomic DNA from the cells in the sample,
determining the level of genomic DNA in the sample from self and non-self cells based on copy number variation (CNV) polymorphisms that are informative markers of the self and non-self cells,
determining the total level of genomic DNA isolated from the self and non-self cells in the sample,
wherein the informative markers comprise:

- a CND polymorphism that is homozygous deleted in isolated genomic DNA from self cells and heterozygous or homozygous non-deleted in isolated genomic DNA from non-self cells; and
- a CND polymorphism that is homozygous deleted in the isolated genomic DNA from non-self cells and heterozygous or homozygous non-deleted in the isolated genomic DNA from self cells;

wherein the level of genomic DNA in the sample from the self and non-self cells and
the total level of genomic DNA isolated from the self and non-self cells provides the measure of chimerism in the sample, and a level of self cells greater than about 1% indicates reestablishment of the HSCT recipient's bone marrow.

**17.** A method of measuring maternal contamination in a cord blood sample comprising:

isolating the genomic DNA from the cells in the cord blood sample,
determining the level of genomic DNA in the sample from foetal and maternal cells based on copy number variation (CNV) polymorphisms that are informative markers of the foetal and maternal cells,
determining the total level of genomic DNA isolated from the foetal and maternal cells in the sample,
wherein the informative markers comprise:

- a CND polymorphism that is homozygous deleted in isolated genomic DNA from foetal cells and heterozygous or homozygous non-deleted in isolated genomic DNA from maternal cells; and
- a CND polymorphism that is homozygous deleted in the isolated genomic DNA from maternal cells and heterozygous or homozygous non-deleted in the isolated genomic DNA from foetal cells;

wherein the level of genomic DNA in the cord blood sample from the foetal and
maternal cells and the total level of genomic DNA isolated from the foetal and maternal cells provides the measure of chimerism in the cord blood sample, and a ratio of foetal cells:maternal cells greater than about 1:100, about 1:50, about 1:20, about 1:10, about 3:20 indicates the cord blood sample is not suitable for use in a haematopoietic stem cell transplant.

## Patentansprüche

**1.** Verfahren zur Messung von Chimärismus in einer biologischen Probe, die von einem Probanden erhalten wurde, der zwei oder mehr genetisch unterschiedliche Zellpopulationen umfasst, wobei das Verfahren umfasst:

Isolierung der genomischen DNA aus den Zellen der Probe,
Bestimmung des Gehalts an genomischer DNA in der Probe aus einer ersten genetisch unterschiedlichen Zellpopulation auf der Grundlage eines Kopienzahlvariations- (CNV-) Polymorphismus, der ein informativer Marker der ersten genetisch unterschiedlichen Zellpopulation ist,
Bestimmung des Gesamtgehalts der aus den genetisch unterschiedlichen Zellpopulationen in der biologischen Probe isolierten genomischen DNA,
wobei der Gehalt an genomischer DNA in der Probe aus der ersten genetisch unterschiedlichen Zellpopulation und der Gesamtgehalt an genomischer DNA, die aus den genetisch unterschiedlichen Zellpopulationen isoliert wurde, das Maß des Chimärismus in der Probe bereitstellt.

**2.** Verfahren nach Anspruch 1, weiter umfassend das Bestimmen des Gehalts an genomischer DNA in der Probe aus einer zweiten genetisch unterschiedlichen Zellpopulation auf der Grundlage eines CNV-Polymorphismus, der ein informativer Marker der zweiten genetisch unterschiedlichen Zellpopulation ist, wobei die Gehalte an genomischer DNA aus der ersten und der zweiten genetisch unterschiedlichen Zellpopulation und der Gesamtgehalt an genomischer DNA, die aus den genetisch unterschiedlichen Zellpopulationen isoliert wurde, das Maß an Chimärismus in der Probe bereitstellen.

**3.** Verfahren nach Anspruch 1 oder 2, wobei ein informativer Marker für eine genetisch unterschiedliche Zellpopulation umfasst:

- einen CNV-Polymorphismus, der in isolierter genomischer DNA aus der ersten Zellpopulation homozygot deletiert ist und in isolierter genomischer DNA aus einer zweiten Zellpopulation homozygot nicht deletiert ist; oder
- einen CNV-Polymorphismus, der in der isolierten genomischen DNA aus einer zweiten Zellpopulation homozygot deletiert ist und in der isolierten genomischen DNA aus der ersten Zellpopulation homozygot nicht deletiert ist.

**4.** Verfahren nach einem der Ansprüche 1 bis 3, weiter umfassend die Validierung des Gehalts an isolierter DNA aus einer genetisch unterschiedlichen Zellpopulation durch Bestimmung der gesamten isolierten genomischen DNA in der Probe,
wobei Gehalte an isolierter genomischer DNA aus den genetisch unterschiedlichen Zellpopulationen gleich dem Gehalt an Gesamt-DNA anzeigt, dass der Gehalt an isolierter genomischer DNA validiert ist.

**5.** Verfahren nach Anspruch 4, wobei das Maß des Chimärismus ausgedrückt wird als:

- ein Prozentsatz genetisch unterschiedlicher Zellen in der gesamten Zellpopulation in der Probe; oder
- ein Verhältnis von genetisch unterschiedlichen Zellen in der Probe (erste Zellpopulation:zweite Zellpopulation).

**6.** Verfahren nach einem der Ansprüche 1 bis 5, wobei der CNV-Polymorphismus ein Kopienzahl-Deletions- (CND-) Polymorphismus ist.

**7.** Verfahren nach Anspruch 6, wobei ein informativer Marker für eine genetisch unterschiedliche Zellpopulation umfasst:

(i)

- einen CND-Polymorphismus, der in isolierter genomischer DNA aus der ersten Zellpopulation homozygot deletiert und in der isolierten genomischen DNA aus einer zweiten Zellpopulation heterozygot oder homozygot nicht deletiert ist; und/oder
- einen CND-Polymorphismus, der in isolierter genomischer DNA aus einer zweiten Zellpopulation homozygot deletiert und in der isolierten genomischen DNA aus der ersten Zellpopulation heterozygot oder homozygot nicht deletiert ist;

(ii)

- mindestens zwei CND-Polymorphismen, die in isolierter genomischer DNA aus der ersten Zellpopulation homozygot deletiert und in isolierter genomischer DNA aus einer zweiten Zellpopulation heterozygot oder homozygot nicht deletiert sind; und/oder
- mindestens zwei CND-Polymorphismen, die in isolierter genomischer DNA aus der zweiten Zellpopulation homozygot deletiert und in der isolierten genomischen DNA aus der ersten Zellpopulation heterozygot oder homozygot nicht deletiert sind;

(iii)

- mindestens drei CND-Polymorphismen, die in isolierter genomischer DNA aus der ersten Zellpopulation homozygot deletiert und in isolierter genomischer DNA aus einer zweiten Zellpopulation heterozygot oder homozygot nicht deletiert sind; und/oder
- mindestens drei CND-Polymorphismen, die in isolierter genomischer DNA aus der zweiten Zellpopulation homozygot deletiert und in der isolierten genomischen DNA aus der ersten Zellpopulation heterozygot oder homozygot nicht deletiert sind;

(iv)

- mindestens 4, mindestens 5 oder mindestens 6 oder mindestens 7 oder mindestens 10 oder mindestens 20 oder mindestens 30 oder mindestens 38 CND-Polymorphismen, die in isolierter genomischer DNA aus der ersten Zellpopulation homozygot deletiert sind und in isolierter genomischer DNA aus einer zweiten Zellpopulation heterozygot oder homozygot nicht deletiert sind; oder
- mindestens 4, mindestens 5 oder mindestens 6 oder mindestens 7 oder mindestens 10 oder mindestens 20 oder mindestens 30 oder mindestens 38 CND-Polymorphismen, die in isolierter genomischer DNA aus der zweiten Zellpopulation homozygot deletiert und in der isolierten genomischen DNA aus der ersten Zellpopulation heterozygot oder homozygot nicht deletiert sind.

**8.** Verfahren nach einem der Ansprüche 1 bis 7, wobei die Bestimmung des Gehalts an isolierter genomischer DNA aus den genetisch unterschiedlichen Zellpopulationen das Unterziehen der DNA einer Amplifikationsreaktion mit Primern umfasst, die auf die CNV-Polymorphismen abzielen, die informative Marker der genetisch unterschiedlichen Zellpopulationen sind, wobei die Primer bevorzugt auf eine interne Region der informativen CNV(s) abzielen und die CNV-Polymorphismen optional unter Verwendung von Primern amplifiziert werden, die aus Tabelle 7 ausgewählt sind.

**9.** Verfahren nach einem der Ansprüche 1 bis 8, wobei die CNV-Polymorphismen aus den in Tabelle 1 aufgeführten CND-Polymorphismen ausgewählt sind.

**10.** Verfahren nach einem der Ansprüche 1 bis 9, wobei die biologische Probe:

- verarbeitet wurde, um zirkulierende zellfreie DNA aus der Probe im Wesentlichen zu entfernen;
- eine Blutprobe ist;
- mononukleäre Zellen des peripheren Blutes sind;
- eine gereinigte Immunzellpopulation oder eine gereinigte Mischung von Immunzellen ist;
- eine gereinigte Population von weißen Blutkörperchen ist;
- eine gereinigte Population von T-Zellen, B-Zellen, Granulozyten oder eine Mischung davon ist;
- gereinigt wird, um mindestens zwei Zellpopulationen zu erhalten, und Chimärismus in jeder Zellpopulation gemessen wird;
- gereinigt wird, um eine Population von B-Zellen und eine Population von Granulozyten zu erhalten, und der Chimärismus in jeder Zellpopulation gemessen wird;
- gereinigt wird, um mindestens drei Zellpopulationen zu erhalten, und Chimärismus in jeder Zellpopulation gemessen wird;
- gereinigt wird, um eine Population von B-Zellen, eine Population von Granulozyten und eine Population von T-Zellen bereitzustellen, und Chimärismus in jeder Zellpopulation gemessen wird; oder
- Nabelschnurblut ist.

**11.** Verfahren nach einem der Ansprüche 1 bis 10, wobei die genetisch unterschiedlichen Zellpopulationen Selbst-Zellen (erste Zellpopulation) und Nicht-Selbst-Zellen (zweite Zellpopulation) umfassen, wobei optional die Selbst-Zellen (erste Zellpopulation) fötale Zellen sind und die Nicht-Selbst-Zellen (zweite Zellpopulation) mütterliche Zellen sind.

**12.** Verfahren nach einem der Ansprüche 1 bis 11, wobei der Proband ein Empfänger einer hämatopoetischen Stammzelltransplantation (HSCT) ist.

**13.** Verfahren nach einem der Ansprüche 1 bis 12, wobei

- die biologische Probe von einem HSCT-Empfänger erhalten wurde und eine Selbst-Zellpopulation und eine Nicht-Selbst-Zellpopulation umfasst, und der Gehalt an Nicht-Selbst-Zellen den Grad der Verpflanzung in einem HSCT-Empfänger bestimmt, wobei bevorzugt ein Gehalt an Nicht-Selbst-Zellen von mehr als etwa 90% die Verpflanzung des Transplantats anzeigt;
- die biologische Probe von einem HSCT-Empfänger erhalten wurde und eine Selbst-Zellpopulation und eine Nicht-Selbst-Zellpopulation umfasst, und der Gehalt an Selbst-Zellen die Wiederherstellung des eigenen Knochenmarks eines Empfängers einer hämatopoetischen Stammzelltransplantation (HSCT) nach der HSCT-Transplantation überwacht, bevorzugt wobei:

  (a) ein Gehalt an Selbst-Zellen von mehr als etwa 10 % eine Wiederherstellung des Knochenmarks des HSCT-Empfängers anzeigt;
  (b) ein Anstieg des Gehalts an Selbst-Zellen und/oder ein Rückgang des Gehalts an Nicht-Selbst-Zellen eine Wiederherstellung des Knochenmarks des HSCT-Empfängers anzeigt; oder
  (c) ein Anstieg des Gehalts an Selbst-Zellen und/oder ein Rückgang des Gehalts an Nicht-Selbst-Zellen anzeigt, dass dem HSCT-Empfänger eine immunsuppressive Therapie verabreicht wurde oder die immunsuppressive Therapie des HSCT-Empfängers geändert werden sollte;

- die biologische Probe von einem HSCT-Empfänger erhalten wurde und eine Selbst-Zellpopulation und eine Nicht-Selbst-Zellpopulation umfasst und ein Anstieg des Gehalts an Selbst-Zellen oder eine Abnahme des Gehalts an Nicht-Selbst-Zellen eine minimale Resterkrankung bei einem Patienten, der diese benötigt, überwacht, bevorzugt, wobei ein Gehalts an Selbst-Zellen von mehr als etwa 1% ein Rezidiv der hämatologischen Erkrankung des HSCT-Empfängers anzeigt; oder
- die biologische Probe eine Nabelschnurblutprobe ist und eine fötale Zellpopulation und eine mütterliche Zellpopulation umfasst, und der Grad des Chimärismus in der Nabelschnurblutprobe den Grad der mütterlichen Kontamination einer Nabelschnurblutprobe bestimmt,

bevorzugt, wobei ein Chimärismusverhältnis (fötale Zellen:mütterliche Zellen) von mehr als etwa 1:100, etwa 1:50, etwa 1:20, etwa 1:10, etwa 3:20 anzeigt, dass die Nabelschnurblutprobe nicht für die Verwendung in einer hämatopoetischen Stammzelltransplantation geeignet ist.

**14.** Verfahren nach Anspruch 13, wobei der HSCT-Empfänger eine HSCT zur Behandlung einer hämatologischen Erkrankung, bevorzugt einer hämatologischen Malignität, besonders bevorzugt Leukämie, erhalten hat, und optional, wobei:

- ein Gehalt an Selbst-Zellen von mehr als etwa 1% ein Rezidiv der hämatologischen Erkrankung des HSCT-Empfängers anzeigt;
- ein Anstieg Gehalts an Selbst-Zellen und/oder ein Rückgang des Spiegels der Nicht-Selbst-Zellen ein Rezidiv der hämatologischen Erkrankung des HSCT-Empfängers anzeigt; oder
- ein Anstieg des Gehalts an Selbst-Zellen und/oder ein Rückgang des Gehalts an Nicht-Selbst-Zellen anzeigt, dass dem HSCT-Empfänger eine Therapie für die hämatologische Erkrankung verabreicht werden sollte oder die Therapie des HSCT-Empfängers modifiziert werden sollte.

**15.** Verfahren zur Messung der HSCT-Transplantation in einer biologischen Probe, die von einem HSCT-Transplantatempfänger erhalten wurde, wobei das Verfahren umfasst:

Isolierung der genomischen DNA aus den Zellen der Probe,
Bestimmung des Gehalts an genomischer DNA in der Probe aus den Selbst- und Nicht-Selbst-Zellen auf der Grundlage von Kopienzahlvariations- (CNV-) Polymorphismen, die informative Marker der Selbst- und Nicht-Selbst-Zellen sind,
Bestimmung des Gesamtgehalts an genomischer DNA, die aus den Selbst- und Nicht-Selbst-Zellen der Probe isoliert wurde,
wobei die informativen Marker umfassen:

- einen CND-Polymorphismus, der in isolierter genomischer DNA von Selbst-Zellen homozygot deletiert ist und in isolierter genomischer DNA von Nicht-Selbst-Zellen heterozygot oder homozygot nicht deletiert ist; und
- einen CND-Polymorphismus, der in der isolierten genomischen DNA von Nicht-Selbst-Zellen homozygot deletiert ist und in der isolierten genomischen DNA von Selbst-Zellen heterozygot oder homozygot nicht-deletiert ist;

wobei der Gehalt an genomischer DNA in der Probe aus den Selbst- und Nicht-Selbst-Zellen und der Gesamtgehalt an genomischer DNA, die aus den Selbst- und Nicht-Selbst-Zellen isoliert wurde, das Maß für den Chimärismus in der Probe bereitstellt und ein Gehalt an genomischer DNA in der Probe aus den Nicht-Selbst-Zellen, der größer als etwa 90% ist, die Einpflanzung des Knochenmarks des HSCT-Empfängers anzeigt.

**16.** Verfahren zur Messung der minimalen Resterkrankung in einer biologischen Probe, die von einem HSCT-Empfänger erhalten wurde, wobei das Verfahren umfasst:

Isolierung der genomischen DNA aus den Zellen der Probe,
Bestimmung des Gehalts an genomischer DNA in der Probe aus Selbst- und Nicht-Selbst-Zellen auf der Grundlage von Kopienzahlvariations- (CNV-) Polymorphismen, die informative Marker der Selbst- und Nicht-Selbst-Zellen sind,
Bestimmung des Gesamtgehalts an genomischer DNA, die aus den Selbst- und Nicht-Selbst-Zellen der Probe isoliert wurde,
wobei die informativen Marker umfassen:

- einen CND-Polymorphismus, der in isolierter genomischer DNA von Selbst-Zellen homozygot deletiert ist und in isolierter genomischer DNA von Nicht-Selbst-Zellen heterozygot oder homozygot nicht deletiert ist; und
- einen CND-Polymorphismus, der in der isolierten genomischen DNA von Nicht-Selbst-Zellen homozygot deletiert ist und in der isolierten genomischen DNA von Selbst-Zellen heterozygot oder homozygot nicht-deletiert ist;

wobei der Gehalt an genomischer DNA in der Probe aus den Selbst- und Nicht-Selbst-Zellen und der Gesamtgehalt an genomischer DNA, die aus den Selbst- und Nicht-Selbst-Zellen isoliert wurde, das Maß für den Chimärismus in der Probe bereitstellt, und ein Gehalt an Selbst-Zellen von mehr als etwa 1% die Wiederherstellung des Knochenmarks des HSCT-Empfängers anzeigt.

**17.** Verfahren zur Messung der mütterlichen Kontamination in einer Nabelschnurblutprobe, umfassend:

Isolierung der genomischen DNA aus den Zellen in der Nabelschnurblutprobe,
Bestimmung des Gehalts an genomischer DNA in der Probe aus fötalen und mütterlichen Zellen auf der Grundlage von Kopienzahlvariations- (CNV-) Polymorphismen, die informative Marker der fötalen und mütterlichen Zellen sind,
Bestimmung des Gesamtgehalts an genomischer DNA, die aus den fötalen und mütterlichen Zellen in der Probe isoliert wurde,
wobei die informativen Marker umfassen:

- einen CND-Polymorphismus, der in isolierter genomischer DNA aus fötalen Zellen homozygot deletiert und in isolierter genomischer DNA aus mütterlichen Zellen heterozygot oder homozygot nicht deletiert ist; und
- einen CND-Polymorphismus, der in der isolierten genomischen DNA aus mütterlichen Zellen homozygot deletiert und in der isolierten genomischen DNA aus fötalen Zellen heterozygot oder homozygot nicht deletiert ist;

wobei der Gehalt an genomischer DNA in der Nabelschnurblutprobe aus den fötalen und mütterlichen Zellen und der Gesamtgehalt an genomischer DNA, die aus den fötalen und mütterlichen Zellen isoliert wurde, das Maß für den Chimärismus in der Nabelschnurblutprobe bereitstellt, und ein Verhältnis von fötalen Zellen:mütterlichen Zellen von mehr als etwa 1:100, etwa 1:50, etwa 1:20, etwa 1:10, etwa 3:20 anzeigt, dass die Nabelschnurblutprobe nicht für die Verwendung in einer hämatopoetischen Stammzelltransplantation geeignet ist.

## Revendications

**1.** Procédé de mesure de chimérisme dans un échantillon biologique obtenu à partir d'un sujet comprenant deux populations cellulaires génétiquement distinctes ou plus, le procédé comprenant :

l'isolement de l'ADN génomique provenant des cellules dans l'échantillon,
la détermination du taux d'ADN génomique dans l'échantillon provenant d'une première population cellulaire génétiquement distincte sur la base d'un polymorphisme de variation de nombre de copies (CNV) qui est un marqueur informatif de la première population cellulaire génétiquement distincte,
la détermination du taux total d'ADN génomique isolé à partir des populations cellulaires génétiquement distinctes dans l'échantillon biologique,
dans lequel le taux d'ADN génomique dans l'échantillon provenant de ladite première population cellulaire génétiquement distincte et le taux total d'ADN génomique isolé à partir des populations cellulaires génétiquement distinctes fournissent la mesure de chimérisme dans l'échantillon.

**2.** Procédé selon la revendication 1, comprenant en outre la détermination du taux d'ADN génomique dans l'échantillon provenant d'une deuxième population cellulaire génétiquement distincte sur la base d'un polymorphisme de CNV qui est un marqueur informatif de la deuxième population cellulaire génétiquement distincte, dans lequel les taux d'ADN génomique provenant desdites première et deuxième populations cellulaires génétiquement distinctes et le taux total d'ADN génomique isolé à partir des populations cellulaires génétiquement distinctes fournissent la mesure de chimérisme dans l'échantillon.

**3.** Procédé selon les revendications 1 ou 2, dans lequel un marqueur informatif d'une population cellulaire génétiquement distincte comprend :

- un polymorphisme de CNV qui est homozygote avec délétion dans l'ADN génomique isolé provenant de la première population cellulaire et homozygote sans délétion dans l'ADN génomique isolé provenant d'une deuxième population cellulaire; ou,
- un polymorphisme de CNV qui est homozygote avec délétion dans l'ADN génomique isolé provenant d'une deuxième population cellulaire et homozygote sans délétion dans l'ADN génomique isolé provenant de la première population cellulaire.

**4.** Procédé selon l'une quelconque des revendications 1 à 3, comprenant en outre la validation du taux d'ADN isolé provenant d'une population cellulaire génétiquement distincte en déterminant l'ADN génomique isolé total dans l'échantillon,
dans lequel des taux d'ADN génomique isolé provenant des populations cellulaires génétiquement distinctes égaux au taux d'ADN total indiquent que les taux d'ADN génomique isolé sont validés.

**5.** Procédé selon la revendication 4, dans lequel la mesure de chimérisme est exprimée sous la forme :

- d'un pourcentage de cellules génétiquement distinctes dans la population cellulaire totale dans l'échantillon ; ou
- d'un rapport de cellules génétiquement distinctes dans l'échantillon (première population cellulaire : deuxième population cellulaire).

**6.** Procédé selon l'une quelconque des revendications 1 à 5, dans lequel le polymorphisme de CNV est un polymorphisme de délétion de nombre de copies (CND).

**7.** Procédé selon la revendication 6, dans lequel un marqueur informatif d'une population cellulaire génétiquement distincte comprend :

(i)

- un polymorphisme de CND qui est homozygote avec délétion dans l'ADN génomique isolé provenant de la première population cellulaire et hétérozygote ou homozygote sans délétion dans l'ADN génomique isolé provenant d'une deuxième population cellulaire ; et/ou,
- un polymorphisme de CND qui est homozygote avec délétion dans l'ADN génomique isolé provenant d'une deuxième population cellulaire et hétérozygote ou homozygote sans délétion dans l'ADN génomique isolé provenant de la première population cellulaire ;

(ii)

- au moins deux polymorphismes de CND qui sont homozygotes avec délétion dans l'ADN génomique isolé provenant de la première population cellulaire et hétérozygotes ou homozygotes sans délétion dans l'ADN génomique isolé provenant d'une deuxième population cellulaire ; et/ou,
- au moins deux polymorphismes de CND qui sont homozygotes avec délétion dans l'ADN génomique isolé provenant de la deuxième population cellulaire et hétérozygotes ou homozygotes sans délétion dans l'ADN génomique isolé provenant de la première population cellulaire ;

(iii)

- au moins trois polymorphismes de CND qui sont homozygotes avec délétion dans l'ADN génomique isolé provenant de la première population cellulaire et hétérozygotes ou homozygotes sans délétion dans l'ADN génomique isolé provenant d'une deuxième population cellulaire ; et/ou,
- au moins trois polymorphismes de CND qui sont homozygotes avec délétion dans l'ADN génomique isolé provenant de la deuxième population cellulaire et hétérozygotes ou homozygotes sans délétion dans l'ADN génomique isolé provenant de la première population cellulaire ;

(iv)

- au moins 4, au moins 5, ou au moins 6, ou au moins 7, ou au moins 10, ou au moins 20, ou au moins 30, ou au moins 38 polymorphismes de CND qui sont homozygotes avec délétion dans l'ADN génomique isolé provenant de la première population cellulaire et hétérozygotes ou homozygotes sans délétion dans l'ADN génomique isolé provenant d'une deuxième population cellulaire ; ou,

- au moins 4, au moins 5, ou au moins 6, ou au moins 7, ou au moins 10, ou au moins 20, ou au moins 30, ou au moins 38 polymorphismes de CND qui sont homozygotes avec délétion dans l'ADN génomique isolé provenant de la deuxième population cellulaire et hétérozygotes ou homozygotes sans délétion dans l'ADN génomique isolé provenant de la première population cellulaire.

**8.** Procédé selon l'une quelconque des revendications 1 à 7, dans lequel la détermination du taux d'ADN génomique isolé provenant des populations cellulaires génétiquement distinctes comprend la soumission de l'ADN à une réaction d'amplification avec des amorces qui ciblent les polymorphismes de CNV qui sont des marqueurs informatifs des populations cellulaires génétiquement distinctes, dans lequel les amorces ciblent de préférence une région interne de la ou des CNV informative (s) et les polymorphismes de CNV sont facultativement amplifiés en utilisant des amorces sélectionnées dans le Tableau 7.

**9.** Procédé selon l'une quelconque des revendications 1 à 8, dans lequel les polymorphismes de CNV sont sélectionnés parmi les polymorphismes de CND indiqués dans le Tableau 1.

**10.** Procédé selon l'une quelconque des revendications 1 à 9, dans lequel l'échantillon biologique :

- a été traité pour éliminer sensiblement l'ADN acellulaire circulant de l'échantillon ;
- est un échantillon de sang ;
- est des cellules mononucléaires du sang périphérique ;
- est une population purifiée de cellules immunitaires ou un mélange purifié de cellules immunitaires ;
- est une population purifiée de globules blancs ;
- est une population purifiée de lymphocytes T, de lymphocytes B, de granulocytes ou un mélange de ceux-ci ;
- est purifié pour fournir au moins deux populations cellulaires et le chimérisme est mesuré dans chaque population cellulaire ;
- est purifié pour fournir une population de lymphocytes B et une population de granulocytes et le chimérisme est mesuré dans chaque population cellulaire ;
- est purifié pour fournir au moins trois populations cellulaires et le chimérisme est mesuré dans chaque population cellulaire ;
- est purifié pour fournir une population de lymphocytes B, une population de granulocytes et une population de lymphocytes T et le chimérisme est mesuré dans chaque population cellulaire ; ou
- est du sang de cordon ombilical.

**11.** Procédé selon l'une quelconque des revendications 1 à 10, dans lequel les populations cellulaires génétiquement distinctes comprennent des cellules du soi (première population cellulaire) et des cellules du non-soi (deuxième population cellulaire), dans lequel facultativement les cellules du soi (première population cellulaire) sont des cellules fœtales et les cellules du non-soi (deuxième population cellulaire) sont des cellules maternelles.

**12.** Procédé selon l'une quelconque des revendications 1 à 11, dans lequel le sujet est un receveur de transplantation de cellules souches hématopoïétiques (HSCT).

**13.** Procédé selon l'une quelconque des revendications 1 à 12, dans lequel

- l'échantillon biologique a été obtenu à partir d'un receveur de HSCT et comprend une population de cellules du soi et une population de cellules du non-soi, et le taux de cellules du non-soi détermine le niveau de prise de greffe d'un receveur de HSCT, de préférence, dans lequel un taux de cellules du non-soi supérieur à environ 90 % indique une prise de la transplantation ;
- l'échantillon biologique a été obtenu à partir d'un receveur de HSCT et comprend une population de cellules du soi et une population de cellules du non-soi, et le taux de cellules du soi surveille le rétablissement de la propre moelle osseuse posttransplantation de HSCT d'un receveur de transplantation de cellules souches hématopoïétiques (HSCT), de préférence, dans lequel :

(a) un taux de cellules du soi supérieur à environ 10 % indique le rétablissement de la moelle osseuse du receveur de HSCT ;

(b) une augmentation du taux de cellules du soi et/ou une diminution du taux de cellules du non-soi indiquent le rétablissement de la moelle osseuse du receveur de HSCT; ou,
(c) une augmentation du taux de cellules du soi et/ou une diminution du taux de cellules du non-soi indiquent qu'un traitement immunosuppresseur doit être administré au receveur de HSCT ou que le traitement immunosuppresseur du receveur de HSCT doit être modifié ;

- l'échantillon biologique a été obtenu à partir d'un receveur de HSCT et comprend une population de cellules du soi et une population de cellules du non-soi, et une augmentation du taux de cellules du soi ou une diminution du taux de cellules du non-soi surveille une maladie résiduelle minimale chez un patient en ayant besoin, de préférence, dans lequel un taux de cellules du soi supérieur à environ 1 % indique une rechute du trouble hématologique du receveur de HSCT ; ou,
- l'échantillon biologique est un échantillon de sang de cordon ombilical et comprend une population de cellules fœtales et une population de cellules maternelles, et le niveau de chimérisme dans l'échantillon de sang de cordon ombilical détermine le niveau de contamination maternelle d'un échantillon de sang de cordon ombilical, de préférence, dans lequel un rapport de chimérisme (cellules fœtales : cellules maternelles) supérieur à environ 1:100, environ 1 : 50, environ 1 : 20, environ 1:10, environ 3 : 20 indique que l'échantillon de sang de cordon ombilical n'est pas approprié pour une utilisation dans une transplantation de cellules souches hématopoïétiques.

**14.** Procédé selon la revendications 13, dans lequel le receveur de HSCT a reçu une HSCT pour le traitement d'un trouble hématologique, de préférence une malignité hématologique, plus préférentiellement une leucémie et facultativement, dans lequel :

- un taux de cellules du soi supérieur à environ 1 % indique une rechute du trouble hématologique du receveur de HSCT ;
- une augmentation du taux de cellules du soi et/ou une diminution du taux de cellules du non-soi indiquent une rechute du trouble hématologique du receveur de HSCT; ou,
- une augmentation du taux de cellules du soi et/ou une diminution du taux de cellules du non-soi indique indiquent qu'un traitement pour le trouble hématologique doit être administré au receveur de HSCT ou le traitement du receveur de HSCT doit être modifié.

**15.** Procédé de mesure d'une prise de greffe de HSCT dans un échantillon biologique obtenu à partir d'un receveur de transplantation de HSCT, le procédé comprenant :

l'isolement de l'ADN génomique provenant des cellules dans l'échantillon,
la détermination du taux d'ADN génomique dans l'échantillon provenant des cellules du soi et du non-soi sur la base de polymorphismes de variation de nombre de copies (CNV) qui sont des marqueurs informatifs des cellules du soi et du non-soi,
la détermination du taux total d'ADN génomique isolé à partir des cellules du soi et du non-soi dans l'échantillon, dans lequel les marqueurs informatifs comprennent :

- un polymorphisme de CND qui est homozygote avec délétion dans l'ADN génomique isolé provenant des cellules du soi et hétérozygote ou homozygote sans délétion dans l'ADN génomique isolé provenant des cellules du non-soi ; et
- un polymorphisme de CND qui est homozygote avec délétion dans l'ADN génomique isolé provenant des cellules du non-soi et hétérozygote ou homozygote sans délétion dans l'ADN génomique isolé provenant des cellules du soi ;

dans lequel le taux d'ADN génomique dans l'échantillon provenant des cellules du soi et du non-soi et le taux total d'ADN génomique isolé à partir des cellules du soi et du non-soi fournissent la mesure de chimérisme dans l'échantillon, et un taux d'ADN génomique dans l'échantillon provenant des cellules du non-soi supérieur à environ 90 % indique une prise de greffe de la moelle osseuse du receveur de HSCT.

**16.** Procédé de mesure d'une maladie résiduelle minimale dans un échantillon biologique obtenu à partir d'un receveur de HSCT, le procédé comprenant :

l'isolement de l'ADN génomique provenant des cellules dans l'échantillon,
la détermination du taux d'ADN génomique dans l'échantillon provenant des cellules du soi et du non-soi sur la base de polymorphismes de variation de nombre de copies (CNV) qui sont des marqueurs informatifs des cellules du soi et du non-soi,
la détermination du taux total d'ADN génomique isolé à partir des cellules du soi et du non-soi dans l'échantillon,
dans lequel les marqueurs informatifs comprennent :

- un polymorphisme de CND qui est homozygote avec délétion dans l'ADN génomique isolé provenant des cellules du soi et hétérozygote ou homozygote sans délétion dans l'ADN génomique isolé provenant des cellules du non-soi ; et
- un polymorphisme de CND qui est homozygote avec délétion dans l'ADN génomique isolé provenant des cellules du non-soi et hétérozygote ou homozygote sans délétion dans l'ADN génomique isolé provenant des cellules du soi ;

dans lequel le taux d'ADN génomique dans l'échantillon provenant des cellules du soi et du non-soi et le taux total d'ADN génomique isolé à partir des cellules du soi et du non-soi fournissent la mesure de chimérisme dans l'échantillon, et un taux de cellules du soi supérieur à environ 1 % indique le rétablissement de la moelle osseuse du receveur de HSCT.

**17.** Procédé de mesure de contamination maternelle dans un échantillon de sang de cordon ombilical comprenant :

l'isolement de l'ADN génomique provenant des cellules dans l'échantillon de sang de cordon ombilical,
la détermination du taux d'ADN génomique dans l'échantillon provenant des cellules fœtales et maternelles sur la base de polymorphismes de variation de nombre de copies (CNV) qui sont des marqueurs informatifs des cellules fœtales et maternelles,
la détermination du taux total d'ADN génomique isolé à partir des cellules fœtales et maternelles dans l'échantillon,
dans lequel les marqueurs informatifs comprennent :

- un polymorphisme de CND qui est homozygote avec délétion dans l'ADN génomique isolé provenant des cellules fœtales et hétérozygote ou homozygote sans délétion dans l'ADN génomique isolé provenant des cellules maternelles ; et
- un polymorphisme de CND qui est homozygote avec délétion dans l'ADN génomique isolé provenant des cellules maternelles et hétérozygote ou homozygote sans délétion dans l'ADN génomique isolé provenant des cellules fœtales ;

dans lequel le taux d'ADN génomique dans l'échantillon de sang de cordon ombilical provenant des cellules fœtales et maternelles et le taux total d'ADN génomique isolé à partir des cellules fœtales et maternelles fournissent la mesure de chimérisme dans l'échantillon de sang de cordon ombilical, et un rapport cellules fœtales : cellules maternelles supérieur à environ 1 : 100, environ 1: 50, environ 1 : 20, environ 1:10, environ 3 : 20 indique que l'échantillon de sang de cordon ombilical n'est pas approprié pour une utilisation dans une transplantation de cellules souches hématopoïétiques.

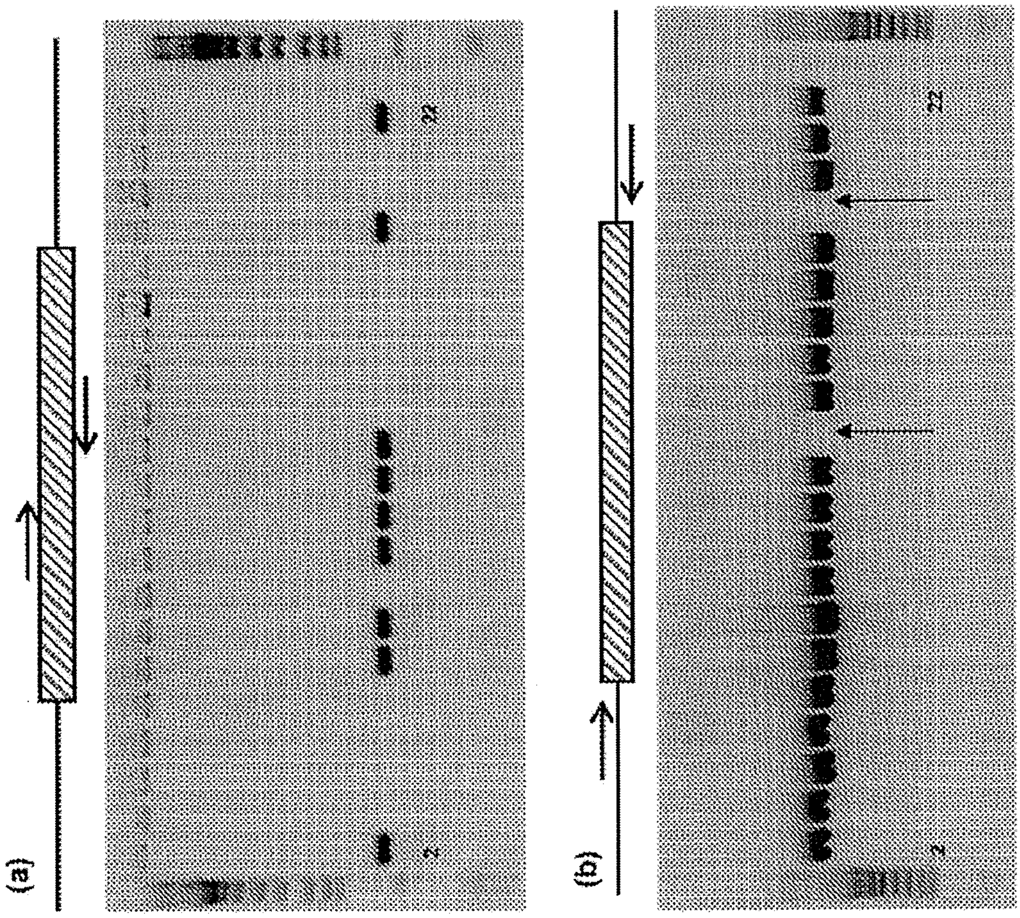


Figure 1

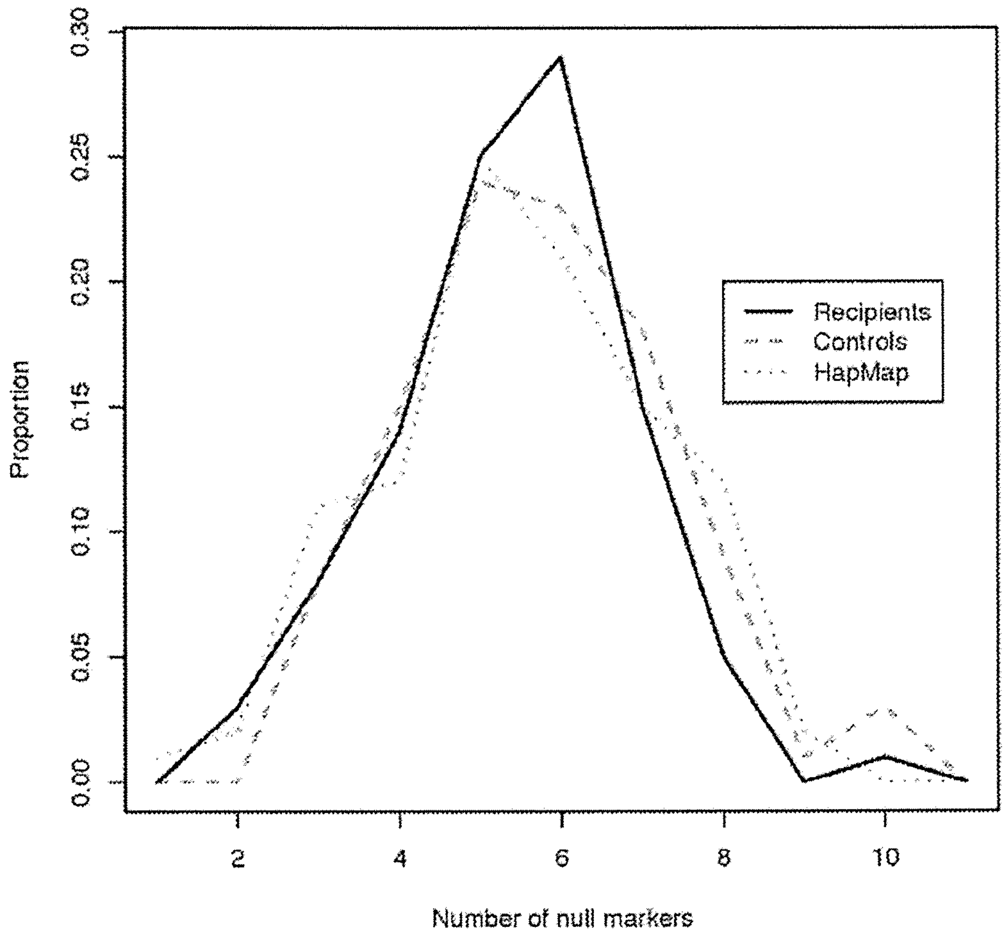
0.30
0.25
0.20
0.15
0.10
0.05
0.00
Proportion
Recipients
Controls
HapMap
2
4
6
8
10
Number of null markers

Figure 2

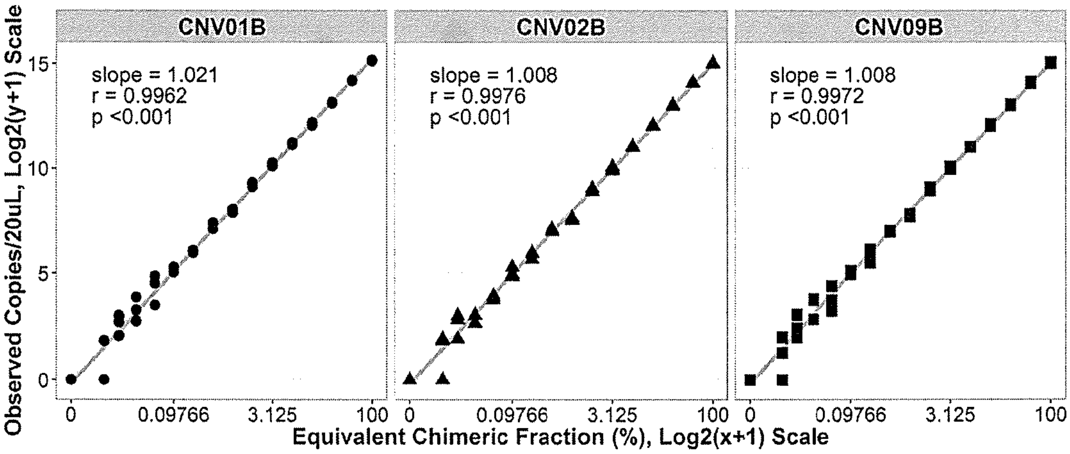
CNV01B
CNV02B
CNV09B
slope = 1.021
r = 0.9962
p <0.001
slope = 1.008
r = 0.9976
p <0.001
slope = 1.008
r = 0.9972
p <0.001
Observed Copies/20uL, Log2(y+1) Scale
15
10
5
0
0
0.09766
3.125
100
Equivalent Chimeric Fraction (%), Log2(x+1) Scale

Figure 3

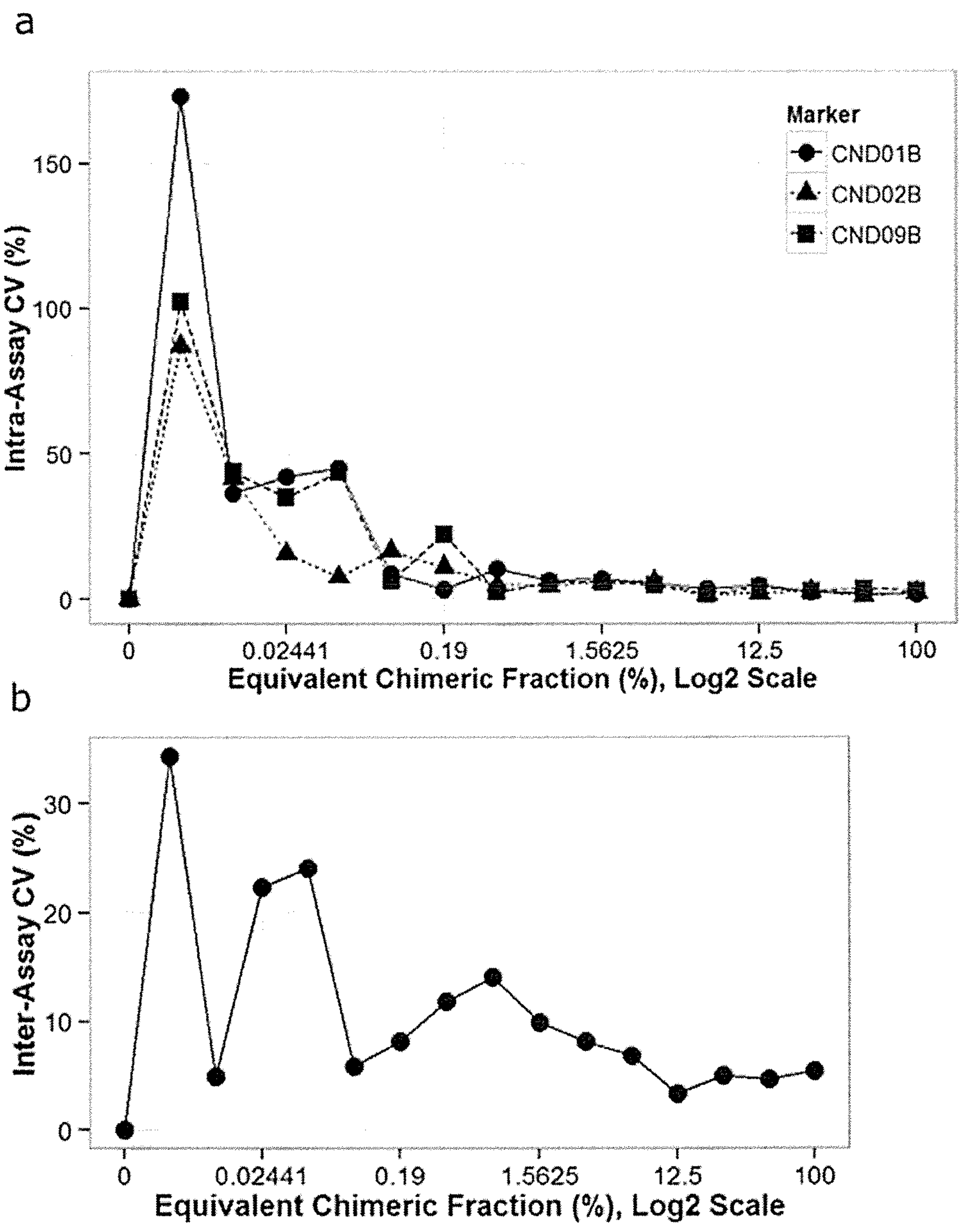
a
Marker
CND01B
CND02B
CND09B
Intra-Assay CV (%)
150
100
50
0
0
0.02441
0.19
1.5625
12.5
100
Equivalent Chimeric Fraction (%), Log2 Scale
b
Inter-Assay CV (%)
30
20
10
0
0
0.02441
0.19
1.5625
12.5
100
Equivalent Chimeric Fraction (%), Log2 Scale

Figure 4

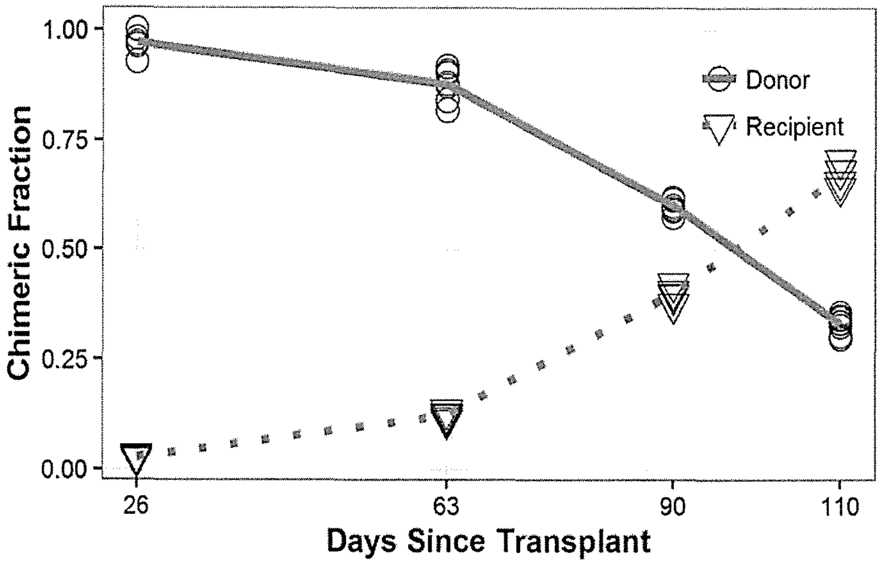


Inter-Assay Variation (standard deviation, CV)

| | | | | |
|---|---|---|---|---|
| **Donor** | 0.02, 6% | 0.03, 2% | 0.02, 4% | 0.02, 3% |
| **Recipient** | 0.003, 4% | 0.01, 11% | 0.02, 8% | 0.03, 4% |

**Figure 5**

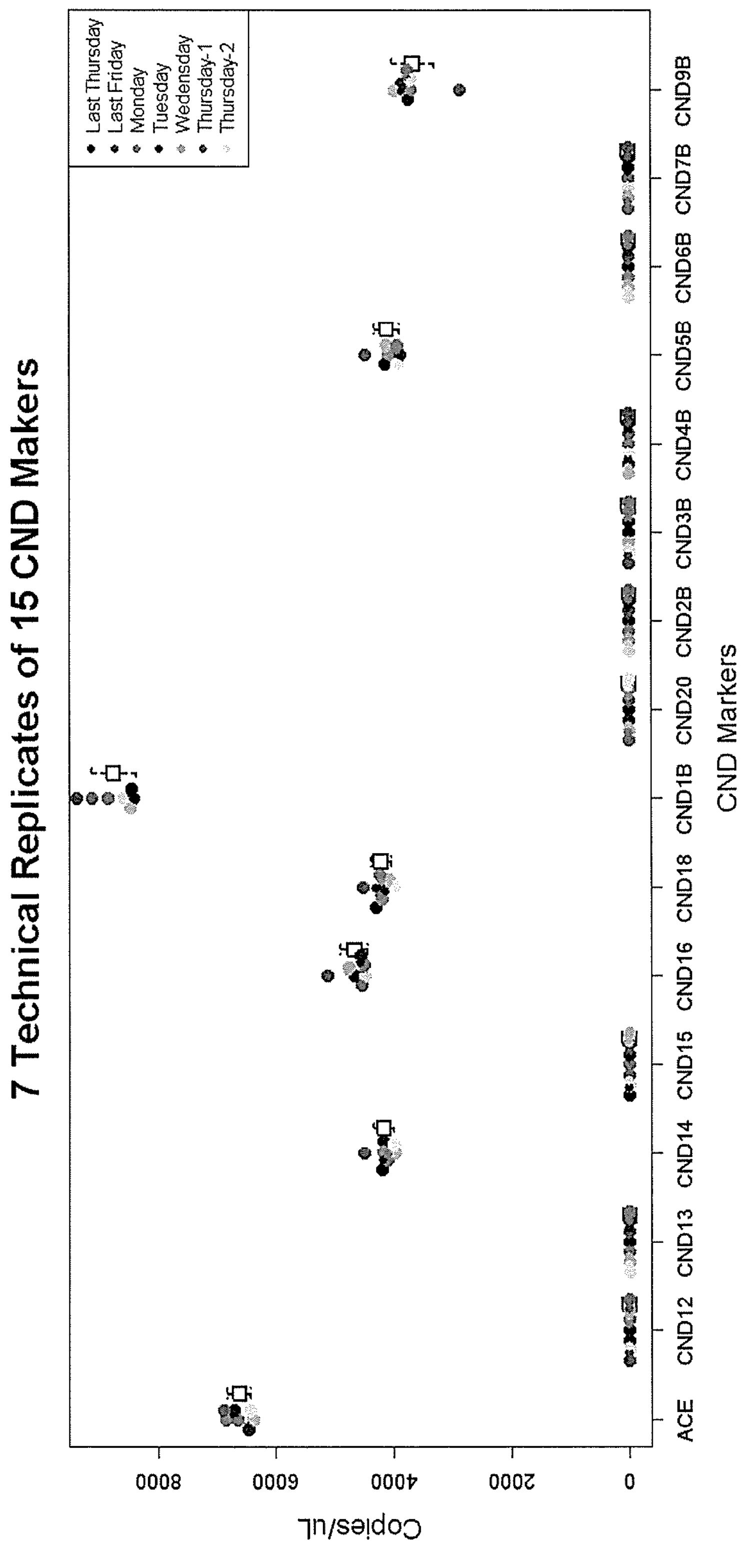
7 Technical Replicates of 15 CND Makers
Last Thursday
Last Friday
Monday
Tuesday
Wedensday
Thursday-1
Thursday-2
Copies/uL
0
2000
4000
6000
8000
ACE
CND12
CND13
CND14
CND15
CND16
CND18
CND1B
CND20
CND2B
CND3B
CND4B
CND5B
CND6B
CND7B
CND9B
CND Markers

Figure 6

## Patient 1 Results

| | Informative markers | | | | | | |
|---|---|---|---|---|---|---|---|
| | | Recipient informative | | | | Donor informative | |
| | ACE | CND12 | CND1B | CND20 | CND4B* | CND2B | CND5B |
| P1_DONOR | 4250 | 0 | 0 | 0 | 0 | 2090 | 2200 |
| P1_RECIPIENT | 3850 | 1910 | 1800 | 2000 | 1180 | 0 | 0 |
| P1_TEST_A | 4420 | 1230 | 1270 | 1250 | 770 | 930 | 950 |
| P1 Test_B | 4230 | 1770 | 1660 | 1690 | 890 | 406 | 457 |
| P1_TEST_C | 4430 | 1900 | 1930 | 1730 | 1080 | 328 | 359 |

*bad performace marker

| | Ave recipient | RSD | Ave Donor | RSD | total |
|---|---|---|---|---|---|
| P1_TEST_A | 1250 | 20 | 940 | 14 | 2190 |
| P1_TEST_B | 1707 | 57 | 432 | 36 | 2138 |
| P1_TEST_C | 1853 | 108 | 344 | 22 | 2197 |

| | Chimerism results | | | |
|---|---|---|---|---|
| | Recipient% | %SD | Donor% | %SD |
| **P1_TEST_A** | 57.1% | 0.9% | 42.9% | 0.6% |
| **P1_TEST_B** | 79.8% | 2.7% | 20.2% | 1.7% |
| **P1_TEST_C** | 84.4% | 4.9% | 15.6% | 1.0% |

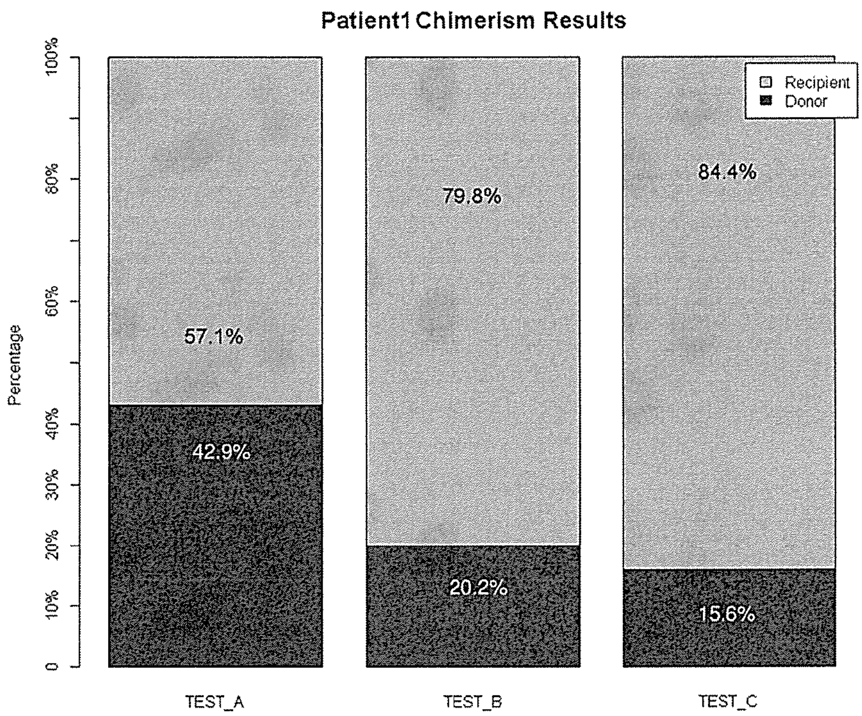


Figure 7

## Patient 2 Results

| | | Informative markers | | | | | | |
|---|---|---|---|---|---|---|---|---|
| | | Recipient informative | | | Donor informative | | | |
| | ACE | CND20 | CND7B | CND9B | CND12 | CND13 | CND3B | CND6B |
| P2_DONOR | 4270 | 0 | 0 | 0 | 2090 | 2170 | 1990 | 1940 |
| P2_RECIPIENT | 4620 | 2390 | 2070 | 2060 | 0 | 0 | 0 | 0 |
| P2_TEST_A | 4690 | 1230 | 1050 | 1120 | 1080 | 1070 | 749 | 1090 |
| P2_TEST_B | 4440 | 3 | 3 | 3 | 2280 | 2220 | 2220 | 2170 |
| P2_TEST_C | 4260 | 11 | 10 | 5 | 2090 | 2000 | 1960 | 2060 |
| P2_TEST_D | 4460 | 9 | 6 | 10 | 2300 | 2360 | 2220 | 2220 |

| | Ave recipient | RSD | Ave Donor | RSD | total |
|---|---|---|---|---|---|
| P2_TEST_A | 1133 | 91 | 1080 | 14 | 2213 |
| P2_TEST_B | 3 | 0 | 2195 | 45 | 2198 |
| P2_TEST_C | 9 | 3 | 2030 | 59 | 2039 |
| P2_TEST_D | 8 | 2 | 2290 | 68 | 2298 |

| | Chimerism results | | | |
|---|---|---|---|---|
| | Recipient% | %SD | Donor% | %SD |
| P2_TEST_A | 51.20% | 4.10% | 48.80% | 0.45% |
| P2_TEST_B | 0.13% | 0.01% | 99.87% | 2.05% |
| P2_TEST_C | 0.42% | 0.17% | 99.58% | 2.87% |
| P2_TEST_D | 0.36% | 0.09% | 99.64% | 2.96% |

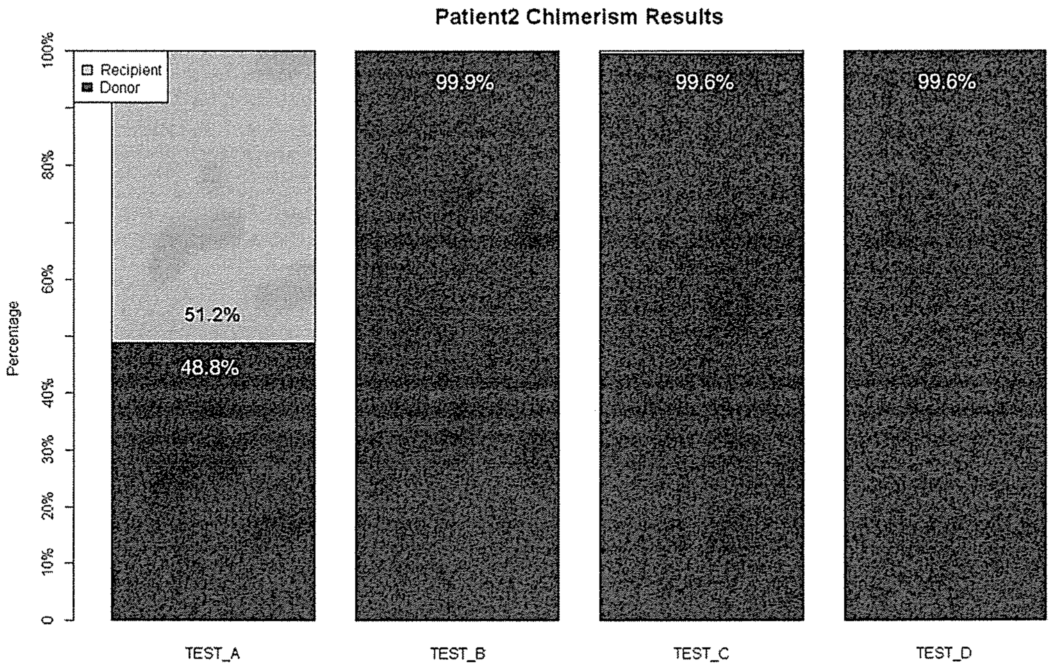


Figure 8

## Patient 3 Results

| | | Informative markers | | |
|---|---|---|---|---|
| | | Recipient marker | Donor Marker | |
| | ACE | CND12 | CND2B | CND3B |
| P3 DONOR | 4400 | 0 | 1640 | 2190 |
| P3 RECIPIENT | 3170 | 1570 | 0 | 0 |
| P3_TestA | 4420 | 1030 | 990 | 1050 |
| P3_TestB | 4660 | 180 | 2180 | 2050 |
| P3_TestC | 4030 | 395 | 1590 | 1710 |
| P3_TestD | 4050 | 466 | 1530 | 1540 |
| P3_TestE | 3950 | 596 | 1390 | 1420 |

| | Ave recipient | Ave Donor | RSD | total |
|---|---|---|---|---|
| P3_TestB | 180 | 2115 | 91.92388 | 2295 |
| P3_TestC | 395 | 1650 | 84.85281 | 2045 |
| P3_TestD | 466 | 1535 | 7.071068 | 2001 |
| P3_TestE | 596 | 1405 | 21.2132 | 2001 |

| | Chimerism results | | |
|---|---|---|---|
| | Recipient% | Donor% | %SD |
| | Recipient% | Donor% | %SD |
| P3_TestA | 50.2% | 49.8% | 2.1% |
| P3_TestB | 7.8% | 92.2% | 4.0% |
| P3_TestC | 19.3% | 80.7% | 4.1% |
| P3_TestD | 23.3% | 76.7% | 0.4% |
| P3_TestE | 29.8% | 70.2% | 1.1% |

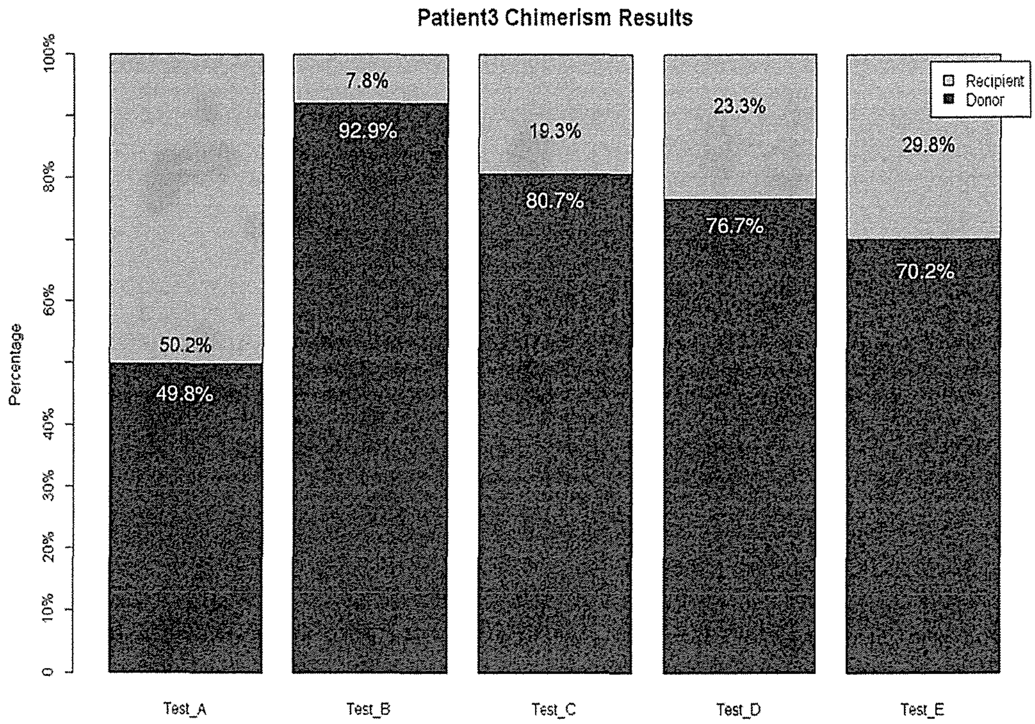


**Figure 9**

## Patient 4 Results

| | | Informtive markers | | | | | | | |
|---|---|---|---|---|---|---|---|---|---|
| | | Recipient informative markers | | | | | Donor informative markers | | |
| | ACE | CND5B | CND6B | CND16 | CND7B | CND14 | CND15 | CND38 | CND4B* |
| P4 DONOR | 6390 | 0 | 0 | 0 | 0 | 0 | 3080 | 3140 | 2400 |
| P4 RECIPIENT | 4630 | 2260 | 2290 | 2270 | 4370 | 4290 | 8 | 13 | 6 |
| P4 recipient_2 | | | | | | | 10 | 17 | 11 |
| P4_TestA | 3390 | 254 | 276 | 286 | 520 | 544 | 1380 | 1320 | 1070 |
| P4_TestB | 4160 | 465 | 489 | 518 | 950 | 1010 | 1410 | 1430 | 1110 |
| P4_TestC | 4520 | 103 | 85 | 114 | 207 | 226 | 2000 | 2110 | 1570 |

*bad performace marker

| | Ave recipient | RSD | Ave Donor | RSD | total |
|---|---|---|---|---|---|
| P4_TEST_A | 272 | 16 | 1350 | 42 | 1622 |
| P4_TEST_B | 491 | 27 | 1420 | 14 | 1911 |
| P4_TEST_C | 101 | 15 | 2055 | 78 | 2156 |

| | Chimerism results | | | |
|---|---|---|---|---|
| | Recipient% | %SD | Donor% | %SD |
| P4_TEST_A | 16.8% | 1.0% | 83.2% | 2.6% |
| P4_TEST_B | 25.7% | 1.4% | 74.3% | 0.7% |
| P4_TEST_C | 4.7% | 0.7% | 95.3% | 3.6% |

**Patient4 Chimerism Results**

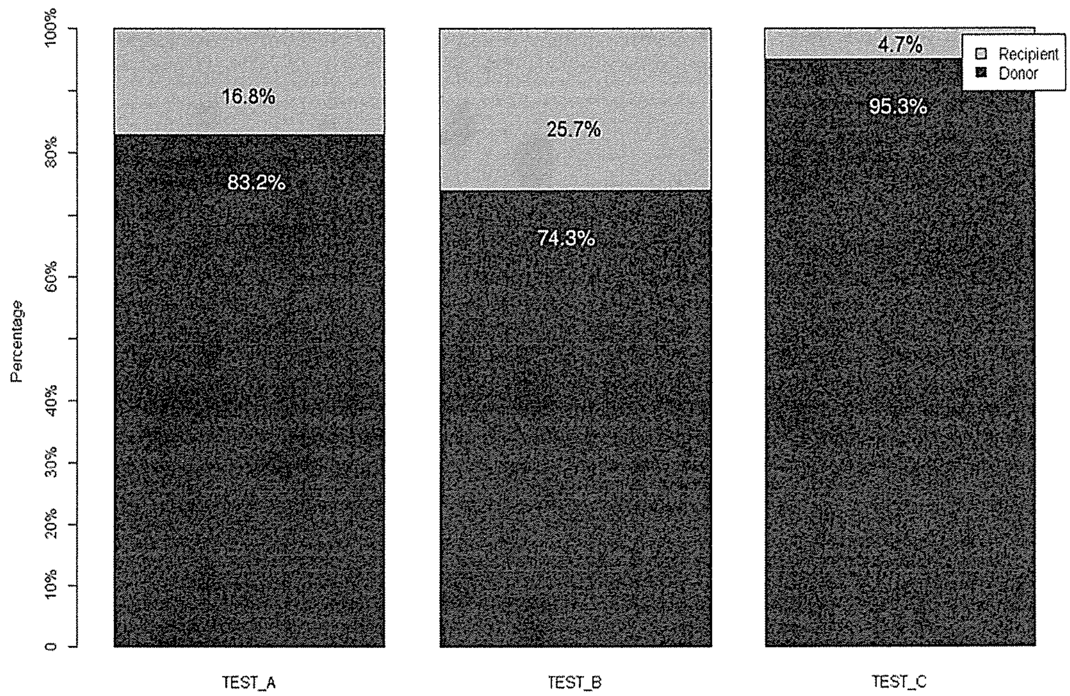


**Figure 10**

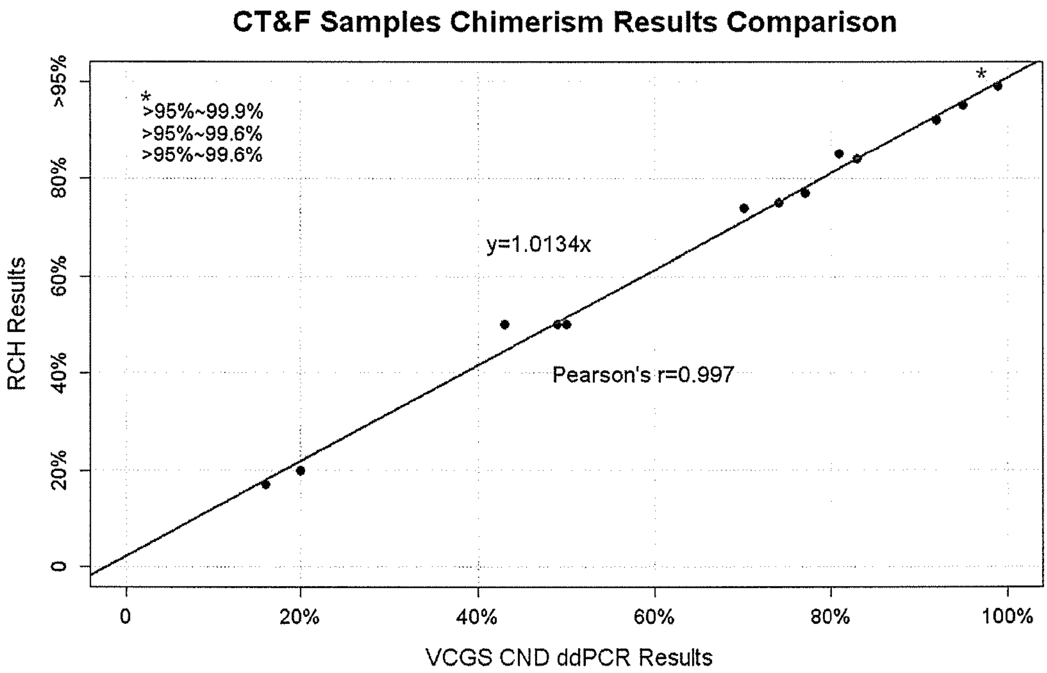
CT&F Samples Chimerism Results Comparison
*
>95%~99.9%
>95%~99.6%
>95%~99.6%
*
y=1.0134x
Pearson's r=0.997
RCH Results
0
20%
40%
60%
80%
>95%
0
20%
40%
60%
80%
100%
VCGS CND ddPCR Results

**Figure 11**

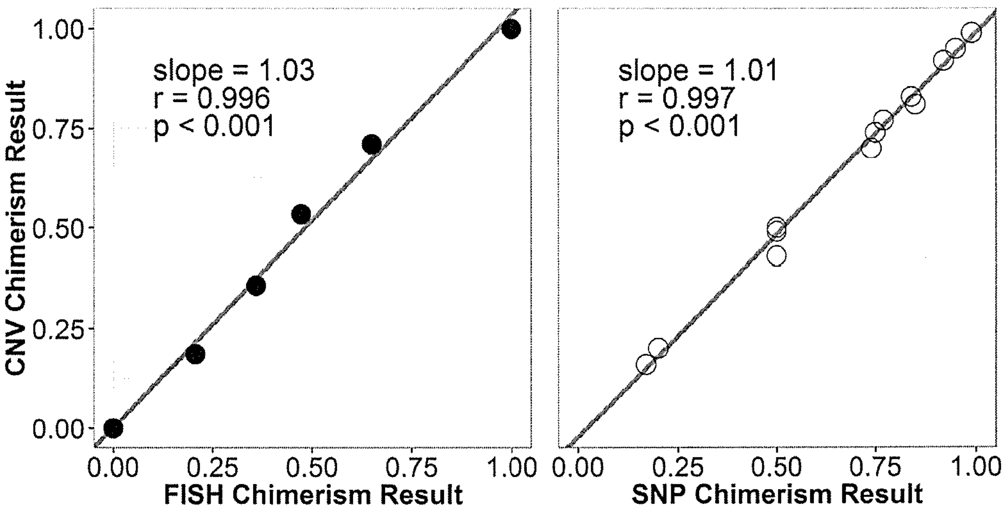
slope = 1.03
r = 0.996
p < 0.001
CNV Chimerism Result
1.00
0.75
0.50
0.25
0.00
0.00 0.25 0.50 0.75 1.00
FISH Chimerism Result
slope = 1.01
r = 0.997
p < 0.001
0.00 0.25 0.50 0.75 1.00
SNP Chimerism Result

Figure 12

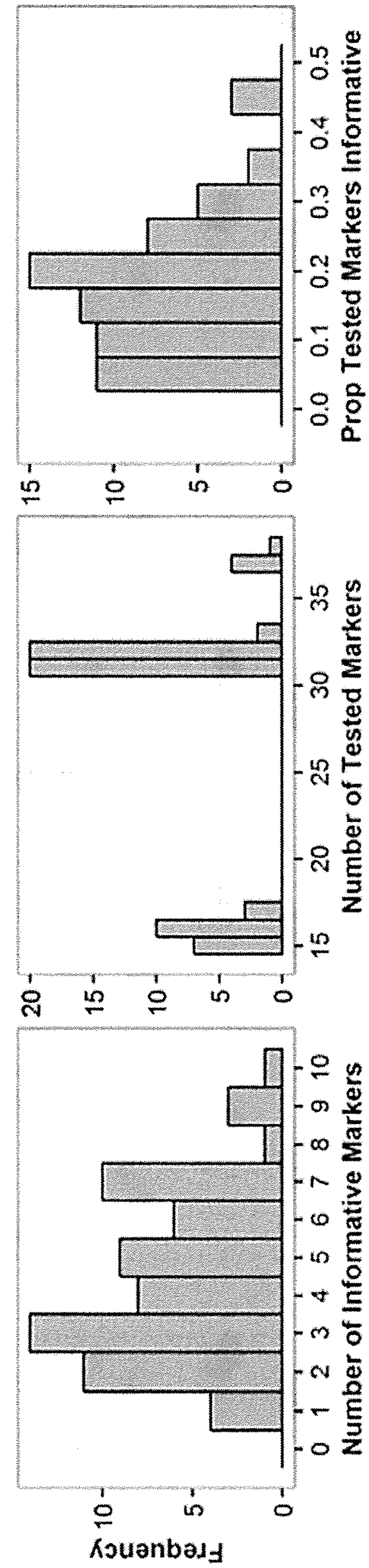
Frequency
Number of Informative Markers
Number of Tested Markers
Prop Tested Markers Informative

Figure 13

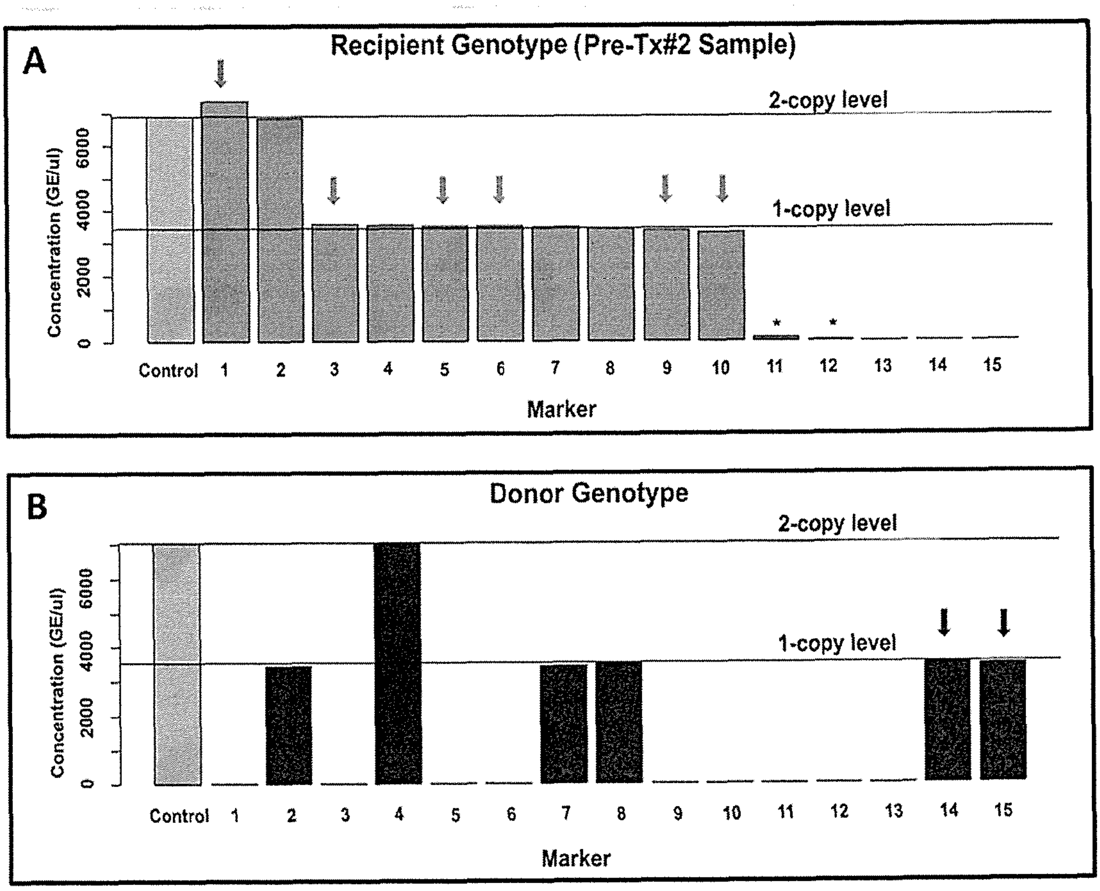
A
Recipient Genotype (Pre-Tx#2 Sample)
2-copy level
1-copy level
Concentration (GE/ul)
0
2000
4000
6000
Control 1 2 3 4 5 6 7 8 9 10 11 12 13 14 15
Marker
B
Donor Genotype
2-copy level
1-copy level
Concentration (GE/ul)
0
2000
4000
6000
Control 1 2 3 4 5 6 7 8 9 10 11 12 13 14 15
Marker

Figure 14

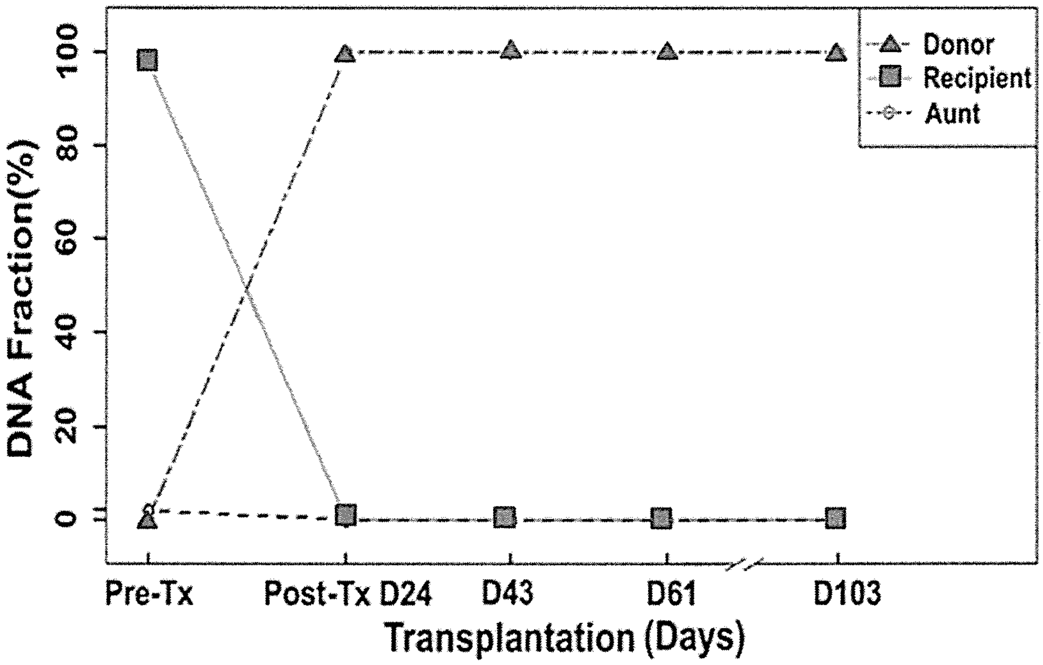
DNA Fraction(%)
0
20
40
60
80
100
Donor
Recipient
Aunt
Pre-Tx
Post-Tx D24
D43
D61
D103
Transplantation (Days)

Figure 15

| ID | Sample | ACE | CND12 | CND13 | CND14 | CND15 | CND16 | CND18 | CND1B* | CND20 | CND2B | CND3B* | CND4B | CND5B | CND6B | CND7B | CND9B |
|---|---|---|---|---|---|---|---|---|---|---|---|---|---|---|---|---|---|
| 15C400208 | Maternal blood | 6420 | 1 | 0 | 0 | 6930 | 0 | 0 | 3260 | 0 | 0 | 3440 | 0 | 0 | 0 | 7270 | 3320 |
| 15C400209 | Cord blood | 6170 | 3060 | 0 | 0 | 3140 | 0 | 0 | 0 | 0 | 0 | 0 | 0 | 0 | 0 | 6460 | 3100 |

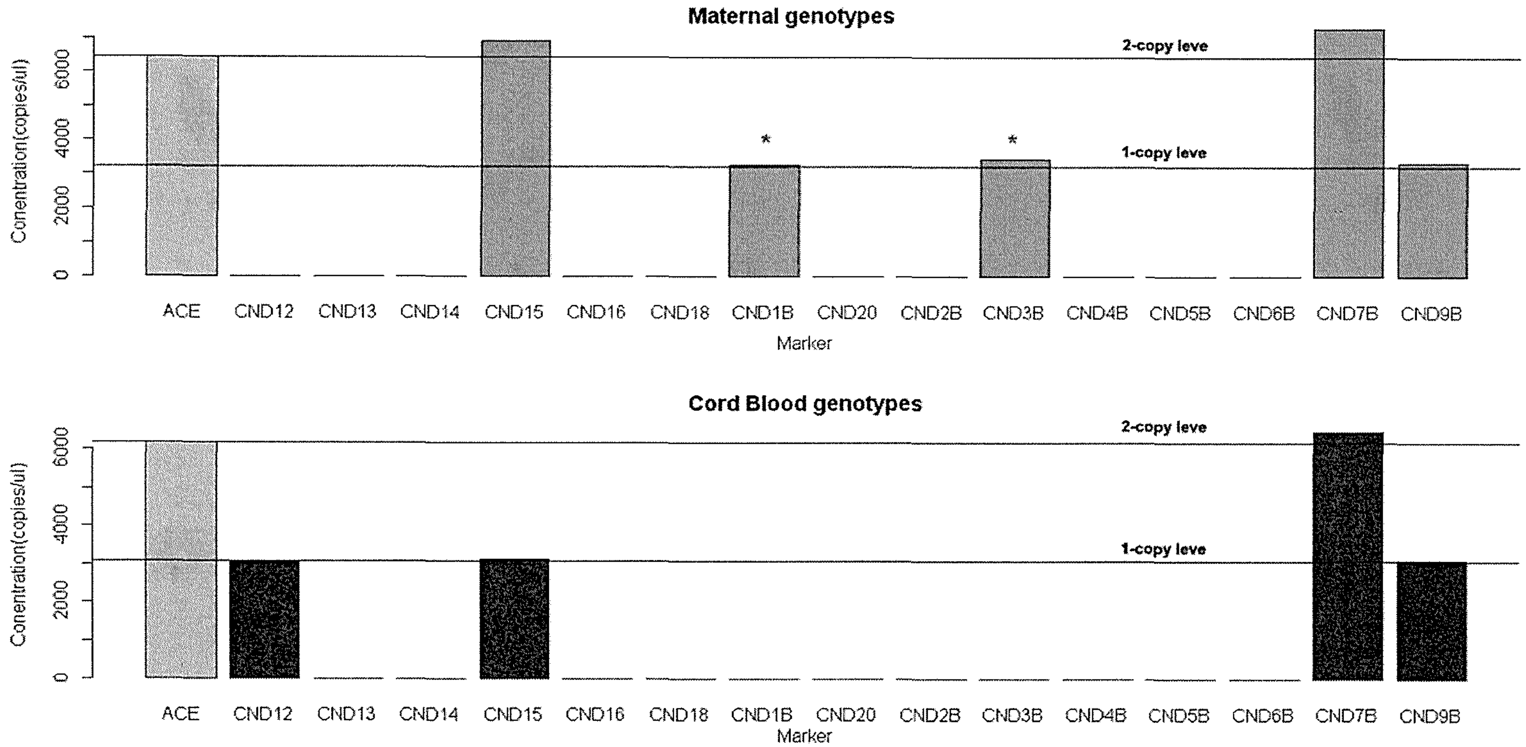


**Figure 16**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- AU 2015902274

### Non-patent literature cited in the description


- **THIEDE et al.** *Bone Marrow Transplant,* 1999, vol. 23, 1055-1060
- **NUCKOLS et al.** *Am J Clin Pathol,* 2000, vol. 113, 135-140
- **J. PERBAL.** A Practical Guide to Molecular Cloning. John Wiley and Sons, 1984
- **J. SAMBROOK et al.** Molecular Cloning: A Laboratory Manual. Cold Spring Harbour Laboratory Press, 1989
- Essential Molecular Biology: A Practical Approach. IRL Press, 1991, vol. 1, 2
- Current Protocols in Molecular Biology. Greene Pub. Associates and Wiley-Interscience, 1988
- Antibodies: A Laboratory Manual. Cold Spring Harbour Laboratory, 1988
- Current Protocols in Immunology. John Wiley & Sons
- **LO et al.** *Am J HumGenet.,* 1998, vol. 62, 768
- **CONRAD et al.** *Nature,* 2010, vol. 4 (54), 704-712
- **MCCARROL et al.** *Nature Genet,* 2008, vol. 40 (10), 1166-1174
- **STEVENS et al.** *EJHG,* 2012, vol. 20, 657-667
- **STEVENS et al.** *PLOS One,* 2012, vol. 7, 49575

SEQUENCE LISTING

<110> Murdoch Childrens Research Institute

<120> METHOD OF MEASURING CHIMERISM

<130> 522589

<160> 78

<170> PatentIn version 3.5

<210> 1
<211> 19
<212> DNA
<213> Artificial Sequence

<220>
<223> Artificial Sequence

<400> 1
agcctcaatt caggaccta 19

<210> 2
<211> 21
<212> DNA
<213> Artificial Sequence

<220>
<223> Artificial Sequence

<400> 2
ctccttggag agcttgttat c 21

<210> 3
<211> 23
<212> DNA
<213> Artificial Sequence

<220>
<223> Artificial Sequence

<400> 3
tgtcatgtgc aatagagact gtt 23

<210> 4
<211> 18
<212> DNA
<213> Artificial Sequence

<220>
<223> Artificial Sequence

<400> 4
ttctggccag ggcaattc 18

<210> 5
<211> 22
<212> DNA
<213> Artificial Sequence

<220>
<223> Artificial Sequence

<400> 5
actggcttcc aaatgtgact aa 22

<210> 6
<211> 18
<212> DNA
<213> Artificial Sequence

<220>
<223> Artificial Sequence

<400> 6
taacctgcag ctctggga 18

<210> 7
<211> 21
<212> DNA
<213> Artificial Sequence

<220>
<223> Artificial Sequence

<400> 7
actgacacac tcaagctatg g 21

<210> 8
<211> 22
<212> DNA
<213> Artificial Sequence

<220>
<223> Artificial Sequence

<400> 8
agcataaatc tctgctccct tt 22

<210> 9
<211> 24
<212> DNA
<213> Artificial Sequence

<220>
<223> Artificial Sequence

<400> 9
agcaaatgat gatgttgtat gagc 24

<210> 10
<211> 20
<212> DNA
<213> Artificial Sequence

<220>
<223> Artificial Sequence

<400> 10
taaatgccag caccctgcta 20

<210> 11
<211> 17
<212> DNA
<213> Artificial Sequence

<220>
<223> Artificial Sequence

<400> 11
gccatgcctc ccacatt 17

<210> 12
<211> 22
<212> DNA
<213> Artificial Sequence

<220>
<223> Artificial Sequence

<400> 12
tggccctaac agtttggata tg 22

<210> 13
<211> 21
<212> DNA
<213> Artificial Sequence

<220>
<223> Artificial Sequence

<400> 13
tcacaggtcc taccagcttt a 21

<210> 14
<211> 27
<212> DNA
<213> Artificial Sequence

<220>
<223> Artificial Sequence

<400> 14
gacgaaagat gtcttaattt ggttcag 27

<210> 15
<211> 22
<212> DNA

<213> Artificial Sequence

<220>
<223> Artificial Sequence

<400> 15
ccagcgtctt ctcttatgtc ag 22

<210> 16
<211> 20
<212> DNA
<213> Artificial Sequence

<220>
<223> Artificial Sequence

<400> 16
acaagccaac aacgtacagg 20

<210> 17
<211> 21
<212> DNA
<213> Artificial Sequence

<220>
<223> Artificial Sequence

<400> 17
tttggtgact gatacatggc t 21

<210> 18
<211> 25
<212> DNA
<213> Artificial Sequence

<220>
<223> Artificial Sequence

<400> 18
cctagctact attccctcta gaaca 25

<210> 19
<211> 22
<212> DNA
<213> Artificial Sequence

<220>
<223> Artificial Sequence

<400> 19
atatcctctg gatttgacag tg 22

<210> 20
<211> 20
<212> DNA
<213> Artificial Sequence

<220>

<223> Artificial Sequence

<400> 20
atgttgagag cattggactt 20

<210> 21
<211> 21
<212> DNA
<213> Artificial Sequence

<220>
<223> Artificial Sequence

<400> 21
ttccaaagac acaaggcagt a 21

<210> 22
<211> 21
<212> DNA
<213> Artificial Sequence

<220>
<223> Artificial Sequence

<400> 22
ggacattcct gatgggagaa a 21

<210> 23
<211> 24
<212> DNA
<213> Artificial Sequence

<220>
<223> Artificial Sequence

<400> 23
ccaagtatga tgttatttgt ggga 24

<210> 24
<211> 25
<212> DNA
<213> Artificial Sequence

<220>
<223> Artificial Sequence

<400> 24
cacaagtaga ttcagattag aaggg 25

<210> 25
<211> 25
<212> DNA
<213> Artificial Sequence

<220>
<223> Artificial Sequence

<400> 25

catattgaga tccagaaaca acctt 25

<210> 26
<211> 23
<212> DNA
<213> Artificial Sequence

<220>
<223> Artificial Sequence

<400> 26
agaaataaac tgaggcctgg ata 23

<210> 27
<211> 24
<212> DNA
<213> Artificial Sequence

<220>
<223> Primer

<400> 27
ccttactgac actctctgtt tctt 24

<210> 28
<211> 25
<212> DNA
<213> Artificial Sequence

<220>
<223> Primer

<400> 28
gagcacccag taggaataga attag 25

<210> 29
<211> 25
<212> DNA
<213> Artificial Sequence

<220>
<223> Artificial Sequence

<400> 29
cagggagaga gtgtagaaag aatag 25

<210> 30
<211> 22
<212> DNA
<213> Artificial Sequence

<220>
<223> Primer

<400> 30
gcctgatctc taaacactgg ag 22

<210> 31

<211> 22
<212> DNA
<213> Artificial Sequence

<220>
<223> Primer

<400> 31
acactccaat tccaactcta cc 22

<210> 32
<211> 21
<212> DNA
<213> Artificial Sequence

<220>
<223> Primer

<400> 32
gagaaatcag aggcccaaag a 21

<210> 33
<211> 22
<212> DNA
<213> Artificial Sequence

<220>
<223> Primer

<400> 33
gcctgtgcct acctgtttaa ta 22

<210> 34
<211> 21
<212> DNA
<213> Artificial Sequence

<220>
<223> Primer

<400> 34
aaatccacag ccagtacctt c 21

<210> 35
<211> 22
<212> DNA
<213> Artificial Sequence

<220>
<223> Primer

<400> 35
gagaaatcca tcttcaggtc ag 22

<210> 36
<211> 21
<212> DNA
<213> Artificial Sequence

<220>
<223> Primer

<400> 36
aaagaccctt cacactttgt t 21

<210> 37
<211> 20
<212> DNA
<213> Artificial Sequence

<220>
<223> Primer

<400> 37
tacaccagtg gaaggactat 20

<210> 38
<211> 24
<212> DNA
<213> Artificial Sequence

<220>
<223> Primer

<400> 38
ccctactaat gacatccaga ataa 24

<210> 39
<211> 22
<212> DNA
<213> Artificial Sequence

<220>
<223> Primer

<400> 39
gaggaatgag agacaacact tc 22

<210> 40
<211> 20
<212> DNA
<213> Artificial Sequence

<220>
<223> Primer

<400> 40
ccttctgtag cctcttccta 20

<210> 41
<211> 21
<212> DNA
<213> Artificial Sequence

<220>
<223> Primer

<400> 41
ggcagcaata atcacctctg a 21

<210> 42
<211> 26
<212> DNA
<213> Artificial Sequence

<220>
<223> Primer

<400> 42
caatctgata agctttctag ggtagt 26

<210> 43
<211> 20
<212> DNA
<213> Artificial Sequence

<220>
<223> Primer

<400> 43
ttcttccctt tccagactca 20

<210> 44
<211> 21
<212> DNA
<213> Artificial Sequence

<220>
<223> Primer

<400> 44
gaaagccaga gaatgagaga g 21

<210> 45
<211> 23
<212> DNA
<213> Artificial Sequence

<220>
<223> Primer

<400> 45
ctagaacctt ccatccttaa tgt 23

<210> 46
<211> 20
<212> DNA
<213> Artificial Sequence

<220>
<223> Primer

<400> 46
gctcctacat ggctacaaag 20

<210> 47
<211> 20
<212> DNA
<213> Artificial Sequence

<220>
<223> Primer

<400> 47
gctttgttgg gagagttaga 20

<210> 48
<211> 21
<212> DNA
<213> Artificial Sequence

<220>
<223> Primer

<400> 48
caattcattg agaagtgcca a 21

<210> 49
<211> 21
<212> DNA
<213> Artificial Sequence

<220>
<223> Primer

<400> 49
aaagcagctc aacctcttaa t 21

<210> 50
<211> 24
<212> DNA
<213> Artificial Sequence

<220>
<223> Primer

<400> 50
tgaaagattc ctgagttatt agcc 24

<210> 51
<211> 22
<212> DNA
<213> Artificial Sequence

<220>
<223> Primer

<400> 51
ccgactttct acttattcca ca 22

<210> 52
<211> 20
<212> DNA

<213> Artificial Sequence

<220>
<223> Primer

<400> 52
gctgtataaa ccctccacag 20

<210> 53
<211> 21
<212> DNA
<213> Artificial Sequence

<220>
<223> Primer

<400> 53
cccaaactcc tctcatcttt c 21

<210> 54
<211> 20
<212> DNA
<213> Artificial Sequence

<220>
<223> Primer

<400> 54
aaggctgatt agccgtaatg 20

<210> 55
<211> 21
<212> DNA
<213> Artificial Sequence

<220>
<223> Primer

<400> 55
aaagtcaact tctctgtgcg a 21

<210> 56
<211> 25
<212> DNA
<213> Artificial Sequence

<220>
<223> Primer

<400> 56
ttattcttcc accattatcc ctcat 25

<210> 57
<211> 23
<212> DNA
<213> Artificial Sequence

<220>

<223> Primer

<400> 57
tgtcgtttct aaggatggga aag 23

<210> 58
<211> 21
<212> DNA
<213> Artificial Sequence

<220>
<223> Primer

<400> 58
gtttcatgaa gctgcctctc t 21

<210> 59
<211> 20
<212> DNA
<213> Artificial Sequence

<220>
<223> Primer

<400> 59
atgcctggct tcatgttact 20

<210> 60
<211> 25
<212> DNA
<213> Artificial Sequence

<220>
<223> Primer

<400> 60
aattactgac ctagaccttt caaga 25

<210> 61
<211> 20
<212> DNA
<213> Artificial Sequence

<220>
<223> Primer

<400> 61
gtgtggacat tggttccttt 20

<210> 62
<211> 22
<212> DNA
<213> Artificial Sequence

<220>
<223> Primer

<400> 62

ttagggcaaa caagatgaga aa 22

<210> 63
<211> 20
<212> DNA
<213> Artificial Sequence

<220>
<223> Primer

<400> 63
tttcttccag cagagctcac 20

<210> 64
<211> 17
<212> DNA
<213> Artificial Sequence

<220>
<223> Primer

<400> 64
gccagccctg gatgaat 17

<210> 65
<211> 21
<212> DNA
<213> Artificial Sequence

<220>
<223> Primer

<400> 65
tcactcttcc caccctctta t 21

<210> 66
<211> 22
<212> DNA
<213> Artificial Sequence

<220>
<223> Primer

<400> 66
catctcaaga aagagggctc aa 22

<210> 67
<211> 20
<212> DNA
<213> Artificial Sequence

<220>
<223> Primer

<400> 67
cccaactcaa tttcaagctg 20

<210> 68

<211> 22
<212> DNA
<213> Artificial Sequence

<220>
<223> Primer

<400> 68
ccaaagatcc aattagcaca tc 22

<210> 69
<211> 24
<212> DNA
<213> Artificial Sequence

<220>
<223> Primer

<400> 69
acctatcaac ttggttaccc taaa 24

<210> 70
<211> 24
<212> DNA
<213> Artificial Sequence

<220>
<223> Primer

<400> 70
tttgtgtggg aatctttatc tgtg 24

<210> 71
<211> 19
<212> DNA
<213> Artificial Sequence

<220>
<223> Primer

<400> 71
tgtcccaatg cacagactt 19

<210> 72
<211> 18
<212> DNA
<213> Artificial Sequence

<220>
<223> Primer

<400> 72
aaacagggtg ggcctaaa 18

<210> 73
<211> 20
<212> DNA
<213> Artificial Sequence

<220>
<223> Primer

<400> 73
ctttcggttc tctccaacca 20

<210> 74
<211> 20
<212> DNA
<213> Artificial Sequence

<220>
<223> Primer

<400> 74
aacaacttcc ctgagacgtg 20

<210> 75
<211> 24
<212> DNA
<213> Artificial Sequence

<220>
<223> Primer

<400> 75
cacaccaaat tgttaagagt tacc 24

<210> 76
<211> 25
<212> DNA
<213> Artificial Sequence

<220>
<223> Primer

<400> 76
gtcctggtaa caaatccagt taata 25

<210> 77
<211> 19
<212> DNA
<213> Artificial Sequence

<220>
<223> Primer

<400> 77
ggtctttagt gggcgtacc 19

<210> 78
<211> 20
<212> DNA
<213> Artificial Sequence

<220>
<223> Primer

<400> 78
tcctctcacc gaagatacca 20